# EUROPEAN PATENT APPLICATION

(11) **EP 4 585 605 A1**
(43) Date of publication of application: **16.07.2025**
(21) Application number: 23879878.9
(22) Date of filing: 19.10.2023
(51) Int. Cl.: C07K 1/14, C07K 1/30, C07K 7/54, C30B 7/04, C30B 7/06, C30B 29/54

(54) **METHOD FOR PRODUCING CYCLIC PEPTIDE CRYSTALS**

(30) Priority: 20.10.2022 JP 2022168237
(71) Applicant: CHUGAI SEIYAKU KABUSHIKI KAISHA, Tokyo 115-8543 (JP)
(72) Inventor: AOKI Masahide, Gotemba-shi, Shizuoka 412-8513 (JP); TANIDA Satoshi, Gotemba-shi, Shizuoka 412-8513 (JP); SAKON Aya, Gotemba-shi, Shizuoka 412-8513 (JP); IWATA Tomoya, Gotemba-shi, Shizuoka 412-8513 (JP)
(74) Representative: Vossius & Partner Patentanwälte Rechtsanwälte mbB
(86) International application number: PCT/JP2023/037907
(87) International publication number: WO 2024/085235

(57) **Abstract**

There is disclosed a method for producing cyclic peptide crystals, the method comprising a step of bringing a cyclic peptide into contact with a specific solvent.

## Description

### [Technical Field]

The present invention relates to a method for producing crystals of a cyclic peptide containing a N-substituted amino acid residue, and a method for screening those crystals.

### [Background Art]

Up to now, compounds used as oral drugs have been considered to be desirable to have a molecular weight of 500 g/mol or less, as is known as the Lipinski rule (Non Patent Literature 1). In recent years, it has become known that compounds with a molecular weight of more than 500 g/mol may contribute to the inhibition of protein-protein interactions in proteins that have been considered difficult to target with conventional small molecule compounds, referred to as tough targets. These molecules are called middle molecule compounds (molecular weight 500 to 2000 g/mol), which are neither small molecules with a molecular weight of 500 g/mol or less that have been mainly used as oral drugs, nor polymeric molecules with a molecular weight of more than 100000 g/mol, such as antibody pharmaceuticals, and have gained prominence as new modalities that can realize drug discovery against tough targets (Non Patent Literature 2).

Peptides composed of natural amino acids, such as insulin used in the treatment of hyperglycemia, have poor metabolic stability and have conventionally been difficult to be developed as oral drugs. However, it has been found that the metabolic stability and membrane permeability of the peptide is improved by cyclizing the peptide and using a non-natural amino acid such as N-methylamino acid in the peptide (Non Patent Literatures 3 and 4).

Among cyclic peptides containing non-natural amino acids, it has become known that cyclic peptides particularly containing N-substituted amino acids may have metabolic stability or membrane permeability, that is, may have drug-likeness (Patent Literature 1). It has also been suggested that cyclic peptides containing non-natural amino acids are useful for the creation of inhibitors of protein-protein interactions (Non Patent Literature 5).

While cyclic peptides as middle molecule compounds are attracting attention in drug discovery, there have been no reports on crystals for many cyclic peptides containing N-substituted amino acids. On the other hand, as for cyclosporin A, which has long been known as a cyclic peptide, it has been reported to have a variety of crystal forms (Patent Literatures 2 and 3, Non Patent Literatures 6, 7, and 8).

### [Citation List]

### [Patent Literature]

[Patent Literature 1] International Publication No. WO 2013/100132
[Patent Literature 2] International Publication No. WO 2012/166610
[Patent Literature 3] Japanese Patent Laid-Open No. 2017-210488

### [Non Patent Literature]

[Non Patent Literature 1] Adv. Drug Del. Rev. 1997, 23, 3-25.
[Non Patent Literature 2] Future Med. Chem., 2009, 1, 1289-1310.
[Non Patent Literature 3] Acc. Chem. Res., 2008, 41, 1331-1342.
[Non Patent Literature 4] Angew. Chem. Int. Ed., 2013, 52, 254-269.
[Non Patent Literature 5] Chem. Rev., 2019, 119, 10360-10391.
[Non Patent Literature 6] J. Pharm. Sci., 2018, 107, 3070-3079.
[Non Patent Literature 7] J. Inclusion Phenomena & Macrocyclic Chem., 2000, 37, 137-153.
[Non Patent Literature 8] Zeitschrift fuer Kristallographie, 1996, 211, 313-318.

### [Summary of Invention]

### [Technical Problem]

For the crystallization of cyclic peptides in pharmaceutical development, it has been necessary to search for and find the optimal crystallization conditions for each individual cyclic peptide by trial and error. A general and simple technology for obtaining these cyclic peptides as crystals is needed. The present invention has been made in view of such circumstances. In one aspect, an object of the present invention is to provide a method for producing crystals of a cyclic peptide containing a N-substituted amino acid residue. In one aspect, an object of the present invention is to provide a method for screening crystals of a cyclic peptide containing a N-substituted amino acid residue. In one aspect, an object of the present invention is to provide a method for screening a crystallization method. Furthermore, in one aspect, an object of the present invention is to provide a method for isolating and purifying the target cyclic peptide or salt thereof, or solvate thereof, as crystals without resorting to column chromatography.

### [Solution to Problem]

As a result of diligent investigations in order to solve the above problems, the present inventors have found a method for efficiently screening crystals in order to obtain a cyclic peptide as crystals, by bringing the cyclic peptide into contact with a specific solvent. Furthermore, the present invention has been completed as a method for producing crystals that is applicable to a variety of cyclic peptides.

That is, the present invention provides the followings.
[A1] A method for producing cyclic peptide crystals, the method comprising a step of bringing a cyclic peptide into contact with a solvent, wherein the cyclic peptide is a cyclic peptide having the following characteristics (I), (II), and (III):
   (I) a characteristic of containing a cyclic moiety composed of 8 to 16 residues of amino acids in total, with a total number of amino acids being 8 to 20 residues;
   (II) a characteristic of containing at least 2 residues of N-substituted amino acids; and
   (III) a characteristic of having a molecular weight (g/mol) of 1204 or more and 3000 or less.
[A1-1] The method according to [A1], wherein the crystals have one or more diffraction peaks in powder X-ray diffraction using CuKα radiation, or have polarization in observation using a polarization microscope.
[A1-2] The method according to [A1], wherein the crystals have one or more diffraction peaks in powder X-ray diffraction using CuKα radiation.
[A2] The method according to any one of [A1] to [A1-2], wherein the solvent is any solvent selected from the group consisting of the following (1), (2) and (3):
   (1) (i) a solvent with a molecular weight of 18 or more and 170 or less, or (ii) a mixed solvent containing two or more solvents with a molecular weight of 18 or more and 170 or less;
   (2) water containing 0.01 to 30 wt/v% of a surfactant and 5 to 50 v/v% of a water-soluble organic solvent, based on a total amount of the solvent; and
   (3) (i) a PEG-based solvent, or (ii) a mixed solvent containing one or more selected from the group consisting of an alcohol-based solvent, an aliphatic hydrocarbon-based solvent, and water, and a PEG-based solvent.
[A3] The method according to any one of [A1] to [A1-2], wherein the solvent is (i) a solvent with a molecular weight of 18 or more and 170 or less, or (ii) a mixed solvent containing two or more solvents with a molecular weight of 18 or more and 170 or less.
[A4] The method according to [A3], wherein the solvent is a solvent selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a ketone-based solvent, an aliphatic hydrocarbon-based solvent, and water.
[A5] The method according to [A3], wherein the solvent is a solvent selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent.
[A6] The method according to [A3], wherein the solvent is a solvent selected from the group consisting of an amide-based solvent, a sulfoxide-based solvent, an aromatic hydrocarbon-based solvent, a halogen-based solvent, and an ester-based solvent.
[A7] The method according to [A3], wherein the solvent is a solvent selected from the group consisting of an amide-based solvent, a sulfoxide-based solvent, an aromatic hydrocarbon-based solvent, and a halogen-based solvent.
[A8] The method according to [A3], wherein the solvent is a solvent selected from the group consisting of an amide-based solvent, a sulfoxide-based solvent, and an aromatic hydrocarbon-based solvent.
[A9] The method according to any one of [A1] to [A1-2], wherein the solvent is solvent (A) with a molecular weight of 18 or more and 170 or less, or a mixed solvent of solvent (A) with a molecular weight of 18 or more and 170 or less and solvent (B) with a molecular weight of 18 or more and 170 or less, the solvent (A) is one or more selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent, and the solvent (B) is one or more selected from the group consisting of an aliphatic hydrocarbon-based solvent, ethylene glycol, and water.
[A10] The method according to any one of [A1] to [A1-2], wherein the solvent is solvent (A) with a molecular weight of 18 or more and 170 or less, or a mixed solvent of solvent (A) with a molecular weight of 18 or more and 170 or less and solvent (B) with a molecular weight of 18 or more and 170 or less, the solvent (A) is selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent, and the solvent (B) is selected from the group consisting of an aliphatic hydrocarbon-based solvent, ethylene glycol, and water.
[A11] The method according to [A9], wherein the solvent (A) is one or more selected from the group consisting of an amide-based solvent, a sulfoxide-based solvent, an aromatic hydrocarbon-based solvent, a halogen-based solvent, and an ester-based solvent.
[A12] The method according to [A9], wherein the solvent (A) is one or more selected from the group consisting of an amide-based solvent, a sulfoxide-based solvent, an aromatic hydrocarbon-based solvent, and a halogen-based solvent.
[A13] The method according to [A9], wherein the solvent (A) is one or more selected from the group consisting of an amide-based solvent, a sulfoxide-based solvent, and an aromatic hydrocarbon-based solvent.
[A14] The method according to [A9] or [A10], wherein the solvent (A) is an amide-based solvent.
[A15] The method according to [A14], wherein the amide-based solvent is one or more selected from the group consisting of formamide, N-methylformamide, N,N-dimethylformamide, N,N-dimethylacetamide, 2-pyrrolidone, and N-methylpyrrolidone.
[A16] The method according to [A14], wherein the amide-based solvent is formamide.
[A17] The method according to [A9] or [A10], wherein the solvent (A) is a sulfoxide-based solvent.
[A18] The method according to [A17], wherein the sulfoxide-based solvent is one or more selected from the group consisting of dimethyl sulfoxide, phenyl methyl sulfoxide, and diethyl sulfoxide.
[A19] The method according to [A17], wherein the sulfoxide-based solvent is dimethyl sulfoxide.
[A20] The method according to [A9] or [A10], wherein the solvent (A) is an aromatic hydrocarbon-based solvent.
[A21] The method according to [A20], wherein the aromatic hydrocarbon-based solvent is one or more selected from the group consisting of benzene, toluene, xylene, ethylbenzene, tetralin, and cumene.
[A22] The method according to [A20], wherein the aromatic hydrocarbon-based solvent is toluene, tetralin, or cumene.
[A23] The method according to [A9] or [A10], wherein the solvent (A) is a halogen-based solvent.
[A24] The method according to [A23], wherein the halogen-based solvent is one or more selected from the group consisting of dichloromethane, chloroform, 1,2-dichloroethane, chlorobenzene, bromobenzene, and carbon tetrachloride.
[A25] The method according to [A23], wherein the halogen-based solvent is dichloromethane or chlorobenzene.
[A26] The method according to [A9] or [A10], wherein the solvent (A) is an alcohol-based solvent.
[A27] The method according to [A26], wherein the alcohol-based solvent is one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, and benzyl alcohol.
[A28] The method according to [A26], wherein the alcohol-based solvent is methanol, ethanol, 1-propanol, isopropanol, or n-butanol.
[A29] The method according to [A9] or [A10], wherein the solvent (A) is an ether-based solvent.
[A30] The method according to [A29], wherein the ether-based solvent is one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether.
[A31] The method according to [A29], wherein the ether-based solvent is tetrahydrofuran, 1,4-dioxane, diisopropyl ether, anisole, or t-butyl methyl ether.
[A32] The method according to [A9] or [A10], wherein the solvent (A) is an ester-based solvent.
[A33] The method according to [A32], wherein the ester-based solvent is one or more selected from the group consisting of ethyl formate, methyl acetate, ethyl acetate, methyl propionate, n-butyl acetate, propyl acetate, isopropyl acetate, isobutyl acetate, pentyl acetate, and γ-valerolactone.
[A34] The method according to [A32], wherein the ester-based solvent is ethyl acetate, isopropyl acetate, or n-butyl acetate.
[A35] The method according to [A9] or [A10], wherein the solvent (A) is a nitrile-based solvent.
[A36] The method according to [A35], wherein the nitrile-based solvent is one or more selected from the group consisting of acetonitrile, benzonitrile, and propionitrile.
[A37] The method according to [A35], wherein the nitrile-based solvent is acetonitrile.
[A38] The method according to [A9] or [A10], wherein the solvent (A) is a ketone-based solvent.
[A39] The method according to [A38], wherein the ketone-based solvent is one or more selected from the group consisting of acetone, methyl ethyl ketone, methyl isobutyl ketone, methyl butyl ketone, cyclohexanone, diethyl ketone, cyclopentanone, and 3-acetylpyridine.
[A40] The method according to [A38], wherein the ketone-based solvent is acetone, methyl ethyl ketone, or methyl isobutyl ketone.
[A41] The method according to any one of [A9] to [A40], wherein the solvent (B) is one or more solvents selected from the group consisting of an aliphatic hydrocarbon-based solvent, ethylene glycol, and water.
[A42] The method according to any one of [A9] to [A40], wherein the solvent (B) is an aliphatic hydrocarbon-based solvent, ethylene glycol, or water.
[A43] The method according to any one of [A9] to [A40], wherein the solvent (B) is an aliphatic hydrocarbon-based solvent.
[A44] The method according to [A43], wherein the aliphatic hydrocarbon-based solvent is one or more selected from the group consisting of n-pentane, n-hexane, n-heptane, n-octane, cyclopentane, cyclohexane, methylcycloheptane, and methylcyclohexane.
[A45] The method according to [A43], wherein the aliphatic hydrocarbon-based solvent is n-heptane, cyclohexane, or methylcyclohexane.
[A46] The method according to any one of [A9] to [A40], wherein the solvent (B) is water.
[A46-1] The method according to any one of [A9] to [A40], wherein the solvent (B) is ethylene glycol.
[A47] The method according to any one of [A9] to [A46], wherein a volume ratio (v/v) between the solvent (A) and the solvent (B) in the solvent is 1:0 to 1:40.
[A48] The method according to any one of [A9] to [A46], wherein a volume ratio (v/v) between the solvent (A) and the solvent (B) in the solvent is 1:0 to 1:30, 1:0 to 1:20, 1:0 to 1:10, 1:0 to 1:7, or 1:0 to 1:5.
[A49] The method according to any one of [A9] to [A46], wherein a volume ratio (v/v) between the solvent (A) and the solvent (B) in the solvent is 1:0 to 1:4.
[A50] The method according to any one of [A9] to [A46], wherein a volume ratio (v/v) between the solvent (A) and the solvent (B) in the solvent is 10:1 to 1:40.
[A51] The method according to any one of [A9] to [A46], wherein a volume ratio (v/v) between the solvent (A) and the solvent (B) in the solvent is 5:1 to 1:30, 3:1 to 1:20, 2:1 to 1:10, 1:1 to 1:7, or 1:2 to 1:5.
[A52] The method according to any one of [A9] to [A46], wherein a volume ratio (v/v) between the solvent (A) and the solvent (B) in the solvent is 1:2 to 1:4.
[A52-1] The method according to any one of [A9] to [A52], wherein the solvent (A) is a solvent with a molecular weight of 18 or more and 160 or less, a molecular weight of 18 or more and 150 or less, a molecular weight of 18 or more and 140 or less, or a molecular weight of 32 or more and 135 or less.
[A52-2] The method according to any one of [A9] to [A52], wherein the solvent (A) is a solvent with a molecular weight of 32 or more and 135 or less.
[A52-3] The method according to any one of [A9] to [A52-2], wherein the solvent (B) is a solvent with a molecular weight of 18 or more and 160 or less, a molecular weight of 18 or more and 135 or less, a molecular weight of 18 or more and 120 or less, or a molecular weight of 18 or more and 105 or less.
[A52-4] The method according to any one of [A9] to [A52-2], wherein the solvent (B) is a solvent with a molecular weight of 18 or more and 105 or less.
[A53] The method according to any one of [A9] to [A52-4], wherein the solvent (A) and the solvent (B) have a melting point of 25°C or lower.
[A54] The method according to any one of [A1] to [A1-2], wherein the solvent is water containing 0.01 to 30 wt/v% of a surfactant and 5 to 50 v/v% of a water-soluble organic solvent, based on a total amount of the solvent.
[A55] The method according to [A54], wherein the surfactant is one or more selected from the group consisting of a cationic surfactant, an anionic surfactant, an amphoteric surfactant, and a nonionic surfactant.
[A56] The method according to [A54], wherein the surfactant is one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, a lecithin, an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester.
[A57] The method according to [A54], wherein the surfactant is one or more selected from the group consisting of an alkyl sulfate, an alkyl aryl polyoxyethylene ether, a polyoxyethylene glycerin fatty acid ester, and a polyoxyethylene sorbitan fatty acid ester.
[A58] The method according to [A54], wherein the surfactant is an alkyl sulfate, an alkyl aryl polyoxyethylene ether, a polyoxyethylene glycerin fatty acid ester, or a polyoxyethylene sorbitan fatty acid ester.
[A59] The method according to [A54], wherein the surfactant is sodium lauryl sulfate, 4-(1, 1,3,3-tetramethylbutyl)phenyl-polyethylene glycol, Polyoxyl 35 Hydrogenated Castor Oil, or polyoxyethylene sorbitan monolaurate.
[A59-1] The method according to [A54], wherein the surfactant is Polyoxyl 35 Hydrogenated Castor Oil.
[A60] The method according to [A54], wherein the surfactant is an ionic surfactant or a nonionic surfactant.
[A61] The method according to [A54], wherein the surfactant is an ionic surfactant.
[A62] The method according to [A61], wherein the ionic surfactant is one or more selected from the group consisting of a cationic surfactant, an anionic surfactant, and an amphoteric surfactant.
[A63] The method according to [A61], wherein the ionic surfactant is one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, and a lecithin.
[A64] The method according to [A61], wherein the ionic surfactant is an alkyl sulfate.
[A65] The method according to [A61], wherein the ionic surfactant is a lauryl sulfate.
[A66] The method according to [A61], wherein the ionic surfactant is sodium lauryl sulfate.
[A67] The method according to [A54], wherein the surfactant is a nonionic surfactant.
[A68] The method according to [A67], wherein the nonionic surfactant is one or more selected from the group consisting of an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester.
[A69] The method according to [A67], wherein the nonionic surfactant is one or more selected from the group consisting of an alkyl aryl polyoxyethylene ether, a polyoxyethylene glycerin fatty acid ester, and a polyoxyethylene sorbitan fatty acid ester.
[A70] The method according to [A67], wherein the nonionic surfactant is one or more selected from the group consisting of 4-(1,1,3,3-tetramethylbutyl)phenyl-polyethylene glycol, Polyoxyl 35 Hydrogenated Castor Oil, and polyoxyethylene sorbitan monolaurate.
[A71] The method according to any one of [A54] to [A70], wherein the water-soluble organic solvent is an alcohol-based solvent, an amide-based solvent, a nitrile-based solvent, or a sulfoxide-based solvent.
[A72] The method according to any one of [A54] to [A70], wherein the water-soluble organic solvent is an alcohol-based solvent or a sulfoxide-based solvent.
[A73] The method according to any one of [A54] to [A70], wherein the water-soluble organic solvent is methanol, ethanol, 1-propanol, 2-propanol, or dimethyl sulfoxide.
[A74] The method according to any one of [A54] to [A70], wherein the water-soluble organic solvent is ethanol or dimethyl sulfoxide.
[A75] The method according to any one of [A54] to [A74], wherein a content of the surfactant is 0.02 to 20 wt/v%, 0.05 to 15 wt/v%, 0.1 to 10 wt/v%, 0.12 to 8 wt/v%, 0.15 to 5 wt/v%, or 0.18 to 3 wt/v% based on a total amount of the solvent, and a content of the water-soluble organic solvent is 5 to 40 v/v%, 5 to 30 v/v%, 5 to 25 v/v%, 8 to 20 v/v%, or 10 to 15 v/v% based on a total amount of the solvent.
[A76] The method according to any one of [A54] to [A74], wherein a content of the surfactant is 0.1 to 10 wt/v% based on a total amount of the solvent, and a content of the water-soluble organic solvent is 5 to 25 v/v% based on a total amount of the solvent.
[A77] The method according to any one of [A1] to [A1-2], wherein the solvent is (i) a PEG-based solvent, or (ii) a mixed solvent containing one or more selected from the group consisting of an alcohol-based solvent, an aliphatic hydrocarbon-based solvent, and water, and a PEG-based solvent.
[A78] The method according to any one of [A1] to [A1-2], wherein the solvent is a PEG-based solvent.
[A79] The method according to any one of [A1] to [A1-2], wherein the solvent is a mixed solvent of one or more selected from the group consisting of an alcohol-based solvent, an aliphatic hydrocarbon-based solvent, and water, and a PEG-based solvent.
[A80] The method according to [A78] or [A79], wherein the PEG-based solvent is (i) a solvent represented by R¹(OCHR³CH₂)nOR², n being a natural number of 1 or more and 10 or less, or (ii) a mixture of solvents represented by R¹(OCHR³CH₂)nOR², an average of n being 3 to 100, where R¹ and R² are each independently hydrogen, a C₁ to C₄ alkyl, or -C(=O)R⁴, R³ is hydrogen or a C₁ to C₄ alkyl, and R⁴ is a C₁ to C₁₈ alkyl optionally substituted with a hydroxy group or a C₁ to C₁₈ alkenyl optionally substituted with a hydroxy group.
[A81] The method according to [A78] or [A79], wherein the PEG-based solvent is (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or a C₁ to C₄ alkyl, R² is hydrogen, a C₁ to C₄ alkyl, or -C(=O)R⁴, R³ is hydrogen or a C₁ to C₄ alkyl, and R⁴ is a C₁₀ to C₁₈ alkyl.
[A82] The method according to [A78] or [A79], wherein the PEG-based solvent is (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or methyl, R² is hydrogen, methyl, or -C(=O)R⁴, R³ is hydrogen or methyl, and R⁴ is a C₁₁ to C₁₇ alkyl.
[A83] The method according to [A78] or [A79], wherein the PEG-based solvent is diglyme, triglyme, tetraglyme, ethylene glycol, diethylene glycol, triethylene glycol, tetraethylene glycol, polyethylene glycol, polypropylene glycol, or a polyethylene glycol monofatty acid ester.
[A84] The method according to [A78] or [A79], wherein the PEG-based solvent is polyethylene glycol.
[A84-2] The method according to [A84], wherein the polyethylene glycol has a number average molecular weight of 150 to 5000.
[A84-3] The method according to [A84], wherein the polyethylene glycol has a number average molecular weight of 360 to 440, 540 to 660, 900 to 1100, or 1800 to 2200.
[A85] The method according to [A84], wherein the polyethylene glycol is PEG400, PEG600, PEG1000, or PEG2000.
[A86] The method according to [A78] or [A79], wherein the PEG-based solvent is a polyethylene glycol monofatty acid ester.
[A87] The method according to [A86], wherein the polyethylene glycol monofatty acid ester is polyethylene glycol monostearate or polyethylene glycol monolaurate.
[A88] The method according to any one of [A77] or [A79] to [A87], wherein the one or more selected from the group consisting of an alcohol-based solvent, an aliphatic hydrocarbon-based solvent, and water are one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1, 1,3,3,3-hexafluoro-2-propanol, benzyl alcohol, n-pentane, n-hexane, n-heptane, n-octane, cyclopentane, cyclohexane, methylcycloheptane, and water.
[A89] The method according to any one of [A77] or [A79] to [A87], wherein the one or more selected from the group consisting of an alcohol-based solvent, an aliphatic hydrocarbon-based solvent, and water are one or more selected from the group consisting of 2-propanol, n-heptane, and water.
[B1] A method for producing cyclic peptide crystals, the method comprising a step of bringing a cyclic peptide into contact with a solvent, wherein the cyclic peptide is a cyclic peptide having the following characteristics (I) and (II):
   (I) a characteristic of containing a cyclic moiety composed of 8 to 16 residues of amino acids in total, with a total number of amino acids being 8 to 20 residues; and
   (II) a characteristic of containing at least 2 residues of N-substituted amino acids,
   the solvent is solvent (A) or a mixed solvent containing solvent (A) and solvent (B), the solvent (A) is one or more selected from the group consisting of an amide-based solvent, a sulfoxide-based solvent, an aromatic hydrocarbon-based solvent, a halogen-based solvent, and an ester-based solvent, and the solvent (B) is one or more selected from the group consisting of an aliphatic hydrocarbon-based solvent, ethylene glycol, and water.
[B1-1] The method according to [B1], wherein the crystals have one or more diffraction peaks in powder X-ray diffraction using CuKα radiation, or have polarization in observation using a polarization microscope.
[B1-2] The method according to [B1], wherein the crystals have one or more diffraction peaks in powder X-ray diffraction using CuKα radiation.
[B2] The method according to any one of [B1] to [B1-2], wherein the solvent (A) is one or more selected from the group consisting of an amide-based solvent, a sulfoxide-based solvent, and an aromatic hydrocarbon-based solvent.
[B3] The method according to any one of [B1] to [B1-2], wherein the solvent (A) is an amide-based solvent.
[B4] The method according to [B3], wherein the amide-based solvent is one or more selected from the group consisting of formamide, N-methylformamide, N,N-dimethylformamide, N,N-dimethylacetamide, 2-pyrrolidone, and N-methylpyrrolidone.
[B5] The method according to [B3], wherein the amide-based solvent is formamide.
[B6] The method according to any one of [B1] to [B1-2], wherein the solvent (A) is a sulfoxide-based solvent.
[B7] The method according to [B6], wherein the sulfoxide-based solvent is one or more selected from the group consisting of dimethyl sulfoxide, phenyl methyl sulfoxide, and diethyl sulfoxide.
[B8] The method according to [B6], wherein the sulfoxide-based solvent is dimethyl sulfoxide.
[B9] The method according to any one of [B1] to [B1-2], wherein the solvent (A) is an aromatic hydrocarbon-based solvent.
[B10] The method according to [B9], wherein the aromatic hydrocarbon-based solvent is one or more selected from the group consisting of benzene, toluene, xylene, ethylbenzene, tetralin, and cumene.
[B11] The method according to [B9], wherein the aromatic hydrocarbon-based solvent is toluene, tetralin, or cumene.
[B12] The method according to any one of [B1] to [B 1-2], wherein the solvent (A) is a halogen-based solvent.
[B13] The method according to [B12], wherein the halogen-based solvent is one or more selected from the group consisting of dichloromethane, chloroform, 1,2-dichloroethane, chlorobenzene, bromobenzene, and carbon tetrachloride.
[B14] The method according to [B12], wherein the halogen-based solvent is dichloromethane or chlorobenzene.
[B15] The method according to any one of [B1] to [B1-2], wherein the solvent (A) is an ester-based solvent.
[B16] The method according to [B15], wherein the ester-based solvent is one or more selected from the group consisting of ethyl formate, methyl acetate, ethyl acetate, methyl propionate, n-butyl acetate, propyl acetate, isopropyl acetate, isobutyl acetate, pentyl acetate, and γ-valerolactone.
[B17] The method according to [B15], wherein the ester-based solvent is ethyl acetate, isopropyl acetate, or n-butyl acetate.
[B18] The method according to any one of [B1] to [B 17], wherein the solvent (B) is an aliphatic hydrocarbon-based solvent.
[B19] The method according to [B18], wherein the aliphatic hydrocarbon-based solvent is one or more selected from the group consisting of n-pentane, n-hexane, n-heptane, n-octane, cyclopentane, cyclohexane, methylcycloheptane, and methylcyclohexane.
[B20] The method according to [B18], wherein the aliphatic hydrocarbon-based solvent is n-heptane, cyclohexane, or methylcyclohexane.
[B21] The method according to any one of [B1] to [B17], wherein the solvent (B) is water.
[B21-1] The method according to any one of [B1] to [B17], wherein the solvent (B) is ethylene glycol.
[B22] The method according to any one of [B1] to [B21], wherein a volume ratio (v/v) between the solvent (A) and the solvent (B) in the solvent is 1:0 to 1:40.
[B23] The method according to any one of [B1] to [B21], wherein a volume ratio (v/v) between the solvent (A) and the solvent (B) in the solvent is 1:0 to 1:30, 1:0 to 1:20, 1:0 to 1:10, 1:0 to 1:7, or 1:0 to 1:5.
[B24] The method according to any one of [B1] to [B21], wherein a volume ratio (v/v) between the solvent (A) and the solvent (B) in the solvent is 1:0 to 1:4.
[B25] The method according to any one of [B1] to [B21], wherein a volume ratio (v/v) between the solvent (A) and the solvent (B) in the solvent is 10:1 to 1:40.
[B26] The method according to any one of [B1] to [B21], wherein a volume ratio (v/v) between the solvent (A) and the solvent (B) in the solvent is 5: 1 to 1:30, 3: 1 to 1:20, 2: 1 to 1:10, 1: 1 to 1:7, or 1:2 to 1:5.
[B27] The method according to any one of [B1] to [B21], wherein a volume ratio (v/v) between the solvent (A) and the solvent (B) in the solvent is 1:2 to 1:4.
[B28] The method according to any one of [B1] to [B27], wherein the solvent (A) and the solvent (B) have a melting point of 25°C or lower.
[B29] A method for producing cyclic peptide crystals, the method comprising a step of bringing a cyclic peptide into contact with a solvent, wherein the cyclic peptide is a cyclic peptide having the following characteristics (I) and (II):
   (I) a characteristic of containing a cyclic moiety composed of 8 to 16 residues of amino acids in total, with a total number of amino acids being 8 to 20 residues; and
   (II) a characteristic of containing at least 2 residues of N-substituted amino acids, and
   the solvent is water containing 0.01 to 30 wt/v% of a surfactant and 5 to 50 v/v% of a water-soluble organic solvent, based on a total amount of the solvent.
[B29-1] The method according to [B29], wherein the crystals have one or more diffraction peaks in powder X-ray diffraction using CuKα radiation, or have polarization in observation using a polarization microscope.
[B29-2] The method according to [B29], wherein the crystals have one or more diffraction peaks in powder X-ray diffraction using CuKα radiation.
[B30] The method according to any one of [B29] to [B29-2], wherein the surfactant is one or more selected from the group consisting of a cationic surfactant, an anionic surfactant, an amphoteric surfactant, and a nonionic surfactant.
[B31] The method according to any one of [B29] to [B29-2], wherein the surfactant is one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, a lecithin, an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester.
[B32] The method according to any one of [B29] to [B29-2], wherein the surfactant is one or more selected from the group consisting of an alkyl sulfate, an alkyl aryl polyoxyethylene ether, a polyoxyethylene glycerin fatty acid ester, and a polyoxyethylene sorbitan fatty acid ester. [B33] The method according to any one of [B29] to [B29-2], wherein the surfactant is an alkyl sulfate, an alkyl aryl polyoxyethylene ether, a polyoxyethylene glycerin fatty acid ester, or a polyoxyethylene sorbitan fatty acid ester.
[B34] The method according to any one of [B29] to [B29-2], wherein the surfactant is sodium lauryl sulfate, 4-(1,1,3,3-tetramethylbutyl)phenyl-polyethylene glycol, Polyoxyl 35 Hydrogenated Castor Oil, or polyoxyethylene sorbitan monolaurate.
[B35] The method according to any one of [B29] to [B29-2], wherein the surfactant is an ionic surfactant or a nonionic surfactant.
[B36] The method according to any one of [B29] to [B29-2], wherein the surfactant is an ionic surfactant.
[B37] The method according to [B36], wherein the ionic surfactant is one or more selected from the group consisting of a cationic surfactant, an anionic surfactant, and an amphoteric surfactant.
[B38] The method according to [B36], wherein the ionic surfactant is one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, and a lecithin.
[B39] The method according to [B36], wherein the ionic surfactant is an alkyl sulfate.
[B40] The method according to [B36], wherein the ionic surfactant is a lauryl sulfate.
[B41] The method according to [B36], wherein the ionic surfactant is sodium lauryl sulfate.
[B42] The method according to any one of [B29] to [B29-2], wherein the surfactant is a nonionic surfactant.
[B43] The method according to [B42], wherein the nonionic surfactant is one or more selected from the group consisting of an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester.
[B44] The method according to [B42], wherein the nonionic surfactant is one or more selected from the group consisting of an alkyl aryl polyoxyethylene ether, a polyoxyethylene glycerin fatty acid ester, and a polyoxyethylene sorbitan fatty acid ester.
[B45] The method according to [B42], wherein the nonionic surfactant is one or more selected from the group consisting of 4-(1,1,3,3-tetramethylbutyl)phenyl-polyethylene glycol, Polyoxyl 35 Hydrogenated Castor Oil, and polyoxyethylene sorbitan monolaurate.
[B46] The method according to any one of [B29] to [B45], wherein the water-soluble organic solvent is an alcohol-based solvent, an amide-based solvent, a nitrile-based solvent, or a sulfoxide-based solvent.
[B47] The method according to any one of [B29] to [B45], wherein the water-soluble organic solvent is an alcohol-based solvent or a sulfoxide-based solvent.
[B48] The method according to any one of [B29] to [B45], wherein the water-soluble organic solvent is methanol, ethanol, 1-propanol, 2-propanol, or dimethyl sulfoxide.
[B49] The method according to any one of [B29] to [B45], wherein the water-soluble organic solvent is ethanol or dimethyl sulfoxide.
[B50] The method according to any one of [B29] to [B49], wherein a content of the surfactant is 0.02 to 20 wt/v%, 0.05 to 15 wt/v%, 0.1 to 10 wt/v%, 0.12 to 8 wt/v%, 0.15 to 5 wt/v%, or 0.18 to 3 wt/v% based on a total amount of the solvent, and a content of the water-soluble organic solvent is 5 to 40 v/v%, 5 to 30 v/v%, 5 to 25 v/v%, 8 to 20 v/v%, or 10 to 15 v/v% based on a total amount of the solvent.
[B51] The method according to any one of [B29] to [B49], wherein a content of the surfactant is 0.1 to 10 wt/v% based on a total amount of the solvent, and a content of the water-soluble organic solvent is 5 to 25 v/v% based on a total amount of the solvent.
[B52] A method for producing cyclic peptide crystals, the method comprising a step of bringing a cyclic peptide into contact with a solvent, wherein the cyclic peptide is a cyclic peptide having the following characteristics (I) and (II):
   (I) a characteristic of containing a cyclic moiety composed of 8 to 16 residues of amino acids in total, with a total number of amino acids being 8 to 20 residues; and
   (II) a characteristic of containing at least 2 residues of N-substituted amino acids, and
   the solvent is (i) a PEG-based solvent, or (ii) a mixed solvent containing one or more selected from the group consisting of an alcohol-based solvent, an aliphatic hydrocarbon-based solvent, and water, and a PEG-based solvent.
[B52-1] The method according to [B52], wherein the crystals have one or more diffraction peaks in powder X-ray diffraction using CuKα radiation, or have polarization in observation using a polarization microscope.
[B52-2] The method according to [B52], wherein the crystals have one or more diffraction peaks in powder X-ray diffraction using CuKα radiation.
[B53] The method according to any one of [B52] to [B52-2], wherein the solvent is a PEG-based solvent.
[B54] The method according to any one of [B52] to [B52-2], wherein the solvent is a mixed solvent of one or more selected from the group consisting of an alcohol-based solvent, an aliphatic hydrocarbon-based solvent, and water, and a PEG-based solvent.
[B55] The method according to [B53] or [B54], wherein the PEG-based solvent is (i) a solvent represented by R¹(OCHR³CH₂)nOR², n being a natural number of 1 or more and 10 or less, or (ii) a mixture of solvents represented by R¹(OCHR³CH₂)nOR², an average of n being 3 to 100, where R¹ and R² are each independently hydrogen, a C₁ to C₄ alkyl, or -C(=O)R⁴, R³ is hydrogen or a C₁ to C₄ alkyl, and R⁴ is a C₁ to C₁₈ alkyl optionally substituted with a hydroxy group or a C₁ to C₁₈ alkenyl optionally substituted with a hydroxy group.
[B56] The method according to [B53] or [B54], wherein the PEG-based solvent is (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or a C₁ to C₄ alkyl, R² is hydrogen, a C₁ to C₄ alkyl, or -C(=O)R⁴, R³ is hydrogen or a C₁ to C₄ alkyl, and R⁴ is a C₁₀ to C₁₈ alkyl.
[B57] The method according to [B53] or [B54], wherein the PEG-based solvent is (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or methyl, R² is hydrogen, methyl, or -C(=O)R⁴, R³ is hydrogen or methyl, and R⁴ is a C₁₁ to C₁₇ alkyl.
[B58] The method according to [B53] or [B54], wherein the PEG-based solvent is diglyme, triglyme, tetraglyme, ethylene glycol, diethylene glycol, triethylene glycol, tetraethylene glycol, polyethylene glycol, polypropylene glycol, or a polyethylene glycol monofatty acid ester.
[B59] The method according to [B53] or [B54], wherein the PEG-based solvent is polyethylene glycol.
[B59-2] The method according to [B59], wherein the polyethylene glycol has a number average molecular weight of 150 to 5000.
[B59-3] The method according to [B59], wherein the polyethylene glycol has a number average molecular weight of 360 to 440, 540 to 660, 900 to 1100, or 1800 to 2200.
[B60] The method according to [B59], wherein the polyethylene glycol is PEG400, PEG600, PEG1000, or PEG2000.
[B61] The method according to [B53] or [B54], wherein the PEG-based solvent is a polyethylene glycol monofatty acid ester.
[B62] The method according to [B61], wherein the polyethylene glycol monofatty acid ester is polyethylene glycol monostearate or polyethylene glycol monolaurate.
[B63] The method according to any one of [B52] to [B52-2] or [B54] to [B62], wherein the one or more selected from the group consisting of an alcohol-based solvent, an aliphatic hydrocarbon-based solvent, and water are one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, benzyl alcohol, n-pentane, n-hexane, n-heptane, n-octane, cyclopentane, cyclohexane, methylcycloheptane, and water.
[B64] The method according to any one of [B52] to [B52-2] or [B54] to [B62], wherein the one or more selected from the group consisting of an alcohol-based solvent, an aliphatic hydrocarbon-based solvent, and water are one or more selected from the group consisting of 2-propanol, n-heptane, and water.
[C1] The method according to any one of [A1] to [A89] or [B1] to [B64], wherein, in the step of bringing a cyclic peptide into contact with a solvent, a concentration of the cyclic peptide is 1 mg to 2000 mg/mL.
[C2] The method according to any one of [A1] to [A89] or [B1] to [B64], wherein, in the step of bringing a cyclic peptide into contact with a solvent, the concentration of the cyclic peptide is 5 mg to 1500 mg/mL, 10 mg to 1000 mg/mL, 10 mg to 500 mg/mL, 10 mg to 100 mg/mL, 10 mg to 50 mg/mL, 100 mg to 400 mg/mL, 100 mg to 200 mg/mL, or 500 mg to 1000 mg/mL.
[C3] The method according to any one of [A1] to [A89], [B1] to [B64], or [C1] to [C2], wherein the cyclic peptide used in the step of bringing a cyclic peptide into contact with a solvent is a freeze-dried product.
[C4] The method according to any one of [A1] to [A89], [B 1] to [B64], or [C1] to [C2], wherein the cyclic peptide used in the step of bringing a cyclic peptide into contact with a solvent is a freeze-dried product from a dimethyl sulfoxide solution.
[C5] The method according to any one of [A1] to [A89], [B 1] to [B64], or [C1] to [C4], wherein the cyclic peptide used in the step of bringing a cyclic peptide into contact with a solvent is 0.5 mg to 200 kg.
[C6] The method according to any one of [A1] to [A89], [B 1] to [B64], or [C1] to [C4], wherein the cyclic peptide used in the step of bringing a cyclic peptide into contact with a solvent is 1 mg to 1 g.
[C7] The method according to any one of [A1] to [A89], [B1] to [B64], or [C1] to [C4], wherein the cyclic peptide used in the step of bringing a cyclic peptide into contact with a solvent is 0 g to 200 kg, 100 g to 100 kg, 0.5 mg to 10 g, 1 mg to 1g, 1 mg to 100 mg, 1 mg to 10 mg, or 1 mg to 5 mg.
[C8] The method according to any one of [A1] to [A89], [B1] to [B64], or [C1] to [C7], wherein the step of bringing a cyclic peptide into contact with a solvent does not comprise adding seed crystals.
[C9] The method according to any one of [A1] to [A89], [B1] to [B64], or [C1] to [C7], wherein the step of bringing a cyclic peptide into contact with a solvent comprises adding seed crystals.
[C10] The method according to any one of [A1] to [A89], [B1] to [B64], or [C1] to [C9], further comprising a filtration step after the step of bringing a cyclic peptide into contact with a solvent.
[C11] The method according to any one of [A1] to [A89], [B1] to [B64], or [C1] to [C10], wherein the step of bringing a cyclic peptide into contact with a solvent is performed at a temperature of -10°C to 120°C for 30 minutes to 12 weeks.
[C12] The method according to any one of [A1] to [A89], [B1] to [B64], or [C1] to [C10], wherein the step of bringing a cyclic peptide into contact with a solvent is performed at a constant temperature in 0°C to 110°C, 10°C to 100°C, 15°C to 90°C, or 20°C to 80°C, or performed repeating heating and cooling between a lower limit temperature of 10°C, 20°C, 30°C, 40°C, 45°C, 50°C, or 55°C and an upper limit temperature of 100°C, 90°C, 85°C, 80°C, 75°C, 70°C, or 60°C, 10 times or more, 20 times or more, or 30 times or more, and 1000 times or less, 500 times or less, or 100 times or less, and performed for 1 hour to 6 weeks, 2 hours to 4 weeks, 4 hours to 2 weeks, or 6 hours to 7 days.
[C13] The method according to any one of [A1] to [A89], [B1] to [B64], or [C1] to [C10], wherein the step of bringing a cyclic peptide into contact with a solvent is performed at a constant temperature in 20°C to 90°C for 12 hours to 7 days.
[C14] The method according to any one of [A1] to [A89], [B1] to [B64], or [C1] to [C10], wherein the step of bringing a cyclic peptide into contact with a solvent is performed repeating heating and cooling between a lower limit temperature of 50°C and an upper limit temperature of 90°C, 30 times or more and 100 times or less, and performed for 12 hours to 7 days.
[D1] The method according to any one of [A1] to [A89], [B1] to [B64], or [C1] to [C14], wherein the cyclic peptide contains a cyclic moiety composed of 7 to 15 residues, 8 to 14 residues, 9 to 13 residues, 10 to 13 residues, 11 to 13 residues, 11 to 12 residues, or 11 residues of amino acids in total, with a total number of amino acids being 9 to 18 residues, 10 to 16 residues, 10 to 14 residues, 11 to 14 residues, 11 to 13 residues, 11 to 12 residues, or 11 residues.
[D2] The method according to any one of [A1] to [A89], [B1] to [B64], or [C1] to [C14], wherein the cyclic peptide contains a cyclic moiety composed of 11 to 13 residues of amino acids in total, with a total number of amino acids being 11 to 14 residues.
[D2-1] The method according to any one of [A1] to [A89], [B1] to [B64], or [C1] to [C14], wherein the cyclic peptide contains a cyclic moiety composed of 11 residues of amino acids in total, with a total number of amino acids being 11 residues.
[D3] The method according to any one of [A1] to [A89], [B1] to [B64], [C1] to [C14], or [D1] to [D2], wherein the cyclic peptide contains at least 3 residues, 4 residues, or 5 residues of N-substituted amino acids.
[D3-1] The method according to any one of [A1] to [A89], [B1] to [B64], [C1] to [C14], or [D1] to [D3], wherein the cyclic peptide contains at least 1 residue, 2 residues, or 3 residues of N-unsubstituted amino acids.
[D4] The method according to any one of [A1] to [A89], [B1] to [B64], [C1] to [C14], or [D1] to [D2], wherein the cyclic peptide contains at least 5 residues of N-substituted amino acids.
[D4-1] The method according to any one of [A1] to [A89], [B1] to [B64], [C1] to [C14], or [D1] to [D4], wherein the cyclic peptide contains at least 3 residues of N-unsubstituted amino acids.
[D4-2] The method according to any one of [A1] to [A89], [B1] to [B64], [C1] to [C14], or [D1] to [D4-1], wherein the N-substituted amino acid is a N-alkylamino acid.
[D4-3] The method according to any one of [A1] to [A89], [B1] to [B64], [C1] to [C14], or [D1] to [D4-1], wherein the N-substituted amino acid is a N-methylamino acid or a N-ethylamino acid.
[D4-4] The method according to any one of [A1] to [A89], [B1] to [B64], [C1] to [C14], or [D1] to [D4-1], wherein the N-substituted amino acid is a N-methylamino acid.
[D5] The method according to any one of [A1] to [A89], [B1] to [B64], [C1] to [C14], or [D1] to [D4-4], wherein the cyclic peptide contains at least one β-amino acid skeleton.
[D6] The method according to any one of [A1] to [A89], [B1] to [B64], [C1] to [C14], or [D1] to [D4-4], wherein the cyclic peptide contains at least one β-amino acid skeleton in the cyclic moiety.
[D7] The method according to any one of [A1] to [A89], [B1] to [B64], [C1] to [C14], or [D1] to [D6], wherein the cyclic peptide contains a cyclic moiety composed of a 28- to 55-, 28- to 49-, 31- to 46-, 34- to 43-, 34- to 40-, 34- to 37-, or 34-membered ring.
[D8] The method according to any one of [A1] to [A89], [B1] to [B64], [C1] to [C14], or [D1] to [D6], wherein the cyclic peptide contains a cyclic moiety composed of a 34- to 40-membered ring.
[D8-1] The method according to any one of [A1] to [A89], [B1] to [B64], [C1] to [C14], or [D1] to [D6], wherein the cyclic peptide contains a cyclic moiety composed of a 34-membered ring.
[D8-2] The method according to [D8-1], wherein the cyclic moiety composed of a 34-membered ring has the following cyclic structure, which is a cyclic peptide composed of 10 residues of α-amino acids and 1 residue of an amino acid having a β-amino acid skeleton, with a total of 11 residues of amino acids.
[D8-3] The method according to any one of [A1] to [A89], [B1] to [B64], [C1] to [C14], or [D1] to [D6], wherein the cyclic peptide is the following structure: 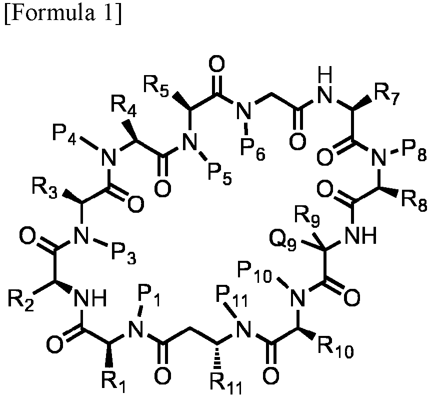 wherein P₁, P₃, P₅, P₆, P₁₀, and P₁₁ are each a C₁ to C₆ alkyl; P₄ is a C₁ to C₆ alkyl or P₄ forms a 4- to 7-membered saturated heterocyclic ring together with the nitrogen atom to which P₄ is bonded, R₄, and the carbon atom to which R₄ is bonded; P₈ is a C₁ to C₆ alkyl or P₈ forms a 4- to 7-membered saturated heterocyclic ring together with the nitrogen atom to which P₈ is bonded, R₈, and the carbon atom to which R₈ is bonded, the 4- to 7-membered saturated heterocyclic ring being optionally substituted by a C₁ to C₆ alkoxy; R₁, R₂, R₃, R₅, R₇, and R₁₀ are each a hydrogen atom, a C₁ to C₆ alkyl, a C₃ to C₆ cycloalkyl, or an aralkyl optionally having a substituent; R₄ is a hydrogen atom or a C₁ to C₆ alkyl, except when R₄ and P₄ form a 4- to 7-membered saturated heterocyclic ring; R₈ is a hydrogen atom or a C₁ to C₆ alkyl, except when R₈ and P₈ form a 4- to 7-membered saturated heterocyclic ring; R₉ forms a 3- to 7-membered saturated carbocyclic ring together with Q₉ and the carbon atom to which R₉ and Q₉ are bonded; and R₁₁ is a hydrogen atom, a C₁ to C₆ alkyl, a di-C₁ to C₆ alkylaminocarbonyl, or a 4- to 8-membered cyclic aminocarbonyl.
[D8-4] The method according to [D8-3], wherein P₁, P₃, P₅, P₆, P₁₀, and P₁₁ are each methyl or ethyl.
[D8-5] The method according to [D8-3] or [D8-4], wherein P₄ is methyl or forms a 4-membered saturated heterocyclic ring together with the nitrogen atom to which P₄ is bonded, R₄, and the carbon atom to which R₄ is bonded.
[D8-6] The method according to any one of [D8-3] to [D8-5], wherein P₈ forms a 5-membered saturated heterocyclic ring together with the nitrogen atom to which P₈ is bonded, R₈, and the carbon atom to which R₈ is bonded, the 5-membered saturated heterocyclic ring being optionally substituted by a C₁ to C₆ alkoxy.
[D8-7] The method according to any one of [D8-3] to [D8-6], wherein R₁ and R₂ are each a C₁ to C₆ alkyl; R₃ is a hydrogen atom or a C₁ to C₆ alkyl; R₄ is a hydrogen atom, except when R₄ and P₄ form a 4- to 7-membered saturated heterocyclic ring; R5 is a C₃ to C₆ cycloalkyl or benzyl optionally having a substituent; R₇ is phenylethyl optionally having a substituent; R₉ forms a 5-membered saturated carbocyclic ring together with Q₉ and the carbon atom to which R₉ and Q₉ are bonded; R₁₀ is a C₁ to C₆ alkyl or a C₃ to C₆ cycloalkyl; and R₁₁ is methyl, a di-C₁ to C₆ alkylaminocarbonyl, or a 6-membered cyclic aminocarbonyl.
[D9] The method according to any one of [A1] to [A89], [B1] to [B64], [C1] to [C14], or [D1] to [D8-7], wherein the cyclic peptide has a molecular weight (g/mol) of 1205 or more, 1206 or more, 1207 or more, 1208 or more, 1210 or more, 1220 or more, 1230 or more, 1250 or more, or 1300 or more, and 2800 or less, 2500 or less, 2000 or less, 1900 or less, 1800 or less, 1700 or less, or 1600 or less.
[D10] The method according to any one of [A1] to [A89], [B1] to [B64], [C1] to [C14], or [D1] to [D8-7], wherein the cyclic peptide has a molecular weight (g/mol) of 1300 or more and 1600 or less.
[D11] The method according to any one of [A1] to [A89], [B1] to [B64], [C1] to [C14], or [D1] to [D10], wherein the cyclic peptide has a ClogP of 4 or more and 25 or less.
[D12] The method according to any one of [A1] to [A89], [B1] to [B64], [C1] to [C14], or [D1] to [D10], wherein the cyclic peptide has a ClogP of 5 or more, 6 or more, 7 or more, 8 or more, or 9 or more, and 24 or less, 23 or less, 22 or less, 21 or less, 20 or less, 19 or less, or 18 or less.
[D13] The method according to any one of [A1] to [A89], [B1] to [B64], [C1] to [C14], or [D1] to [D10], wherein the cyclic peptide has a ClogP of 9 or more and 18 or less.
[D14] The method according to any one of [A1] to [A89], [B1] to [B64], [C1] to [C14], or [D1] to [D 10], wherein a ClogP of the cyclic peptide is larger than a ClogP of cyclosporin A.
[D14-1] The method according to any one of [A1] to [A89], [B1] to [B64], [C1] to [C14], or [D1] to [D14], wherein the cyclic peptide has a ClogP/number of amino acid residues of 0.3 or more and 2.3 or less, 0.5 or more and 1.9 or less, 0.7 or more and 1.8 or less, or 0.8 or more and 1.6 or less.
[D 14-2] The method according to any one of [A1] to [A89], [B1] to [B64], [C1] to [C14], or [D1] to [D14], wherein the cyclic peptide has a ClogP/number of amino acid residues of 0.8 or more and 1.6 or less.
[D14-3] The method according to any one of [A1] to [A89], [B1] to [B64], [C1] to [C14], or [D1] to [D14-2], wherein the cyclic peptide has a solubility of 1200 mg/mL or less, 800 mg/mL or less, 600 mg/mL or less, 300 mg/mL or less, 200 mg/mL or less, 100 mg/mL or less, 50 mg/mL or less, 25 mg/mL or less, 10 mg/mL or less, 5.0 mg/mL or less, or 2.6 mg/mL or less in a 50 mM phosphate buffer solution (pH 6.5).
[D14-4] The method according to any one of [A1] to [A89], [B1] to [B64], [C1] to [C14], or [D1] to [D14-2], wherein the cyclic peptide has a solubility of 0.8 mg/mL or more in a 50 mM phosphate buffer solution (pH 6.5).
[D14-5] The method according to any one of [A1] to [A89], [B1] to [B64], [C1] to [C14], or [D1] to [D14-2], wherein the cyclic peptide has a solubility of 0.9 mg/mL or more, 1.0 mg/mL or more, or 1.1 mg/mL or more in a 50 mM phosphate buffer solution (pH 6.5).
[D14-6] The method according to any one of [A1] to [A89], [B1] to [B64], [C1] to [C14], or [D1] to [D14-2], wherein the cyclic peptide has a solubility of 1.1 mg/mL or more in a 50 mM phosphate buffer solution (pH 6.5).
[D14-7] The method according to any one of [A1] to [A89], [B1] to [B64], [C1] to [C14], or [D1] to [D14-2], having two or more diffraction peaks in powder X-ray diffraction using CuKα radiation.
[D14-8] The method according to any one of [A1] to [A89], [B1] to [B64], [C1] to [C14], or [D1] to [D14-2], having three or more diffraction peaks in powder X-ray diffraction using CuKα radiation.
[D15] The method according to any one of [A1] to [A89], [B1] to [B64], [C1] to [C14], or [D1] to [D14-4], wherein the cyclic peptide is a cyclic peptide excluding cyclosporin A.
[D16] The method according to any one of [A1] to [A89], [B1] to [B64], [C1] to [C14], or [D1] to [D15], wherein the cyclic peptide is a cyclic peptide excluding (3s,9s,12s,17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatiicyclo[34.3.0.0^{12,15}]nonatiiacontane-33,1'-cyclopentane]-27-carboxamide. [E1] A method for screening cyclic peptide crystals, the method comprising the following steps (a) and (b):
   (a) a step of producing cyclic peptide crystals by the method according to any one of [A1] to [A89], [B1] to [B64], [C1] to [C14], or [D1] to [D16]; and
   (b) a step of analyzing the crystals by powder X-ray crystal diffraction.
[E2] The method according to [E1], wherein the crystals to be screened include crystals produced using a solvent containing formamide.
[E3] The method according to [E1], wherein the crystals to be screened include crystals produced using a solvent containing formamide and a solvent containing dimethyl sulfoxide.
[E4] The method according to [E1], wherein the crystals to be screened include crystals produced using a solvent containing formamide, a solvent containing dimethyl sulfoxide, a solvent containing toluene, and a solvent containing dichloromethane.
[E5] The method according to [E1], wherein the crystals to be screened include crystals produced using a solvent containing Polyoxyl 35 Hydrogenated Castor Oil.
[E6] The method according to [E1], wherein the crystals to be screened include crystals produced using a solvent containing a polyethylene glycol monofatty acid ester.
[E7] The method according to [E1], wherein the crystals to be screened include crystals produced using a solvent containing polyethylene glycol.
[E8] The method according to [E1], wherein the crystals to be screened include crystals produced using a solvent containing Polyoxyl 35 Hydrogenated Castor Oil, a solvent containing a polyethylene glycol monofatty acid ester, and a solvent containing polyethylene glycol.
[E9] The method according to [E1], wherein the crystals to be screened include crystals produced using a solvent containing Polyoxyl 35 Hydrogenated Castor Oil, crystals produced using a solvent containing a polyethylene glycol monofatty acid ester, and crystals produced using a solvent containing formamide.
[F1] A method for screening a method for crystallizing a cyclic peptide, the method comprising the following steps (a) and (b):
   (a) a step of producing cyclic peptide crystals by the method according to any one of [A1] to [A89], [B1] to [B64], [C1] to [C14], or [D1] to [D16]; and
   (b) a step of analyzing the crystals by powder X-ray crystal diffraction.
[F2] The method according to [F1], wherein the crystallization method to be screened includes a crystallization method using a solvent containing formamide.
[F3] The method according to [F1], wherein the crystallization method to be screened includes a crystallization method using a solvent containing formamide and a solvent containing dimethyl sulfoxide.
[F4] The method according to [F1], wherein the crystallization method to be screened includes a crystallization method using a solvent containing formamide, a solvent containing dimethyl sulfoxide, a solvent containing toluene, and a solvent containing dichloromethane.
[F5] The method according to [F1], wherein the crystallization method to be screened includes a crystallization method using a solvent containing Polyoxyl 35 Hydrogenated Castor Oil.
[F6] The method according to [F1], wherein the crystallization method to be screened includes a crystallization method using a solvent containing a polyethylene glycol monofatty acid ester.
[F7] The method according to [F1], wherein the crystallization method to be screened includes a crystallization method using a solvent containing polyethylene glycol.
[F8] The method according to [F1], wherein the crystallization method to be screened includes a crystallization method using a solvent containing Polyoxyl 35 Hydrogenated Castor Oil, a solvent containing a polyethylene glycol monofatty acid ester, and a solvent containing polyethylene glycol.
[F9] The method according to [F1], wherein the crystallization method to be screened includes a crystallization method using a solvent containing Polyoxyl 35 Hydrogenated Castor Oil, a solvent containing a polyethylene glycol monofatty acid ester, and a solvent containing formamide.
[F10] The method according to any one of [E1] to [E9] or [F1] to [F9], wherein, in the step of bringing a cyclic peptide into contact with a solvent, the cyclic peptide used per condition is 0.5 mg to 10 mg.
[F11] The method according to any one of [E1] to [E9] or [F1] to [F10], wherein the crystals or crystallization method to be screened is of 2 or more types, 5 or more types, 10 or more types, 15 or more types, or 20 or more types.
[F12] The method according to any one of [E1] to [E9] or [F1] to [F11], wherein the step of bringing a cyclic peptide into contact with a solvent does not comprise adding seed crystals.
[G1] A method for increasing the possibility that cyclic peptide crystals are produced, the method comprising a step of producing cyclic peptide crystals by the method according to any one of [A1] to [A89], [B1] to [B64], [C1] to [C14], or [D1] to [D16].
[H1] Use of any solvent selected from the group consisting of an amide-based solvent, a sulfoxide-based solvent, an aromatic hydrocarbon-based solvent, a halogen-based solvent, a polyoxyethylene glycerin fatty acid ester, and a polyethylene glycol monofatty acid ester in production of cyclic peptide crystals.
[H2] Use of an amide-based solvent in production of cyclic peptide crystals.
[H3] Use of formamide in production of cyclic peptide crystals.
[H4] Use of a sulfoxide-based solvent in production of cyclic peptide crystals.
[H5] Use of dimethyl sulfoxide in production of cyclic peptide crystals.
[H6] Use of an aromatic hydrocarbon-based solvent in production of cyclic peptide crystals.
[H7] Use of toluene, tetralin, or cumene in production of cyclic peptide crystals.
[H8] Use of Polyoxyl 35 Hydrogenated Castor Oil in production of cyclic peptide crystals.
[H9] Use of a polyethylene glycol monofatty acid ester in production of cyclic peptide crystals.
[H10] Use of polyethylene glycol in production of cyclic peptide crystals.
[H11] The use according to any one of [H1] to [H10], wherein the cyclic peptide crystals are produced by the method according to any one of [D1] to [D16].
[I1] A method for purifying a cyclic peptide, the method comprising a step of producing cyclic peptide crystals by the method according to any one of [A1] to [A89], [B1] to [B64], [C1] to [C14], or [D1] to [D16], and a step of collecting the crystals by solid-liquid separation.
[J1] A screening kit for producing cyclic peptide crystals, wherein cyclic peptide crystals are produced by the use according to any one of [H1] to [H11].
[K1] A method for producing a cyclic peptide, the method comprising a step of producing cyclic peptide crystals containing formamide.
[K2] A method for producing a cyclic peptide, the method comprising a step of producing cyclic peptide crystals containing dimethyl sulfoxide.
[K3] A method for producing a cyclic peptide, the method comprising a step of producing cyclic peptide crystals containing an aromatic hydrocarbon-based solvent.
[K4] A method for producing a cyclic peptide, the method comprising a step of producing cyclic peptide crystals containing toluene, tetralin, or cumene.
[K5] A method for producing a cyclic peptide, the method comprising a step of producing cyclic peptide crystals containing Polyoxyl 35 Hydrogenated Castor Oil.
[K6] A method for producing a cyclic peptide, the method comprising a step of producing cyclic peptide crystals containing a polyethylene glycol monofatty acid ester.
[K7] A method for producing a cyclic peptide, the method comprising a step of producing cyclic peptide crystals containing polyethylene glycol.
[K8] The method according to any one of [K1] to [K7], wherein cyclic peptide crystals are produced by the method according to any one of [A1] to [A89], [B1] to [B64], [C1] to [C14], or [D1] to [D16].
[L1] A method for producing crystals of (3s,9s,12s,17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide, the method comprising a step of bringing (3s,9s,12s,17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide into contact with a solvent, wherein the solvent is solvent (A) or a mixed solvent containing solvent (A) and solvent (B), the solvent (A) is one or more selected from the group consisting of an amide-based solvent, a sulfoxide-based solvent, an aromatic hydrocarbon-based solvent, a halogen-based solvent, and an ester-based solvent, and the solvent (B) is one or more selected from the group consisting of an aliphatic hydrocarbon-based solvent, ethylene glycol, and water.
[L2] The method according to [L1], wherein the crystals have one or more diffraction peaks in powder X-ray diffraction using CuKα radiation, or have polarization in observation using a polarization microscope.
[L3] The method according to [L1], wherein the crystals have one or more diffraction peaks in powder X-ray diffraction using CuKα radiation.
[L4] The method according to any one of [L1] to [L3], wherein the solvent is toluene.
[L5] The method according to any one of [L1] to [L3], wherein the solvent is dimethyl sulfoxide.
[L6] The method according to any one of [L1] to [L3], wherein the solvent is a mixed solvent of ethanol and water.
[L7] The method according to any one of [L1] to [L3], wherein the solvent is a mixed solvent of acetonitrile and water.
[L8] The method according to any one of [L1] to [L3], wherein the solvent is cumene.
[L9] The method according to any one of [L1] to [L3], wherein the solvent is tetralin.
[L10] The method according to any one of [L1] to [L3], wherein the solvent is formamide.
[L11] The method according to any one of [L1] to [L3], wherein the solvent is diisopropyl ether.
[L12] The method according to any one of [L1] to [L3], wherein the solvent is a mixed solvent of 3-acetylpyridine and ethylene glycol.
[L13] A method for producing crystals of (3s,9s, 12s, 17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide, the method comprising a step of bringing (3s,9s,12s,17s,20s,23s,27s,30s,36s)-30-cyclopentyl-3-[2-[3,5-difluoro-4-(trifluoromethyl)phenyl]ethyl]-1 0-ethyl-23-isobutyl-N,N, 7,17, 18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide into contact with a solvent, wherein the solvent is water containing 0.01 to 30 wt/v% of a surfactant and 5 to 50 v/v% of a water-soluble organic solvent, based on a total amount of the solvent.
[L14] The method according to [L13], wherein the crystals have one or more diffraction peaks in powder X-ray diffraction using CuKα radiation, or have polarization in observation using a polarization microscope.
[L15] The method according to [L13], wherein the crystals have one or more diffraction peaks in powder X-ray diffraction using CuKα radiation.
[L16] The method according to any one of [L13] to [L15], wherein the surfactant is one or more selected from the group consisting of a cationic surfactant, an anionic surfactant, an amphoteric surfactant, and a nonionic surfactant.
[L17] The method according to any one of [L13] to [L15], wherein the surfactant is 4-(1,1,3,3-tetramethylbutyl)phenyl-polyethylene glycol and the water-soluble organic solvent is ethanol.
[L18] The method according to any one of [L13] to [L15], wherein the surfactant is 4-(1,1,3,3-tetramethylbutyl)phenyl-polyethylene glycol and the water-soluble organic solvent is dimethyl sulfoxide.
[L19] The method according to any one of [L13] to [L15], wherein the surfactant is Polyoxyl 35 Hydrogenated Castor Oil and the water-soluble organic solvent is ethanol.
[L20] The method according to any one of [L13] to [L15], wherein the surfactant is Polyoxyl 35 Hydrogenated Castor Oil and the water-soluble organic solvent is dimethyl sulfoxide.
[L21] The method according to any one of [L13] to [L15], wherein the surfactant is polyoxyethylene sorbitan monolaurate (Tween 80) and the water-soluble organic solvent is ethanol.
[L22] The method according to any one of [L13] to [L15], wherein the surfactant is polyoxyethylene sorbitan monolaurate and the water-soluble organic solvent is dimethyl sulfoxide.
[L23] The method according to any one of [L13] to [L15], wherein the surfactant is sodium lauryl sulfate and the water-soluble organic solvent is ethanol.
[L24] The method according to any one of [L13] to [L15], wherein the surfactant is sodium lauryl sulfate and the water-soluble organic solvent is dimethyl sulfoxide.
[L25] A method for producing crystals of (3s,9s, 12s, 17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide, the method comprising a step of bringing (3s,9s,12s,17s,20s,23s,27s,30s,36s)-30-cyclopentyl-3-[2-[3,5-difluoro-4-(trifluoromethyl)phenyl]ethyl]-1 0-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide into contact with a solvent, wherein the solvent is (i) a PEG-based solvent, or (ii) a mixed solvent containing one or more selected from the group consisting of an alcohol-based solvent, an aliphatic hydrocarbon-based solvent, and water, and a PEG-based solvent.
[L26] The method according to [L25], wherein the crystals have one or more diffraction peaks in powder X-ray diffraction using CuKα radiation, or have polarization in observation using a polarization microscope.
[L27] The method according to [L25], wherein the crystals have one or more diffraction peaks in powder X-ray diffraction using CuKα radiation.
[L28] The method according to any one of [L13] to [L15], wherein the solvent is tetraethylene glycol.
[L29] The method according to any one of [L13] to [L15], wherein the solvent is polyethylene glycol.
[L30] The method according to any one of [L13] to [L15], wherein the solvent is triglyme.
[L31] The method according to any one of [L13] to [L15], wherein the solvent is ethylene glycol.
[L32] The method according to any one of [L13] to [L15], wherein the solvent is PEG400.
[M1] Crystals produced by the method according to any one of [A1] to [A89], [B1] to [B64], [C1] to [C14], [D1] to [D16], [K1] to [K7], or [L32] to [L32].
[M2] A pharmaceutical composition comprising crystals produced by the method according to any one of [A1] to [A89], [B1] to [B64], [C1] to [C14], [D1] to [D16], [K1] to [K7], or [L32] to [L32].

### [Advantageous Effects of Invention]

According to the present invention, there is provided a method for producing crystals of a cyclic peptide containing a N-substituted amino acid residue. According to the present invention, there is also provide a method for screening crystals of a cyclic peptide containing a N-substituted amino acid residue. According to the present invention, there is also provide a method for screening a crystallization method. According to the present invention, there is also provide a method for isolating and purifying the target cyclic peptide or salt thereof, or solvate thereof, as crystals without resorting to column chromatography.

### [Brief Description of Drawings]

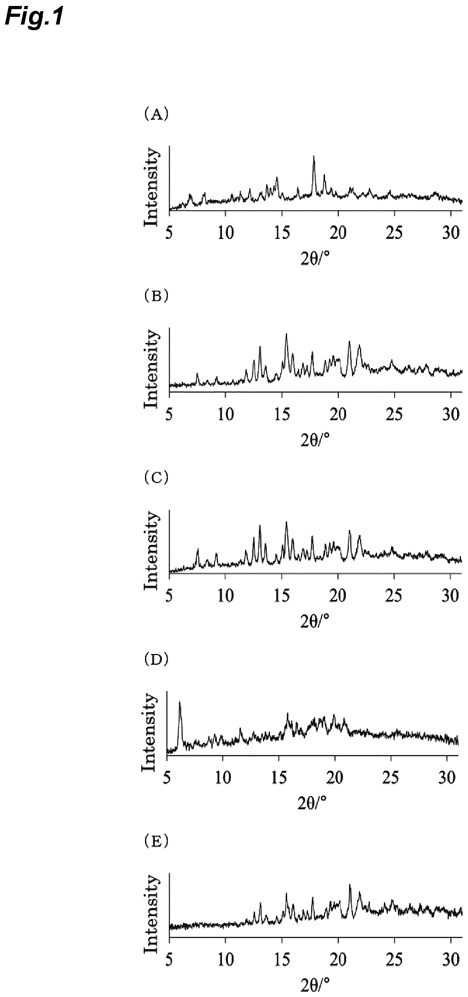

[Figure 1] Figure 1 (A) shows the results of powder X-ray diffraction measurement of the crystals obtained in Example 2-1-2. The ordinate represents diffraction intensity, and the abscissa represents diffraction angle 2θ (°). Figure 1 (B) shows the results of powder X-ray diffraction measurement of the crystals obtained in Example 2-2-1. The ordinate represents diffraction intensity, and the abscissa represents diffraction angle 2θ (°). Figure 1 (C) shows the results of powder X-ray diffraction measurement of the crystals obtained in Example 2-2-2. The ordinate represents diffraction intensity, and the abscissa represents diffraction angle 2θ (°). Figure 1 (D) shows the results of powder X-ray diffraction measurement of the crystals obtained in Example 2-2-3. The ordinate represents diffraction intensity, and the abscissa represents diffraction angle 2θ (°). Figure 1 (E) shows the results of powder X-ray diffraction measurement of the crystals obtained in Example 2-3-1. The ordinate represents diffraction intensity, and the abscissa represents diffraction angle 2θ (°).

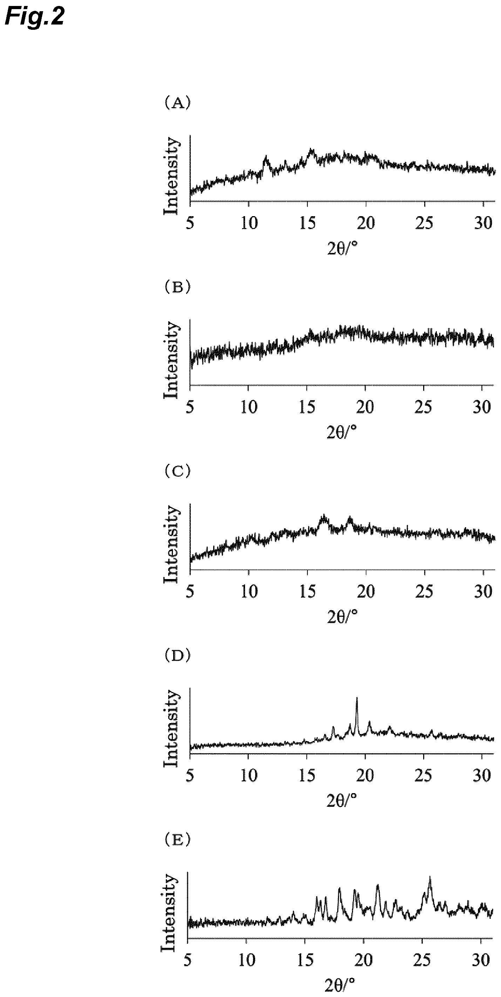

[Figure 2] Figure 2 (A) shows the results of powder X-ray diffraction measurement of the crystals obtained in Example 2-4-1. The ordinate represents diffraction intensity, and the abscissa represents diffraction angle 2θ (°). Figure 2 (B) shows the results of powder X-ray diffraction measurement of the crystals obtained in Example 2-4-2. The ordinate represents diffraction intensity, and the abscissa represents diffraction angle 2θ (°). Figure 2 (C) shows the results of powder X-ray diffraction measurement of the crystals obtained in Example 2-5-1. The ordinate represents diffraction intensity, and the abscissa represents diffraction angle 2θ (°). Figure 2 (D) shows the results of powder X-ray diffraction measurement of the crystals obtained in Example 2-5-2. The ordinate represents diffraction intensity, and the abscissa represents diffraction angle 2θ (°). Figure 2 (E) shows the results of powder X-ray diffraction measurement of the crystals obtained in Example 2-5-3. The ordinate represents diffraction intensity, and the abscissa represents diffraction angle 2θ (°).

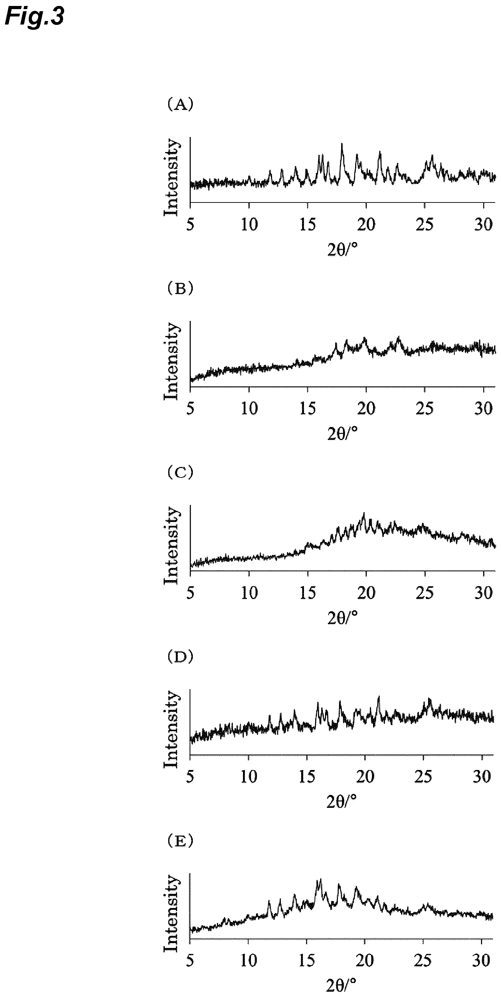

[Figure 3] Figure 3 (A) shows the results of powder X-ray diffraction measurement of the crystals obtained in Example 2-5-4. The ordinate represents diffraction intensity, and the abscissa represents diffraction angle 2θ (°). Figure 3 (B) shows the results of powder X-ray diffraction measurement of the crystals obtained in Example 2-5-5. The ordinate represents diffraction intensity, and the abscissa represents diffraction angle 2θ (°). Figure 3 (C) shows the results of powder X-ray diffraction measurement of the crystals obtained in Example 2-5-6. The ordinate represents diffraction intensity, and the abscissa represents diffraction angle 2θ (°). Figure 3 (D) shows the results of powder X-ray diffraction measurement of the crystals obtained in Example 2-5-7. The ordinate represents diffraction intensity, and the abscissa represents diffraction angle 2θ (°). Figure 3 (E) shows the results of powder X-ray diffraction measurement of the crystals obtained in Example 2-5-8. The ordinate represents diffraction intensity, and the abscissa represents diffraction angle 2θ (°).

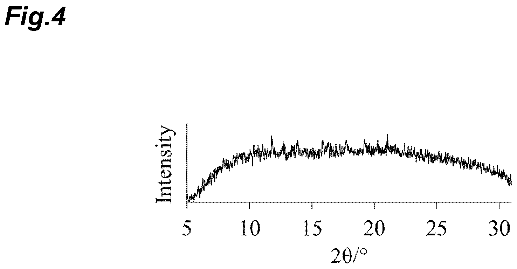

[Figure 4] Figure 4 shows the results of powder X-ray diffraction measurement of the crystals obtained in Example 2-5-9. The ordinate represents diffraction intensity, and the abscissa represents diffraction angle 2θ (°).

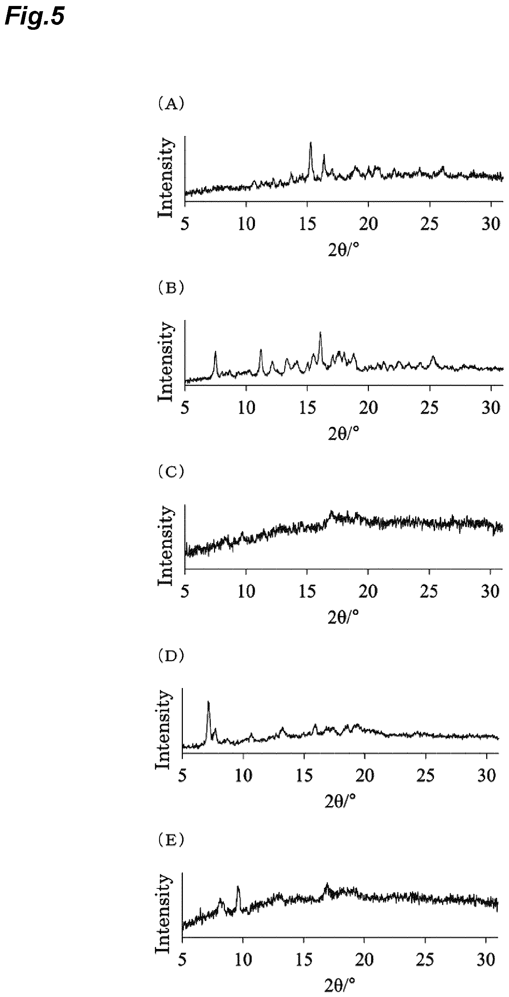

[Figure 5] Figure 5 (A) shows the results of powder X-ray diffraction measurement of the crystals obtained in Example 2-6-1. The ordinate represents diffraction intensity, and the abscissa represents diffraction angle 2θ (°). Figure 5 (B) shows the results of powder X-ray diffraction measurement of the crystals obtained in Example 2-7-1. The ordinate represents diffraction intensity, and the abscissa represents diffraction angle 2θ (°). Figure 5 (C) shows the results of powder X-ray diffraction measurement of the crystals obtained in Example 2-7-2. The ordinate represents diffraction intensity, and the abscissa represents diffraction angle 2θ (°). Figure 5 (D) shows the results of powder X-ray diffraction measurement of the crystals obtained in Example 2-7-3. The ordinate represents diffraction intensity, and the abscissa represents diffraction angle 2θ (°). Figure 5 (E) shows the results of powder X-ray diffraction measurement of the crystals obtained in Example 2-7-4. The ordinate represents diffraction intensity, and the abscissa represents diffraction angle 2θ (°).

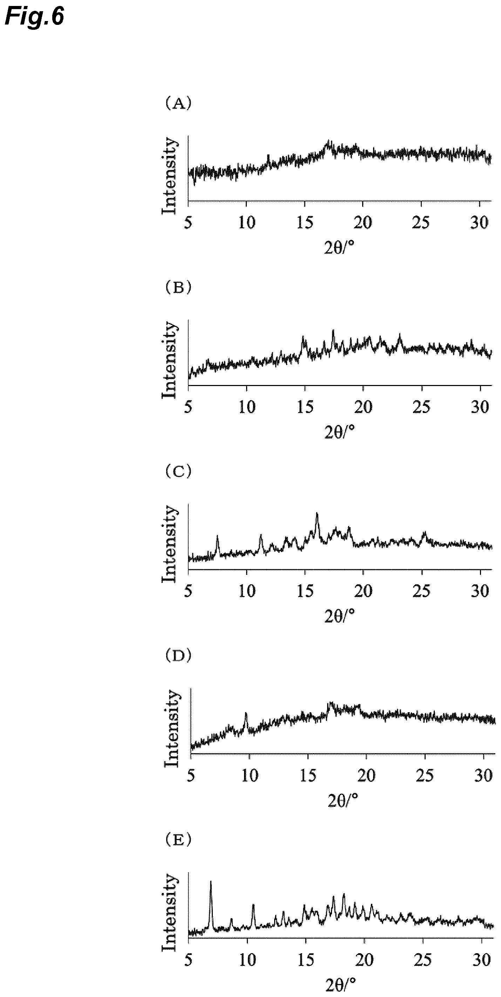

[Figure 6] Figure 6 (A) shows the results of powder X-ray diffraction measurement of the crystals obtained in Example 2-7-8. The ordinate represents diffraction intensity, and the abscissa represents diffraction angle 2θ (°). Figure 6 (B) shows the results of powder X-ray diffraction measurement of the crystals obtained in Example 2-7-9. The ordinate represents diffraction intensity, and the abscissa represents diffraction angle 2θ (°). Figure 6 (C) shows the results of powder X-ray diffraction measurement of the crystals obtained in Example 2-7-10. The ordinate represents diffraction intensity, and the abscissa represents diffraction angle 2θ (°). Figure 6 (D) shows the results of powder X-ray diffraction measurement of the crystals obtained in Example 2-7-12. The ordinate represents diffraction intensity, and the abscissa represents diffraction angle 2θ (°). Figure 6 (E) shows the results of powder X-ray diffraction measurement of the crystals obtained in Example 2-7-14. The ordinate represents diffraction intensity, and the abscissa represents diffraction angle 2θ (°).

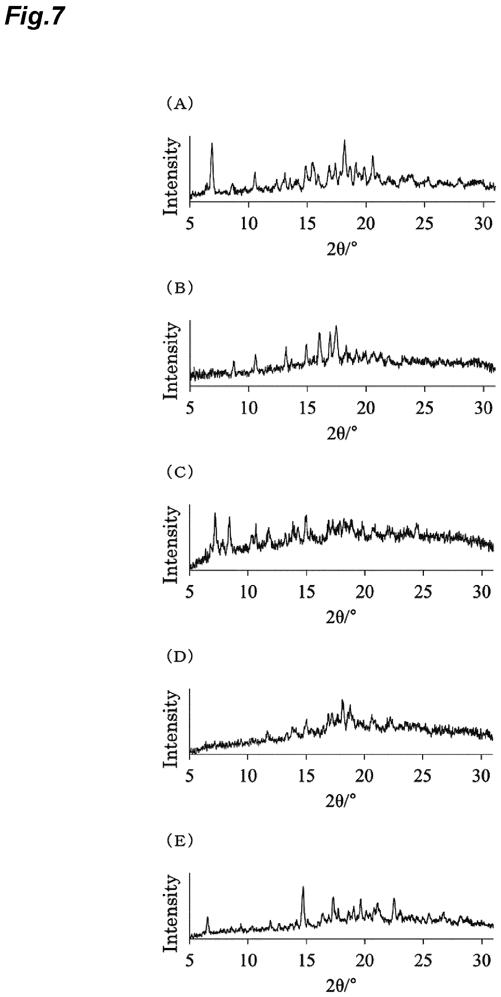

[Figure 7] Figure 7 (A) shows the results of powder X-ray diffraction measurement of the crystals obtained in Example 2-7-15. The ordinate represents diffraction intensity, and the abscissa represents diffraction angle 2θ (°). Figure 7 (B) shows the results of powder X-ray diffraction measurement of the crystals obtained in Example 2-7-16. The ordinate represents diffraction intensity, and the abscissa represents diffraction angle 2θ (°). Figure 7 (C) shows the results of powder X-ray diffraction measurement of the crystals obtained in Example 2-7-17. The ordinate represents diffraction intensity, and the abscissa represents diffraction angle 2θ (°). Figure 7 (D) shows the results of powder X-ray diffraction measurement of the crystals obtained in Example 2-7-18. The ordinate represents diffraction intensity, and the abscissa represents diffraction angle 2θ (°). Figure 7 (E) shows the results of powder X-ray diffraction measurement of the crystals obtained in Example 2-7-19. The ordinate represents diffraction intensity, and the abscissa represents diffraction angle 2θ (°).

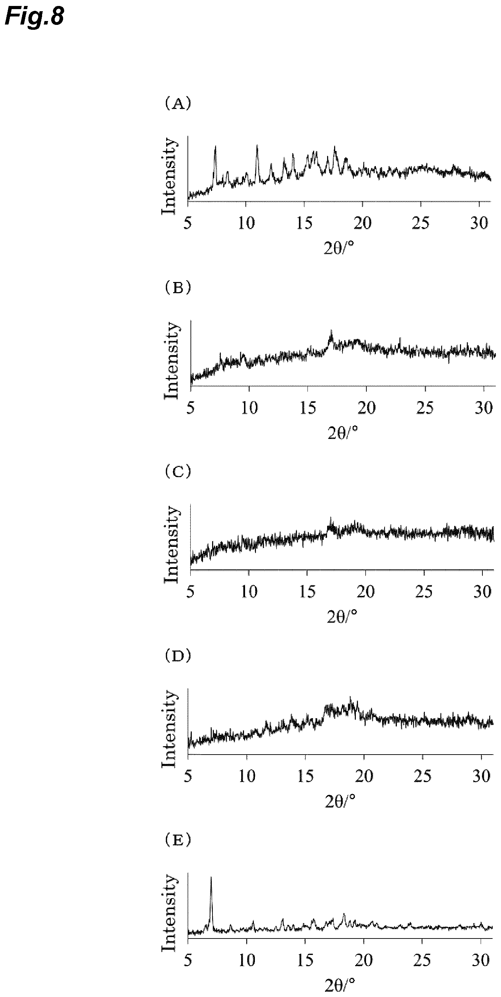

[Figure 8] Figure 8 (A) shows the results of powder X-ray diffraction measurement of the crystals obtained in Example 2-7-20. The ordinate represents diffraction intensity, and the abscissa represents diffraction angle 2θ (°). Figure 8 (B) shows the results of powder X-ray diffraction measurement of the crystals obtained in Example 2-7-21. The ordinate represents diffraction intensity, and the abscissa represents diffraction angle 2θ (°). Figure 8 (C) shows the results of powder X-ray diffraction measurement of the crystals obtained in Example 2-7-22. The ordinate represents diffraction intensity, and the abscissa represents diffraction angle 2θ (°). Figure 8 (D) shows the results of powder X-ray diffraction measurement of the crystals obtained in Example 2-7-23. The ordinate represents diffraction intensity, and the abscissa represents diffraction angle 2θ (°). Figure 8 (E) shows the results of powder X-ray diffraction measurement of the crystals obtained in Example 2-7-24. The ordinate represents diffraction intensity, and the abscissa represents diffraction angle 2θ (°).

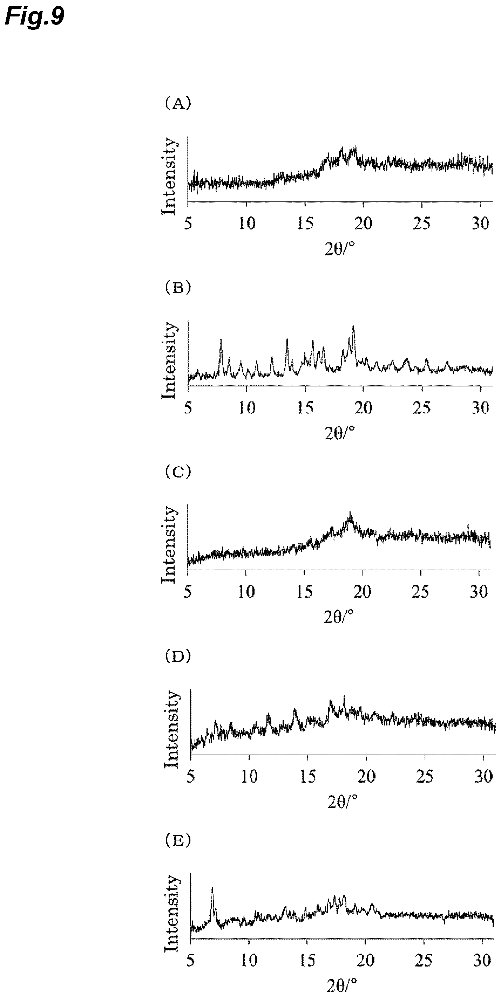

[Figure 9] Figure 9 (A) shows the results of powder X-ray diffraction measurement of the crystals obtained in Example 2-7-25. The ordinate represents diffraction intensity, and the abscissa represents diffraction angle 2θ (°). Figure 9 (B) shows the results of powder X-ray diffraction measurement of the crystals obtained in Example 2-7-26. The ordinate represents diffraction intensity, and the abscissa represents diffraction angle 2θ (°). Figure 9 (C) shows the results of powder X-ray diffraction measurement of the crystals obtained in Example 2-7-29. The ordinate represents diffraction intensity, and the abscissa represents diffraction angle 2θ (°). Figure 9 (D) shows the results of powder X-ray diffraction measurement of the crystals obtained in Example 2-7-31. The ordinate represents diffraction intensity, and the abscissa represents diffraction angle 2θ (°). Figure 9 (E) shows the results of powder X-ray diffraction measurement of the crystals obtained in Example 2-7-32. The ordinate represents diffraction intensity, and the abscissa represents diffraction angle 2θ (°).

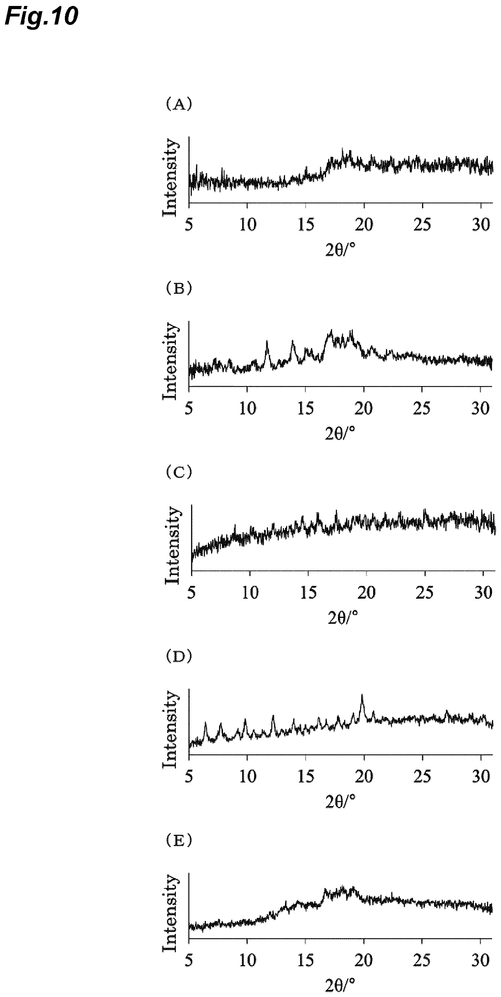

[Figure 10] Figure 10 (A) shows the results of powder X-ray diffraction measurement of the crystals obtained in Example 2-7-33. The ordinate represents diffraction intensity, and the abscissa represents diffraction angle 2θ (°). Figure 10 (B) shows the results of powder X-ray diffraction measurement of the crystals obtained in Example 2-7-34. The ordinate represents diffraction intensity, and the abscissa represents diffraction angle 2θ (°). Figure 10 (C) shows the results of powder X-ray diffraction measurement of the crystals obtained in Example 2-8-1. The ordinate represents diffraction intensity, and the abscissa represents diffraction angle 2θ (°). Figure 10 (D) shows the results of powder X-ray diffraction measurement of the crystals obtained in Example 2-8-3. The ordinate represents diffraction intensity, and the abscissa represents diffraction angle 2θ (°). Figure 10 (E) shows the results of powder X-ray diffraction measurement of the crystals obtained in Example 2-8-5. The ordinate represents diffraction intensity, and the abscissa represents diffraction angle 2θ (°).

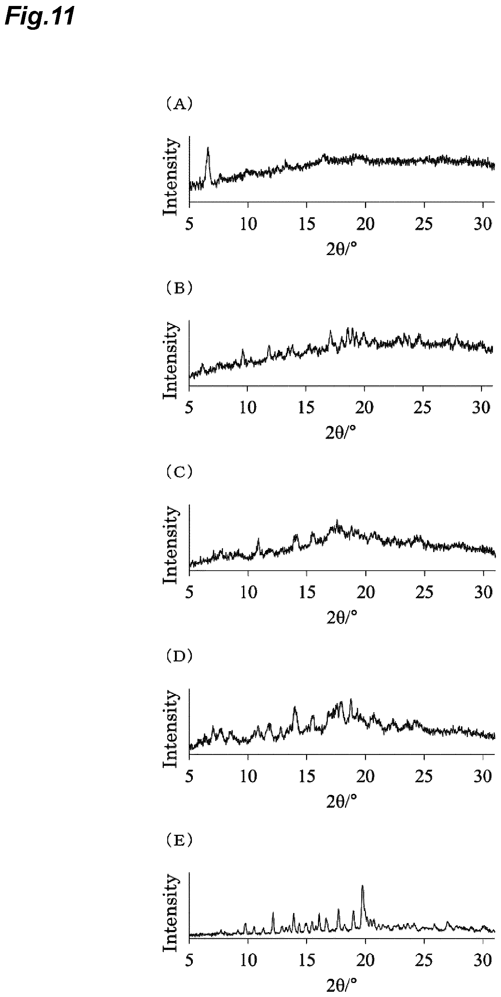

[Figure 11] Figure 11 (A) shows the results of powder X-ray diffraction measurement of the crystals obtained in Example 2-8-6. The ordinate represents diffraction intensity, and the abscissa represents diffraction angle 2θ (°). Figure 11 (B) shows the results of powder X-ray diffraction measurement of the crystals obtained in Example 2-8-10. The ordinate represents diffraction intensity, and the abscissa represents diffraction angle 2θ (°). Figure 11 (C) shows the results of powder X-ray diffraction measurement of the crystals obtained in Example 2-8-13. The ordinate represents diffraction intensity, and the abscissa represents diffraction angle 2θ (°). Figure 11 (D) shows the results of powder X-ray diffraction measurement of the crystals obtained in Example 2-8-14. The ordinate represents diffraction intensity, and the abscissa represents diffraction angle 2θ (°). Figure 11 (E) shows the results of powder X-ray diffraction measurement of the crystals obtained in Example 2-8-15. The ordinate represents diffraction intensity, and the abscissa represents diffraction angle 2θ (°).

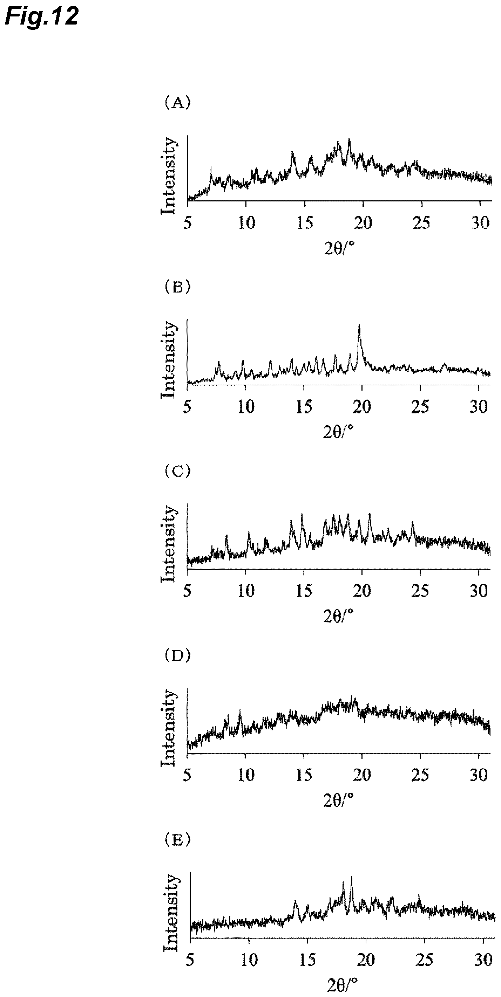

[Figure 12] Figure 12 (A) shows the results of powder X-ray diffraction measurement of the crystals obtained in Example 2-8-16. The ordinate represents diffraction intensity, and the abscissa represents diffraction angle 2θ (°). Figure 12 (B) shows the results of powder X-ray diffraction measurement of the crystals obtained in Example 2-8-17. The ordinate represents diffraction intensity, and the abscissa represents diffraction angle 2θ (°). Figure 12 (C) shows the results of powder X-ray diffraction measurement of the crystals obtained in Example 2-8-19. The ordinate represents diffraction intensity, and the abscissa represents diffraction angle 2θ (°). Figure 12 (D) shows the results of powder X-ray diffraction measurement of the crystals obtained in Example 2-8-20. The ordinate represents diffraction intensity, and the abscissa represents diffraction angle 2θ (°). Figure 12 (E) shows the results of powder X-ray diffraction measurement of the crystals obtained in Example 2-8-21. The ordinate represents diffraction intensity, and the abscissa represents diffraction angle 2θ (°).

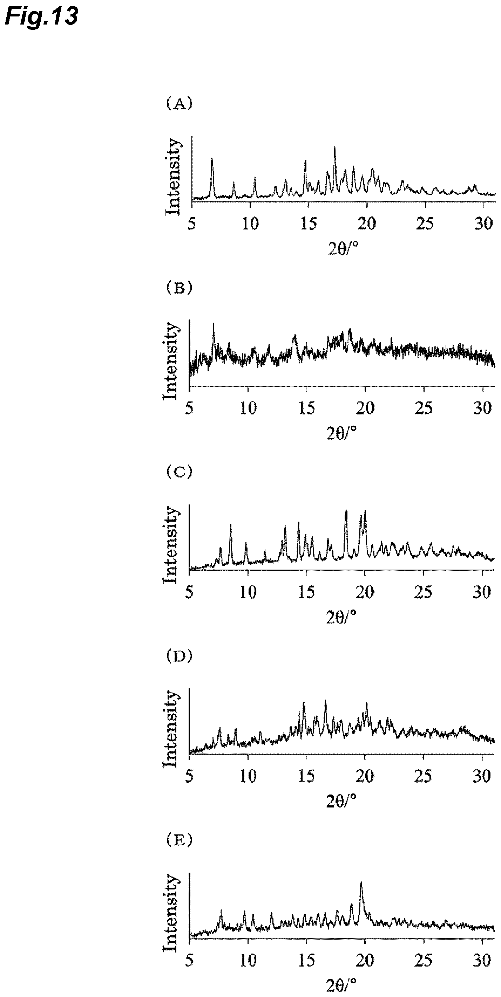

[Figure 13] Figure 13 (A) shows the results of powder X-ray diffraction measurement of the crystals obtained in Example 2-8-22. The ordinate represents diffraction intensity, and the abscissa represents diffraction angle 2θ (°). Figure 13 (B) shows the results of powder X-ray diffraction measurement of the crystals obtained in Example 2-8-23. The ordinate represents diffraction intensity, and the abscissa represents diffraction angle 2θ (°). Figure 13 (C) shows the results of powder X-ray diffraction measurement of the crystals obtained in Example 2-8-24. The ordinate represents diffraction intensity, and the abscissa represents diffraction angle 2θ (°). Figure 13 (D) shows the results of powder X-ray diffraction measurement of the crystals obtained in Example 2-8-25. The ordinate represents diffraction intensity, and the abscissa represents diffraction angle 2θ (°). Figure 13 (E) shows the results of powder X-ray diffraction measurement of the crystals obtained in Example 2-8-26. The ordinate represents diffraction intensity, and the abscissa represents diffraction angle 2θ (°).

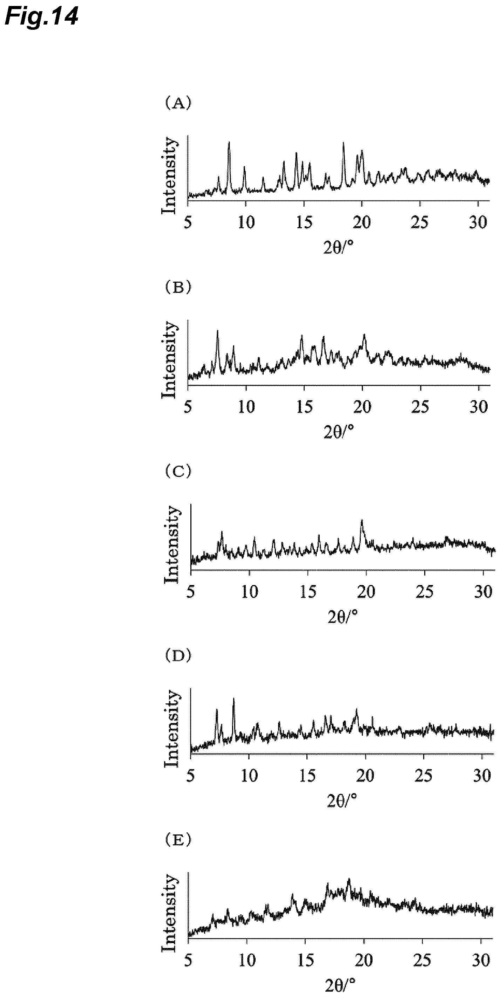

[Figure 14] Figure 14 (A) shows the results of powder X-ray diffraction measurement of the crystals obtained in Example 2-8-28. The ordinate represents diffraction intensity, and the abscissa represents diffraction angle 2θ (°). Figure 14 (B) shows the results of powder X-ray diffraction measurement of the crystals obtained in Example 2-8-30. The ordinate represents diffraction intensity, and the abscissa represents diffraction angle 2θ (°). Figure 14 (C) shows the results of powder X-ray diffraction measurement of the crystals obtained in Example 2-8-31. The ordinate represents diffraction intensity, and the abscissa represents diffraction angle 2θ (°). Figure 14 (D) shows the results of powder X-ray diffraction measurement of the crystals obtained in Example 2-8-32. The ordinate represents diffraction intensity, and the abscissa represents diffraction angle 2θ (°). Figure 14 (E) shows the results of powder X-ray diffraction measurement of the crystals obtained in Example 2-8-33. The ordinate represents diffraction intensity, and the abscissa represents diffraction angle 2θ (°).

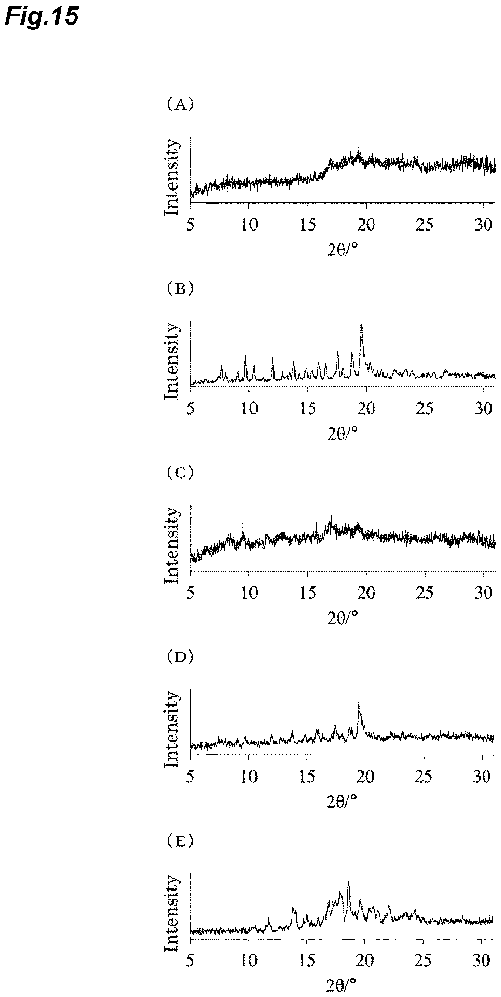

[Figure 15] Figure 15 (A) shows the results of powder X-ray diffraction measurement of the crystals obtained in Example 2-8-34. The ordinate represents diffraction intensity, and the abscissa represents diffraction angle 2θ (°). Figure 15 (B) shows the results of powder X-ray diffraction measurement of the crystals obtained in Example 2-8-35. The ordinate represents diffraction intensity, and the abscissa represents diffraction angle 2θ (°). Figure 15 (C) shows the results of powder X-ray diffraction measurement of the crystals obtained in Example 2-8-36. The ordinate represents diffraction intensity, and the abscissa represents diffraction angle 2θ (°). Figure 15 (D) shows the results of powder X-ray diffraction measurement of the crystals obtained in Example 2-8-37. The ordinate represents diffraction intensity, and the abscissa represents diffraction angle 2θ (°). Figure 15 (E) shows the results of powder X-ray diffraction measurement of the crystals obtained in Example 2-8-38. The ordinate represents diffraction intensity, and the abscissa represents diffraction angle 2θ (°).

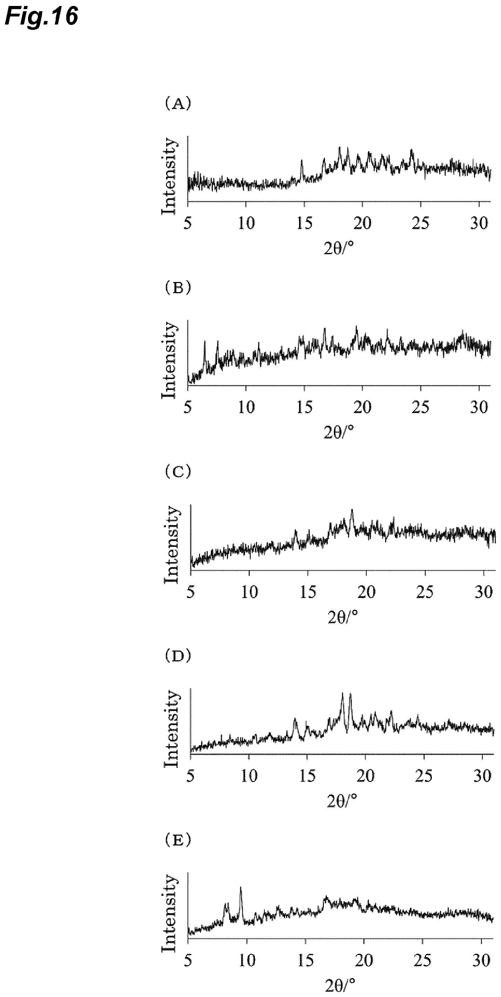

[Figure 16] Figure 16 (A) shows the results of powder X-ray diffraction measurement of the crystals obtained in Example 2-8-39. The ordinate represents diffraction intensity, and the abscissa represents diffraction angle 2θ (°). Figure 16 (B) shows the results of powder X-ray diffraction measurement of the crystals obtained in Example 2-8-40. The ordinate represents diffraction intensity, and the abscissa represents diffraction angle 2θ (°). Figure 16 (C) shows the results of powder X-ray diffraction measurement of the crystals obtained in Example 2-8-41. The ordinate represents diffraction intensity, and the abscissa represents diffraction angle 2θ (°). Figure 16 (D) shows the results of powder X-ray diffraction measurement of the crystals obtained in Example 2-8-42. The ordinate represents diffraction intensity, and the abscissa represents diffraction angle 2θ (°). Figure 16 (E) shows the results of powder X-ray diffraction measurement of the crystals obtained in Example 2-8-43. The ordinate represents diffraction intensity, and the abscissa represents diffraction angle 2θ (°).

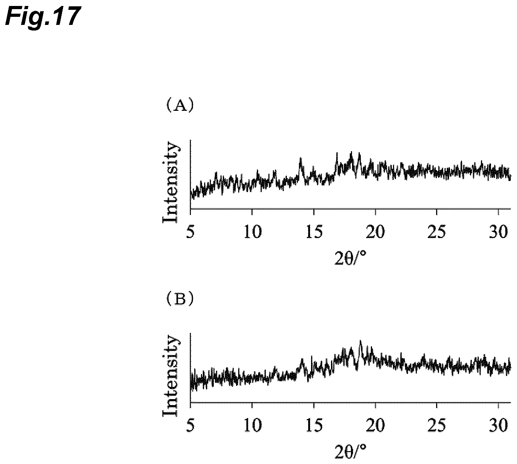

[Figure 17] Figure 17 (A) shows the results of powder X-ray diffraction measurement of the crystals obtained in Example 2-8-44. The ordinate represents diffraction intensity, and the abscissa represents diffraction angle 2θ (°). Figure 17 (B) shows the results of powder X-ray diffraction measurement of the crystals obtained in Example 2-8-45. The ordinate represents diffraction intensity, and the abscissa represents diffraction angle 2θ (°).

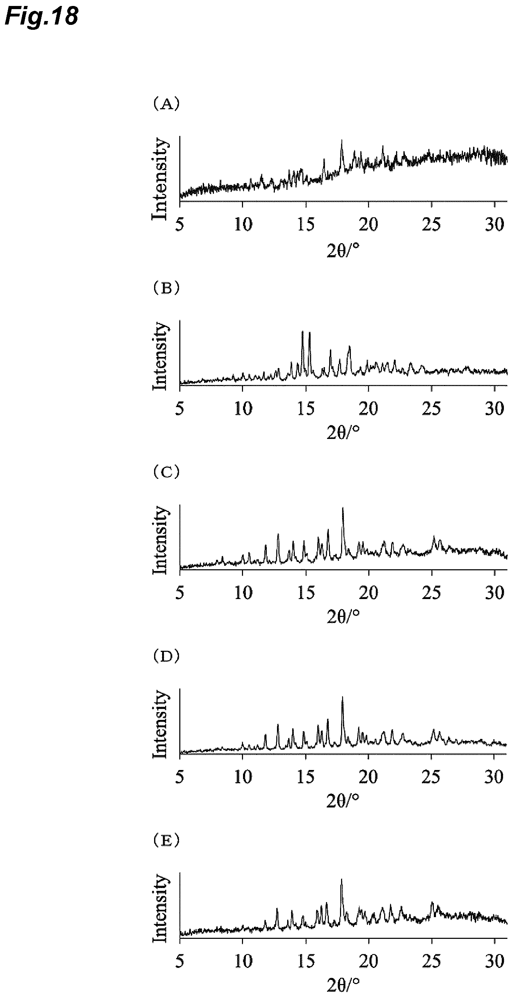

[Figure 18] Figure 18 (A) shows the results of powder X-ray diffraction measurement of the crystals obtained in Example 3-1-1. The ordinate represents diffraction intensity, and the abscissa represents diffraction angle 2θ (°). Figure 18 (B) shows the results of powder X-ray diffraction measurement of the crystals obtained in Example 3-4-1. The ordinate represents diffraction intensity, and the abscissa represents diffraction angle 2θ (°). Figure 18 (C) shows the results of powder X-ray diffraction measurement of the crystals obtained in Example 3-5-1. The ordinate represents diffraction intensity, and the abscissa represents diffraction angle 2θ (°). Figure 18 (D) shows the results of powder X-ray diffraction measurement of the crystals obtained in Example 3-5-2. The ordinate represents diffraction intensity, and the abscissa represents diffraction angle 2θ (°). Figure 18 (E) shows the results of powder X-ray diffraction measurement of the crystals obtained in Example 3-5-3. The ordinate represents diffraction intensity, and the abscissa represents diffraction angle 2θ (°).

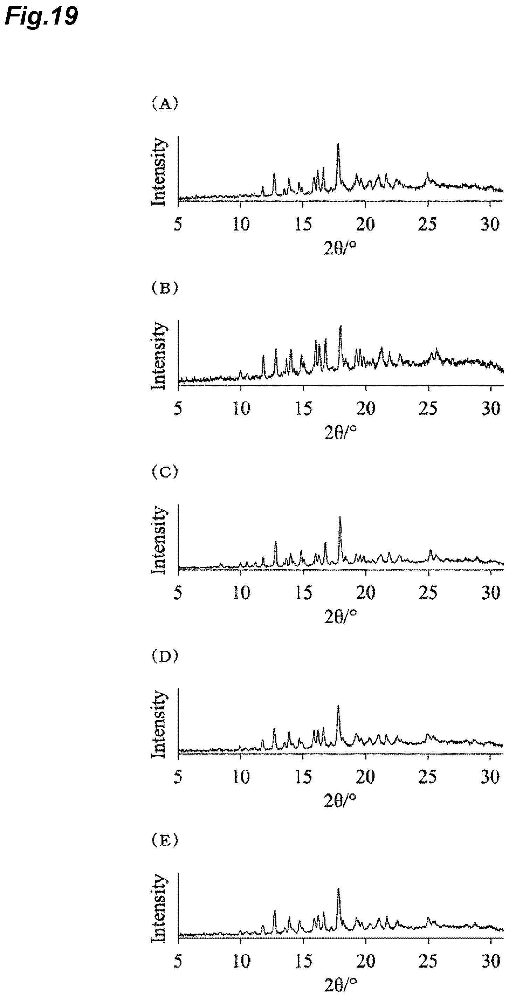

[Figure 19] Figure 19 (A) shows the results of powder X-ray diffraction measurement of the crystals obtained in Example 3-5-4. The ordinate represents diffraction intensity, and the abscissa represents diffraction angle 2θ (°). Figure 19 (B) shows the results of powder X-ray diffraction measurement of the crystals obtained in Example 3-5-5. The ordinate represents diffraction intensity, and the abscissa represents diffraction angle 2θ (°). Figure 19 (C) shows the results of powder X-ray diffraction measurement of the crystals obtained in Example 3-5-6. The ordinate represents diffraction intensity, and the abscissa represents diffraction angle 2θ (°). Figure 19 (D) shows the results of powder X-ray diffraction measurement of the crystals obtained in Example 3-5-7. The ordinate represents diffraction intensity, and the abscissa represents diffraction angle 2θ (°). Figure 19 (E) shows the results of powder X-ray diffraction measurement of the crystals obtained in Example 3-5-8. The ordinate represents diffraction intensity, and the abscissa represents diffraction angle 2θ (°).

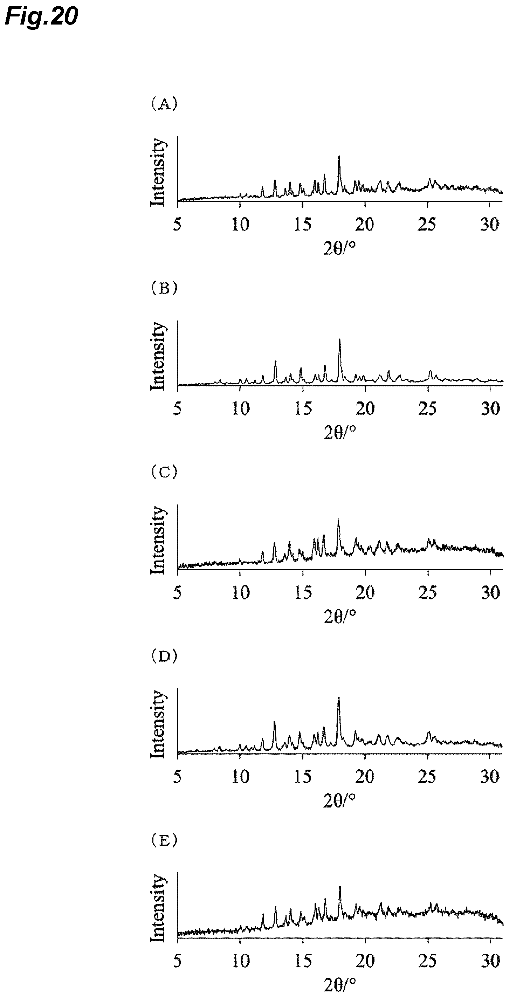

[Figure 20] Figure 20 (A) shows the results of powder X-ray diffraction measurement of the crystals obtained in Example 3-5-9. The ordinate represents diffraction intensity, and the abscissa represents diffraction angle 2θ (°). Figure 20 (B) shows the results of powder X-ray diffraction measurement of the crystals obtained in Example 3-5-10. The ordinate represents diffraction intensity, and the abscissa represents diffraction angle 2θ (°). Figure 20 (C) shows the results of powder X-ray diffraction measurement of the crystals obtained in Example 3-5-11. The ordinate represents diffraction intensity, and the abscissa represents diffraction angle 2θ (°). Figure 20 (D) shows the results of powder X-ray diffraction measurement of the crystals obtained in Example 3-5-12. The ordinate represents diffraction intensity, and the abscissa represents diffraction angle 2θ (°). Figure 20 (E) shows the results of powder X-ray diffraction measurement of the crystals obtained in Example 3-5-13. The ordinate represents diffraction intensity, and the abscissa represents diffraction angle 2θ (°).

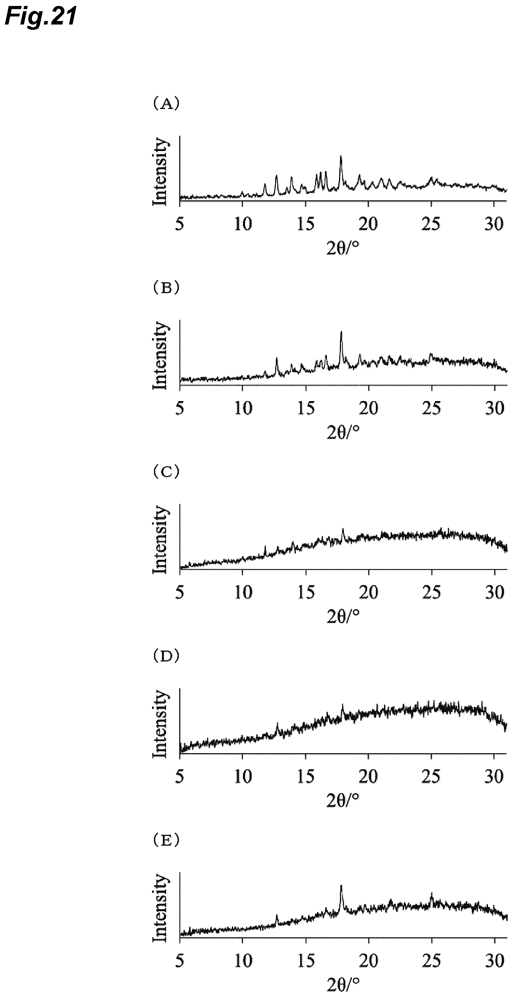

[Figure 21] Figure 21 (A) shows the results of powder X-ray diffraction measurement of the crystals obtained in Example 3-5-14. The ordinate represents diffraction intensity, and the abscissa represents diffraction angle 2θ (°). Figure 21 (B) shows the results of powder X-ray diffraction measurement of the crystals obtained in Example 3-5-15. The ordinate represents diffraction intensity, and the abscissa represents diffraction angle 2θ (°). Figure 21 (C) shows the results of powder X-ray diffraction measurement of the crystals obtained in Example 3-5-16. The ordinate represents diffraction intensity, and the abscissa represents diffraction angle 2θ (°). Figure 21 (D) shows the results of powder X-ray diffraction measurement of the crystals obtained in Example 3-5-17. The ordinate represents diffraction intensity, and the abscissa represents diffraction angle 2θ (°). Figure 21 (E) shows the results of powder X-ray diffraction measurement of the crystals obtained in Example 3-5-18. The ordinate represents diffraction intensity, and the abscissa represents diffraction angle 2θ (°).

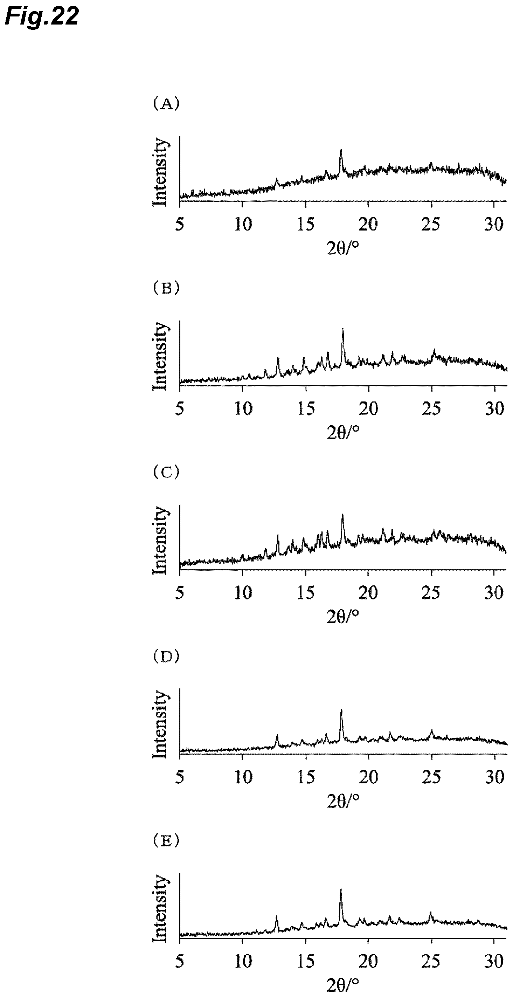

[Figure 22] Figure 22 (A) shows the results of powder X-ray diffraction measurement of the crystals obtained in Example 3-5-19. The ordinate represents diffraction intensity, and the abscissa represents diffraction angle 2θ (°). Figure 22 (B) shows the results of powder X-ray diffraction measurement of the crystals obtained in Example 3-5-20. The ordinate represents diffraction intensity, and the abscissa represents diffraction angle 2θ (°). Figure 22 (C) shows the results of powder X-ray diffraction measurement of the crystals obtained in Example 3-5-21. The ordinate represents diffraction intensity, and the abscissa represents diffraction angle 2θ (°). Figure 22 (D) shows the results of powder X-ray diffraction measurement of the crystals obtained in Example 3-5-22. The ordinate represents diffraction intensity, and the abscissa represents diffraction angle 2θ (°). Figure 22 (E) shows the results of powder X-ray diffraction measurement of the crystals obtained in Example 3-5-23. The ordinate represents diffraction intensity, and the abscissa represents diffraction angle 2θ (°).

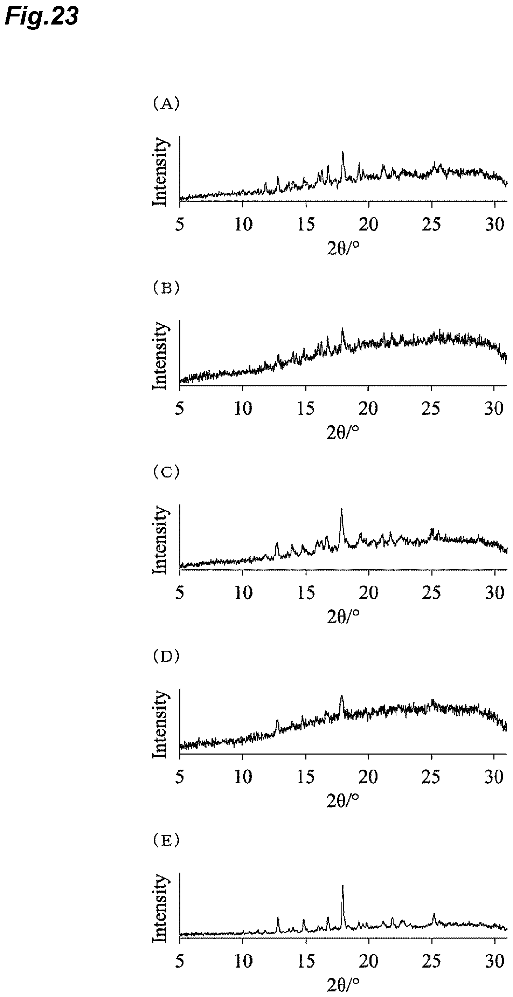

[Figure 23] Figure 23 (A) shows the results of powder X-ray diffraction measurement of the crystals obtained in Example 3-5-24. The ordinate represents diffraction intensity, and the abscissa represents diffraction angle 2θ (°). Figure 23 (B) shows the results of powder X-ray diffraction measurement of the crystals obtained in Example 3-5-25. The ordinate represents diffraction intensity, and the abscissa represents diffraction angle 2θ (°). Figure 23 (C) shows the results of powder X-ray diffraction measurement of the crystals obtained in Example 3-5-26. The ordinate represents diffraction intensity, and the abscissa represents diffraction angle 2θ (°). Figure 23 (D) shows the results of powder X-ray diffraction measurement of the crystals obtained in Example 3-5-27. The ordinate represents diffraction intensity, and the abscissa represents diffraction angle 2θ (°). Figure 23 (E) shows the results of powder X-ray diffraction measurement of the crystals obtained in Example 3-5-28. The ordinate represents diffraction intensity, and the abscissa represents diffraction angle 2θ (°).

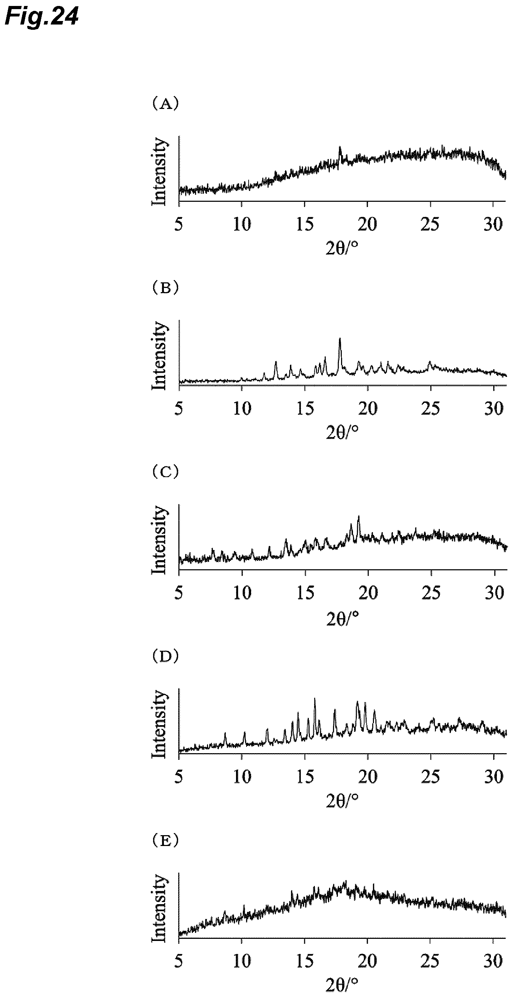

[Figure 24] Figure 24 (A) shows the results of powder X-ray diffraction measurement of the crystals obtained in Example 3-5-29. The ordinate represents diffraction intensity, and the abscissa represents diffraction angle 2θ (°). Figure 24 (B) shows the results of powder X-ray diffraction measurement of the crystals obtained in Example 3-5-30. The ordinate represents diffraction intensity, and the abscissa represents diffraction angle 2θ (°). Figure 24 (C) shows the results of powder X-ray diffraction measurement of the crystals obtained in Example 3-7-1. The ordinate represents diffraction intensity, and the abscissa represents diffraction angle 2θ (°). Figure 24 (D) shows the results of powder X-ray diffraction measurement of the crystals obtained in Example 3-8-1. The ordinate represents diffraction intensity, and the abscissa represents diffraction angle 2θ (°). Figure 24 (E) shows the results of powder X-ray diffraction measurement of the crystals obtained in Example 3-8-2. The ordinate represents diffraction intensity, and the abscissa represents diffraction angle 2θ (°).

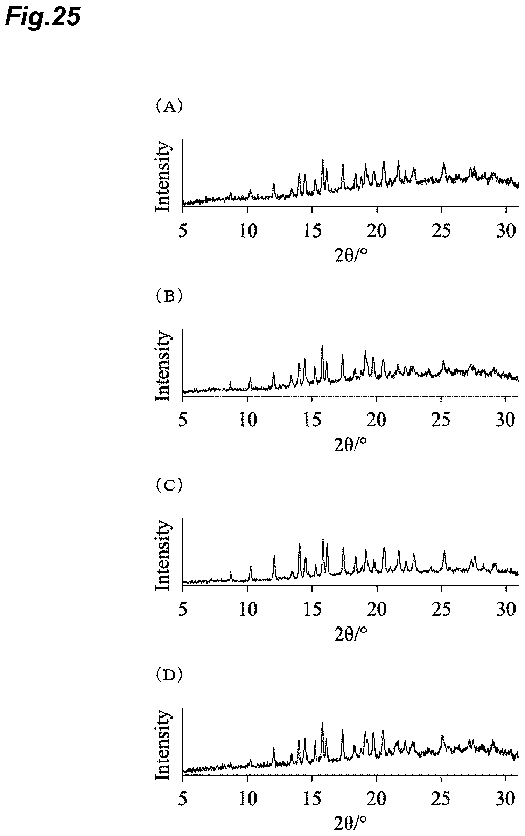

[Figure 25] Figure 25 (A) shows the results of powder X-ray diffraction measurement of the crystals obtained in Example 3-8-3. The ordinate represents diffraction intensity, and the abscissa represents diffraction angle 2θ (°). Figure 25 (B) shows the results of powder X-ray diffraction measurement of the crystals obtained in Example 3-8-4. The ordinate represents diffraction intensity, and the abscissa represents diffraction angle 2θ (°). Figure 25 (C) shows the results of powder X-ray diffraction measurement of the crystals obtained in Example 3-8-5. The ordinate represents diffraction intensity, and the abscissa represents diffraction angle 2θ (°). Figure 25 (D) shows the results of powder X-ray diffraction measurement of the crystals obtained in Example 3-8-6. The ordinate represents diffraction intensity, and the abscissa represents diffraction angle 2θ (°).

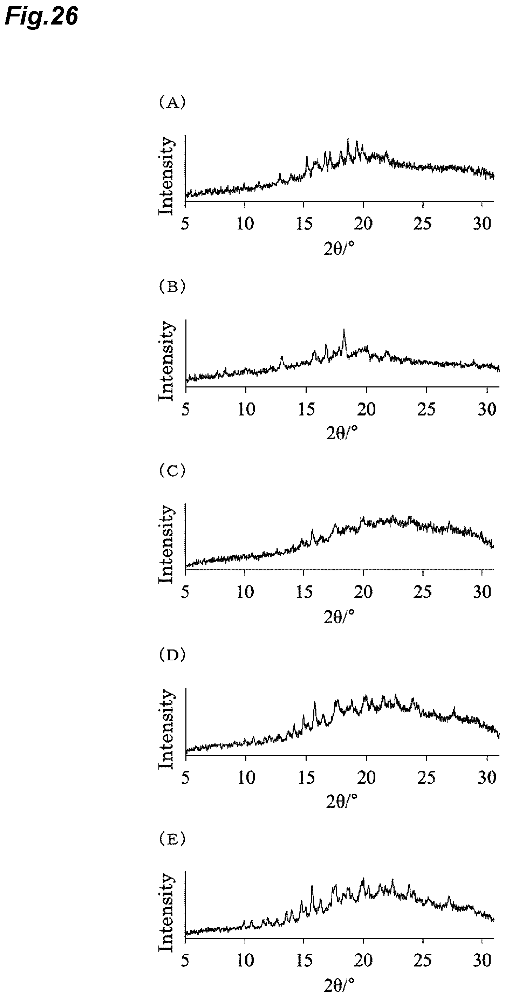

[Figure 26] Figure 26 (A) shows the results of powder X-ray diffraction measurement of the crystals obtained in Example 4-1-1. The ordinate represents diffraction intensity, and the abscissa represents diffraction angle 2θ (°). Figure 26 (B) shows the results of powder X-ray diffraction measurement of the crystals obtained in Example 4-3-1. The ordinate represents diffraction intensity, and the abscissa represents diffraction angle 2θ (°). Figure 26 (C) shows the results of powder X-ray diffraction measurement of the crystals obtained in Example 4-4-1. The ordinate represents diffraction intensity, and the abscissa represents diffraction angle 2θ (°). Figure 26 (D) shows the results of powder X-ray diffraction measurement of the crystals obtained in Example 4-4-2. The ordinate represents diffraction intensity, and the abscissa represents diffraction angle 2θ (°). Figure 26 (E) shows the results of powder X-ray diffraction measurement of the crystals obtained in Example 4-4-3. The ordinate represents diffraction intensity, and the abscissa represents diffraction angle 2θ (°).

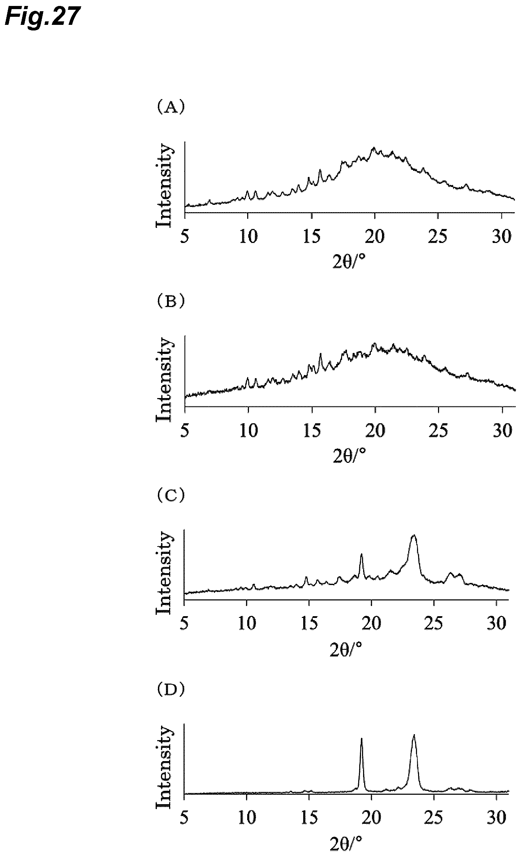

[Figure 27] Figure 27 (A) shows the results of powder X-ray diffraction measurement of the crystals obtained in Example 4-4-4. The ordinate represents diffraction intensity, and the abscissa represents diffraction angle 2θ (°). Figure 27 (B) shows the results of powder X-ray diffraction measurement of the crystals obtained in Example 4-4-5. The ordinate represents diffraction intensity, and the abscissa represents diffraction angle 2θ (°). Figure 27 (C) shows the results of powder X-ray diffraction measurement of the crystals obtained in Example 4-4-6. The ordinate represents diffraction intensity, and the abscissa represents diffraction angle 2θ (°). In addition to 19.2° and 24.4° derived from PEG2000, multiple diffraction peaks are also observed. These are the diffraction peaks of CP04 crystals. Figure 27 (D) shows the results of powder X-ray diffraction measurement of PEG2000. The ordinate represents diffraction intensity, and the abscissa represents diffraction angle 2θ (°). It has characteristic peaks at 19.2° and 24.4°.

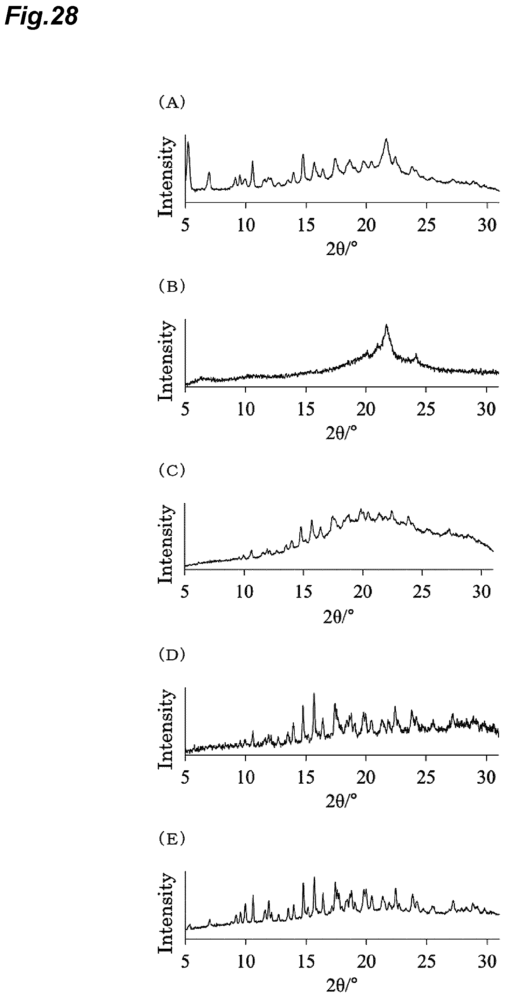

[Figure 28] Figure 28 (A) shows the results of powder X-ray diffraction measurement of the crystals obtained in Example 4-4-7. The ordinate represents diffraction intensity, and the abscissa represents diffraction angle 2θ (°). In addition to 21.7° and 24.1° derived from polyethylene glycol monostearate (n = about 4) (palmitate, stearate mixture), multiple diffraction peaks are also observed. These are the diffraction peaks of CP04 crystals. Figure 28 (B) shows the results of powder X-ray diffraction measurement of polyethylene glycol monostearate (n = about 4) (palmitate, stearate mixture). The ordinate represents diffraction intensity, and the abscissa represents diffraction angle 2θ (°). It has characteristic peaks at 21.7° and 24.1°. Figure 28 (C) shows the results of powder X-ray diffraction measurement of the crystals obtained in Example 4-4-8. The ordinate represents diffraction intensity, and the abscissa represents diffraction angle 2θ (°). Figure 28 (D) shows the results of powder X-ray diffraction measurement of the crystals obtained in Example 4-4-9. The ordinate represents diffraction intensity, and the abscissa represents diffraction angle 2θ (°). Figure 28 (E) shows the results of powder X-ray diffraction measurement of the crystals obtained in Example 4-4-10. The ordinate represents diffraction intensity, and the abscissa represents diffraction angle 2θ (°).

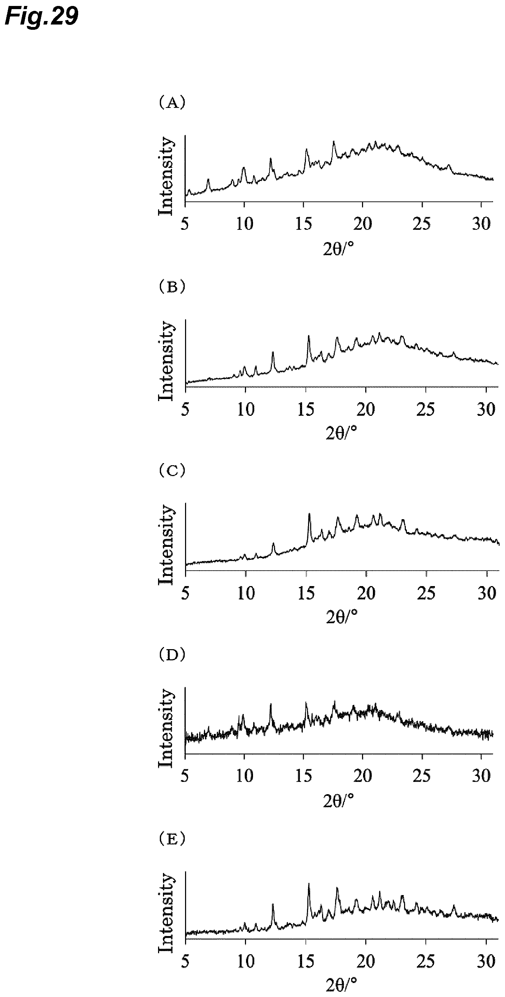

[Figure 29] Figure 29 (A) shows the results of powder X-ray diffraction measurement of the crystals obtained in Example 4-5-1. The ordinate represents diffraction intensity, and the abscissa represents diffraction angle 2θ (°). Figure 29 (B) shows the results of powder X-ray diffraction measurement of the crystals obtained in Example 4-5-2. The ordinate represents diffraction intensity, and the abscissa represents diffraction angle 2θ (°). Figure 29 (C) shows the results of powder X-ray diffraction measurement of the crystals obtained in Example 4-5-3. The ordinate represents diffraction intensity, and the abscissa represents diffraction angle 2θ (°). Figure 29 (D) shows the results of powder X-ray diffraction measurement of the crystals obtained in Example 4-5-4. The ordinate represents diffraction intensity, and the abscissa represents diffraction angle 2θ (°). Figure 29 (E) shows the results of powder X-ray diffraction measurement of the crystals obtained in Example 4-5-5. The ordinate represents diffraction intensity, and the abscissa represents diffraction angle 2θ (°).

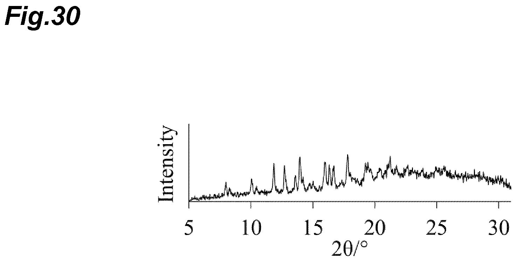

[Figure 30] Figure 30 shows the results of powder X-ray diffraction measurement of the crystals obtained in Example 4-5-6. The ordinate represents diffraction intensity, and the abscissa represents diffraction angle 2θ (°).

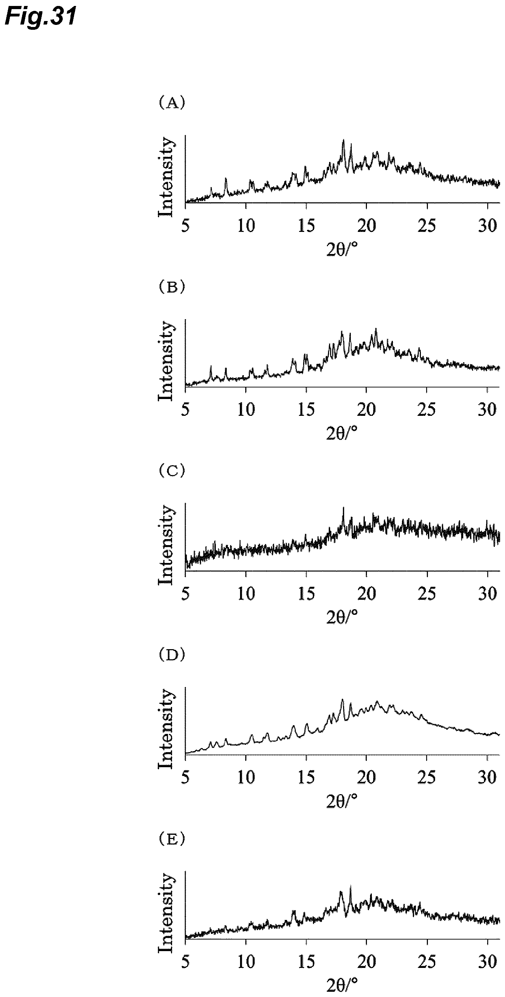

[Figure 31] Figure 31 (A) shows the results of powder X-ray diffraction measurement of the crystals obtained in Example 4-7-1. The ordinate represents diffraction intensity, and the abscissa represents diffraction angle 2θ (°). Figure 31 (B) shows the results of powder X-ray diffraction measurement of the crystals obtained in Example 4-7-2. The ordinate represents diffraction intensity, and the abscissa represents diffraction angle 2θ (°). Figure 31 (C) shows the results of powder X-ray diffraction measurement of the crystals obtained in Example 4-7-3. The ordinate represents diffraction intensity, and the abscissa represents diffraction angle 2θ (°). Figure 31 (D) shows the results of powder X-ray diffraction measurement of the crystals obtained in Example 4-7-4. The ordinate represents diffraction intensity, and the abscissa represents diffraction angle 2θ (°). Figure 31 (E) shows the results of powder X-ray diffraction measurement of the crystals obtained in Example 4-8-1. The ordinate represents diffraction intensity, and the abscissa represents diffraction angle 2θ (°).

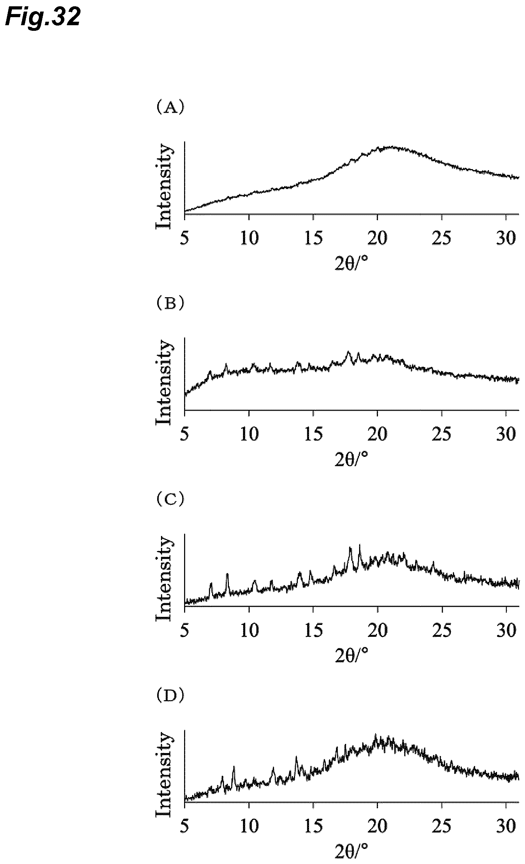

[Figure 32] Figure 32 (A) shows the results of powder X-ray diffraction measurement of the crystals obtained in Example 4-8-2. The ordinate represents diffraction intensity, and the abscissa represents diffraction angle 2θ (°). Figure 32 (B) shows the results of powder X-ray diffraction measurement of the crystals obtained in Example 4-8-3. The ordinate represents diffraction intensity, and the abscissa represents diffraction angle 2θ (°). Figure 32 (C) shows the results of powder X-ray diffraction measurement of the crystals obtained in Example 4-8-4. The ordinate represents diffraction intensity, and the abscissa represents diffraction angle 2θ (°). Figure 32 (D) shows the results of powder X-ray diffraction measurement of the crystals obtained in Example 4-8-5. The ordinate represents diffraction intensity, and the abscissa represents diffraction angle 2θ (°).

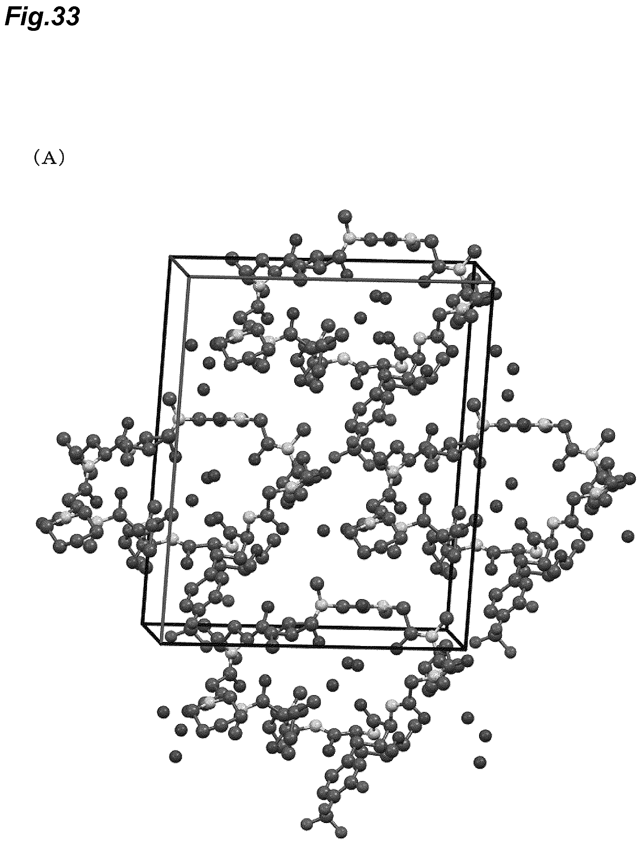

[Figure 33] Figure 33 (A) shows the crystal structure by single crystal X-ray structural analysis on crystals of compound CP01 obtained in Example 2-1-1.

### [Description of Embodiments]

The term "room temperature" as used herein means a temperature of about 20°C to about 25°C.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, the term "to" that indicates a range includes values of both ends thereof. For example, "A to B" means the range of A or more and B or less.

The term "about" as used herein means the value range of + 10% and - 10% of the numeric value, when used in combination with a numeric value.

In the present invention, the term "and/or" is meant to include every combination of the terms "and" and "or" appropriately combined. Specifically, for example, the term "A, B, and/or C" includes the following seven variations; (i) A, (ii) B, (iii) C, (iv) A and B, (v) A and C, (vi) B and C, and (vii) A, B, and C.

As used herein, the term "v/v%" represents % by volume (volume%) and the term "wt/v%" represents % by weight/volume (weight/volume%).

The term "peptide" as used herein means one in which two or more amino acids are linked by an amide bond. Peptides having an ester bond in part of the main chain, such as depsipeptide, are also included in the term "peptide" herein.

The term "cyclic peptide" as used herein is a peptide having a cyclic structure composed of 4 or more amino acid residues. As an aspect of cyclization of the cyclic peptide, it may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization via a covalent bond such as an amide bond, a carbon-sulfur bond, or a carbon-carbon bond is preferred. Cyclization with an amide bond is more preferred, and the position of the carboxyl group or amino group used for cyclization may be on the main chain or on a side chain. Most preferred is cyclization via an amide bond between a carboxyl group on a side chain and the amino group of the main chain at the N-terminus.

The term "heterocyclic ring" as used herein means a nonaromatic heterocyclic ring containing preferably 1 to 5, more preferably 1 to 3, heteroatoms among the atoms constituting the ring. The heterocyclic ring may have a double and/or triple bond in the ring. A carbon atom in the ring may form carbonyl through oxidation, and the ring may be a monocyclic ring, a condensed ring, or a spiro ring. The number of atoms constituting the ring of the heterocyclic ring is preferably 3 to 12 (3- to 12-membered heterocyclic ring), and more preferably 4 to 10 (4-to 10-membered heterocyclic ring). Specific examples of the heterocyclic ring include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, a 6,7-dihydro-pyrrolo[1,2-a]imidazole ring, an azocane ring, a 4,5,6,7-tetrahydropyrazolo[1,5-a]pyrazine ring, an azepane ring, a dioxepane ring, a 5,9-dioxaspiro[3.5]nonane ring, or a ring in which one or more single bonds in any of these saturated heterocyclic rings are replaced with double bonds or triple bonds.

The term "cyclization" of a peptide means formation of a cyclic moiety containing 4 or more amino acid residues. The number of amino acids contained in the cyclic moiety of the cyclic peptide herein is not particularly limited as long as it is 4 or more, and examples thereof include 4 to 20 residues, 5 to 15 residues, 6 to 13 residues, 9 to 13 residues, and 11 residues. It is preferably 5 to 15 residues, more preferably 9 to 13 residues, and most preferably 11 residues. The method for converting a chain peptide to a cyclic peptide can be performed by carrying out an intramolecular bond formation reaction by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March), or the like. It is also possible to further carry out a functional group conversion reaction after the bond formation reaction. Examples of the bond at the cyclization site of the cyclic peptide include a C(O)-N bond formed from a carboxylic acid and an amine, a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O₂)-C bond via a sulfur atom, and a C-N-C bond, a C=N-C bond, a N-C(O)-N bond, a N-C(S)N bond, and a C(S)-N bond via a nitrogen atom. Further examples thereof include a C-C bond formed by a coupling reaction catalyzed by a transition metal, such as the Suzuki reaction, the Heck reaction, and the Sonogashira reaction. Examples of the functional group conversion reaction that is further carried out after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which, among carbon-carbon bonds, a triple bond or a double bond is reduced and converted to a double bond or a single bond. Two amino acids may be bonded at the main chains of the amino acids to form a closed ring structure by a peptide bond, or a covalent bond may be formed between two amino acids via, for example, a bond between side chains or between a side chain and the main chain of the two amino acids.

As used herein, the term "cyclic moiety" of the cyclic peptide means a ring-like portion formed by linkage of 4 or more amino acid residues.

The term "cyclic peptide crystals" as used herein is understood to have the meaning including (i) crystals of a cyclic peptide free form, (ii) crystals of a solvate of a cyclic peptide, and (iii) a mixture thereof, and may be any of them.

The solvate as used herein is one in which a compound and a solvent together form a molecular aggregate, and is not particularly limited as long as it is a solvate formed by a solvent acceptable for ingestion along with administration of a medicament. Examples of the solvate include a hydrate, an alcohol solvate (such as ethanol solvate, methanol solvate, 1-propanol solvate, or 2-propanol solvate), and not only solvates formed with a single solvent such as formamide or dimethyl sulfoxide, but also solvates formed with a plurality of solvents per one molecule of the compound, or solvates formed with a plurality of types of solvents per one molecule of the compound. Note that, as described in Crystal Growth & Design 2012, 12, 2147-2152, solvates are distinguished from cocrystals. As used herein, cocrystals are defined as crystals composed of a compound and a component that is solid at 25°C. While the term "cyclic peptide crystals" as used herein includes (i) crystals of a cyclic peptide free form, (ii) crystals of a solvate of a cyclic peptide, and (iii) a mixture thereof, it neither includes cocrystals containing a cyclic peptide, nor a mixture of cocrystals containing a cyclic peptide and any crystals of the above (i) to (iii).

The term "amino acid" as used herein includes a natural amino acid and a non-natural amino acid. The term "amino acid" as used herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, and Pro. Examples of the non-natural amino acid include, but are not particularly limited to, a β-amino acid, a D-type amino acid, a N-substituted amino acid, an α,α-disubstituted amino acid, an amino acid having a side chain different from that of natural amino acids, and a hydroxycarboxylic acid. As used herein, the amino acid accepts an arbitrary conformation. The selection of a side chain of the amino acid is not particularly limited, and the side chain is freely selected from, in addition to a hydrogen atom, for example, an alkyl group, an alkenyl group, an alkynyl group, an aryl group, a heteroaryl group, an aralkyl group, a heteroarylalkyl group, a cycloalkyl group, a spiro-bonded cycloalkyl group. A substituent may be added to each of the side chains. Such a substituent is not limited either, and can be one or two or more substituents each independently freely selected from any substituents including, for example, a halogen atom, an O atom, a S atom, a N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group that is optionally substituted, or oxo, aminocarbonyl, and a halogen atom. In one non-limiting aspect, the amino acid as used herein may be a compound having a carboxyl group and an amino group in the same molecule. Even in this case, a compound in which the nitrogen atom of an amino group and an arbitrary atom of a side chain together form a ring (such as proline, hydroxyproline, and azetidine-2-carboxylic acid) is also included in the amino acid as used herein.

As used herein, the term "optionally substituted" means that a group is optionally substituted by an arbitrary substituent. Furthermore, a substituent may be added to each of the substituents. Such a substituent is not limited either, and can be one or two or more substituents each independently freely selected from any substituents including, for example, a halogen atom, an oxygen atom, a sulfur atom, a nitrogen atom, a boron atom, a silicon atom, or a phosphorus atom. Examples of the substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The term "halogen" as used herein refers to, for example, F, Cl, Br, or I.

The "alkyl" as used herein is a monovalent group derived from an aliphatic hydrocarbon by removing any one hydrogen atom, and has a subset of hydrocarbyl or hydrocarbon group structures that do not contain a heteroatom (which refers to an atom other than carbon and hydrogen atoms) or an unsaturated carbon-carbon bond, and contain hydrogen and carbon atoms in the backbone. The alkyl includes not only a linear form but also a branched form. Preferred examples of the alkyl include alkyl having 1 to 20 carbon atoms (C₁-C₂₀; hereinafter, "Cₚ-C_{q}" means that the number of carbon atoms is p to q), preferably C₁-C₁₀ alkyl, and more preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

As used herein, the term "alkynyl" is a monovalent group having at least one triple bond (two adjacent SP carbon atoms). The alkynyl includes not only a linear form but a branched form. Preferred examples of the alkynyl include C₂-C₁₀ alkynyl, and more preferably C₂-C₆ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

As used herein, the term "alkenyl" is a monovalent group having at least one double bond (two adjacent SP2 carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. Preferred examples of the alkenyl include C₂-C₁₀ alkenyl, and more preferred examples thereof include C₂-C₆ alkenyl. Specific examples thereof include vinyl, allyl, 1-propenyl, 2-propenyl, 1-butenyl, 2-butenyl (including cis and trans), 3-butenyl, pentenyl, 3-methyl-2-butenyl, and hexenyl.

The term "aryl" as used herein means a monovalent aromatic hydrocarbon ring and an aromatic hydrocarbon ring group. Preferred examples of the aryl include C₆-C₁₀ aryl. Specific examples of the aryl include phenyl and naphthyl (for example, 1-naphthyl and 2-naphthyl).

The term "heteroaryl" as used herein means an aromatic cyclic monovalent group containing 1 to 5 heteroatoms in addition to a carbon atom, and an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, imidazopyridyl, pyrazolopyridyl, imidazopyridyl, triazolopyridyl, pyrrolopyrazinyl, and flopyridyl.

The term "aralkyl (arylalkyl)" as used herein means a group in which at least one hydrogen atom of the "alkyl" as defined above is replaced with the "aryl" as defined above. As the aralkyl, C₇-C₁₄ aralkyl is preferred, and C₇-C₁₀ aralkyl is more preferred. Specific examples of the aralkyl include benzyl, phenethyl, and 3-phenylpropyl.

The term "heteroarylalkyl" as used herein means a group in which at least one hydrogen atom of the "alkyl" as defined above is replaced with the "heteroaryl" as defined above. As the heteroarylalkyl, 5- to 10-membered heteroaryl-C₁-C₆ alkyl is preferred, and 5- to 10-membered heteroaryl-C₁-C₂ alkyl is more preferred. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

The term "cycloalkyl" as used herein means a saturated or partially saturated cyclic monovalent aliphatic hydrocarbon group and includes a monocyclic ring, a bicyclo ring, and a spiro ring. Preferred examples of the cycloalkyl include C₃-C₈ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "alkoxy" as used herein means an oxy group to which the "alkyl" as defined above is bonded. Preferred examples of the alkoxy include C₁-C₆ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkoxyalkyl" as used herein means a group in which one or more hydrogen of the "alkyl" as defined above are replaced with the "alkoxy" as defined above. As the alkoxyalkyl, C₁-C₆ alkoxy-C₁-C₆ alkyl is preferred, and C₁-C₆ alkoxy-C₁-C₂ alkyl is more preferred. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

As used herein, the term "amino" means -NH₂ in the narrow sense and means -NRR' in the broad sense. In this context, R and R' are each independently selected from hydrogen, alkyl, alkenyl, alkynyl, cycloalkyl, heterocyclyl, aryl, and heteroaryl, or R and R' form a ring together with the nitrogen atom bonded thereto. Preferred examples of the amino include -NH₂, mono-C₁-C₆ alkylamino, di-C₁-C₆ alkylamino, and 4- to 8-membered cyclic amino.

The term "monoalkylamino" as used herein means a group of the "amino" as defined above in which R is hydrogen and R' is the "alkyl" as defined above. Preferred examples of the monoalkylamino include mono-C1-C6 alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" as defined above in which R and R' are each independently the "alkyl" as defined above. Preferred examples of the dialkylamino include di-C₁-C₆ alkylamino. Specific examples of the dialkylamino include dimethylamino and diethylamino.

The term "alkylsulfonylamino" as used herein means a group in which sulfonyl is bonded to the "amino" as defined above. Preferred examples thereof include C₁-C₆ alkylsulfonyl-NH- and (C₁-C₆ alkylsulfonyl-)₂-N-. Specific examples of the aminoalkylsulfonyl include methylsulfonylamino, ethylsulfonylamino, bis(methylsulfonyl)amino, and bis(ethylsulfonyl)amino.

The term "aminocarbonyl" as used herein means a carbonyl group to which the "amino" as defined above is bonded. Preferred examples of the aminocarbonyl include -CONH₂, mono-C₁-C₆ alkylaminocarbonyl, di-C₁-C₆ alkylaminocarbonyl, and 4- to 8-membered cyclic aminocarbonyl. Specific examples of the aminocarbonyl include -CONH₂, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octan-8-ylcarbonyl.

As used herein, the "amino acid residue" constituting a peptide is sometimes referred to simply as an "amino acid".

The term "side chain of an amino acid" as used herein means, in the case of an α-amino acid, an atomic group bonded to carbon (α-carbon) to which an amino group and a carboxyl group are bonded, other than the amino group and the carboxyl group. For example, the methyl group of Ala is a side chain of the amino acid. In the case of a β-amino acid, an atomic group bonded to the α-carbon and/or the β-carbon, other than the amino group bonded to the β-carbon and the carboxyl group bonded to the α-carbon, can be a side chain of the amino acid. Also, in the case of a γ-amino acid, an atomic group bonded to the α-carbon, the β-carbon, and/or the γ-carbon, other than the amino group bonded to the γ-carbon and the carboxyl group bonded to the α-carbon, can be a side chain of the amino acid.

The term "main chain of an amino acid" as used herein means, in the case of an α-amino acid, a chain portion constituted by the amino group, the α-carbon, and the carboxyl group; in the case of a β-amino acid, a chain portion constituted by the amino group, the β-carbon, the α-carbon, and the carboxyl group; and in the case of a γ-amino acid, a chain portion constituted by the amino group, the γ-carbon, the β-carbon, the α-carbon, and the carboxyl group. Also, the term "α-amino acid skeleton" means a chain portion constituted by the amino group, the α-carbon, and the carboxyl group; the term "β-amino acid skeleton" means a chain portion constituted by the amino group, the β-carbon, the α-carbon, and the carboxyl group; and the term "γ-amino acid skeleton" means a chain portion constituted by the amino group, the γ-carbon, the β-carbon, the α-carbon, and the carboxyl group. As used herein, an amino acid having a "β-amino acid skeleton" as a whole or as a substructure may be referred to as an "amino acid having a β-amino acid skeleton". For example, aspartic acid has a chain portion constituted by the amino group, the β-carbon, the α-carbon, and the carboxyl group (β-amino acid skeleton), and thus falls under the category of the "amino acid having a β-amino acid skeleton".

The term "main chain of a peptide" as used herein means a structure in which multiple amino acids are linked by an amide bond. The terms "main chain of a cyclic peptide" and "main chain of a cyclic moiety" as used herein mean a structure contained in the cyclic moiety of a cyclic peptide in which multiple amino acids are linked by an amide bond. The "main chain of a peptide", the "main chain of a cyclic peptide", and the "main chain of a cyclic moiety" may in part contain another bond, such as an ester bond, in place of an amide bond. Also, the "main chain of a cyclic peptide" and the "main chain of a cyclic moiety" may include a bond exemplified herein as the bond at the cyclization site of a cyclic peptide, or a bond formed by the cyclization formation reaction of a peptide.

The term "N-substituted amino acid" as used herein means an amino acid in which the amino group contained in the amino acid is substituted, that is, represented by -NHR (R represents alkyl, alkenyl, alkynyl, aryl, heteroaryl, aralkyl, or cycloalkyl optionally having a substituent, and one or two non-adjacent methylene groups in any of these groups is optionally substituted by an oxygen atom, a carbonyl group (-CO-), or a sulfonyl group (-SO₂-); and a carbon chain bonded to the N atom and the carbon atom at position α may form a ring, as in proline.). The "N-substituted amino acid" as used herein may be a N-alkylamino acid, and preferred examples thereof include a N-C₁-C₆ alkylamino acid, more preferred examples thereof include a N-C₁-C₄ alkylamino acid, and most preferred examples thereof include a N-ethylamino acid or a N-methylamino acid although it is not limited to these. Preferred examples of the "N-substituted amino acid" as used herein include a N-substituted amino acid in which the amino group contained in the "main chain of an amino acid" is substituted.

The term "N-unsubstituted amino acid" as used herein means an amino acid in which an amino group contained in the amino acid is not substituted, that is, represented by -NH₂. Preferred examples of the "N-unsubstituted amino acid" as used herein include a N-unsubstituted amino acid in which the amino group contained in the "main chain of an amino acid" is not substituted.

In the case where an amino acid constituting a cyclic peptide as used herein is aspartic acid, or an amino acid in which one or more of hydrogen on the α-carbon and hydrogen on the β-carbon of aspartic acid are substituted, or an amino acid in which the amino group contained in its "main chain of the amino acid" is substituted, the carboxy group bonded to the α-carbon contained in the "main chain of the amino acid" may be contained in the "main chain of a cyclic peptide". On the other hand, a carboxy group bonded to the β-carbon contained in the "side chain of an amino acid" may be contained in the "main chain of a cyclic peptide", in which case the "β-amino acid skeleton" is contained in the "main chain of a cyclic peptide".

As used herein, the term "number of amino acids" or "number of amino acid residues" refers to the number of amino acid residues (amino acid units) constituting a peptide, and means the number of amino acid units generated upon cleavage of amide bonds, ester bonds, and bonds of cyclized portions that link the amino acids. For example, the number of amino acids or the number of amino acid residues of the cyclic peptide in which the cyclic moiety is composed of 10 amino acid residues and the linear portion is composed of 1 amino acid residue is 11.

The term "containing an amino acid in the cyclic moiety of a cyclic peptide" as used herein means that the main chain of the cyclic moiety of a cyclic peptide contains an amino acid as a substructure.

As used herein, the term "saturated heterocyclic ring" means a nonaromatic heterocyclic ring containing 1 to 5 heteroatoms in addition to a carbon atom, without containing a double bond and/or a triple bond in the ring. The saturated heterocyclic ring may be a monocyclic ring or may form a condensed ring with another ring, for example, an aromatic ring such as a benzene ring. Preferred examples of the saturated heterocyclic ring include a 4- to 10-membered saturated heterocyclic ring. Specific examples of the saturated heterocyclic ring include an azetidine ring, an oxoazetidine ring, an oxetane ring, a tetrahydrofuran ring, a tetrahydropyran ring, a morpholine ring, a thiomorpholine ring, a pyrrolidine ring, a 2-oxopyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, an azepane ring, a dioxepane ring, and a 5,9-dioxaspiro[3.5]nonane ring.

As used herein, the molecular weight of a cyclic peptide or a solvent is represented in g/mol, unless otherwise stated. Also, in any case where a crystal of a cyclic peptide is a crystal of the free form of the cyclic peptide, a crystal of a solvate of the cyclic peptide, or a mixture thereof, the "molecular weight of the cyclic peptide" is on the basis of the molecular weight of the free form of the cyclic peptide.

The cyclic peptide according to one embodiment of the present invention may have a molecular weight (g/mol) of 1205 or more, 1206 or more, 1207 or more, 1208 or more, 1210 or more, 1220 or more, 1230 or more, 1250 or more, or 1300 or more, and may be 2800 or less, 2500 or less, 2000 or less, 1900 or less, 1800 or less, 1700 or less, or 1600 or less. The cyclic peptide of the present invention preferably has a molecular weight (g/mol) of 1204 or more and 3000 or less, more preferably 1300 or more and 1600 or less, and most preferably 1400 or more and 1500 or less.

The cyclic peptide according to one embodiment of the present invention may contain a cyclic moiety composed of 8 to 16 residues of amino acids in total, with a total number of amino acids being 8 to 20 residues. Also, the cyclic peptide according to one embodiment of the present invention may contain a cyclic moiety composed of 7 to 15 residues, 8 to 14 residues, 9 to 13 residues, 10 to 13 residues, 11 to 13 residues, 11 to 12 residues, or 11 residues of amino acids in total, with a total number of amino acids being 9 to 18 residues, 10 to 16 residues, 10 to 14 residues, 11 to 14 residues, 11 to 13 residues, 11 to 12 residues, or 11 residues. The cyclic peptide of the present invention preferably contains a cyclic moiety composed of 8 to 16 residues, with a total number of amino acids being 8 to 20 residues, more preferably contains a cyclic moiety composed of 11 to 13 residues, with a total number of amino acids being 11 to 14 residues, and most preferably contains a cyclic moiety composed of 11 residues of amino acids in total, with a total number of amino acids being 11 residues.

The cyclic peptide according to one embodiment of the present invention may contain at least 3 residues, 4 residues, or 5 residues of N-substituted amino acids, and in another embodiment, may contain at least 5 residues of N-substituted amino acids. Also, the cyclic peptide according to one embodiment of the present invention may contain at least 1 residue, 2 residues, or 3 residues of N-unsubstituted amino acids, and in another embodiment, may contain at least 3 residues of N-unsubstituted amino acids. The cyclic peptide of the present invention preferably contains at least 3 residues of N-substituted amino acids, more preferably contains at least 5 residues of N-substituted amino acids, and most preferably contains at least 7 residues of N-substituted amino acids.

The N-substituted amino acid contained in the cyclic peptide according to one embodiment of the present invention may be a N-alkylamino acid, may be a N-methylamino acid or a N-ethylamino acid in another embodiment, and may be a N-methylamino acid in another embodiment. The N-substituted amino acid contained in the cyclic peptide of the present invention is preferably a N-alkylamino acid, more preferably a N-methylamino acid or a N-ethylamino acid, and most preferably a N-methylamino acid.

The cyclic peptide according to one embodiment of the present invention may be a cyclic peptide having the following characteristics (I), (II), and (III):
(I) a characteristic of containing a cyclic moiety composed of 8 to 16 residues of amino acids in total, with a total number of amino acids being 8 to 20 residues;
(II) a characteristic of containing at least 2 residues of N-substituted amino acids; and
(III) a characteristic of having a molecular weight (g/mol) of 1204 or more and 3000 or less.

The cyclic peptide according to one embodiment of the present invention may be a cyclic peptide having the following characteristics (I) and (II):
(I) a characteristic of containing a cyclic moiety composed of 8 to 16 residues of amino acids in total, with a total number of amino acids being 8 to 20 residues; and
(II) a characteristic of containing at least 2 residues of N-substituted amino acids.

The cyclic peptide according to one embodiment of the present invention may contain at least one β-amino acid skeleton. Also, the cyclic peptide according to one embodiment of the present invention may contain at least one β-amino acid skeleton in the cyclic moiety.

The cyclic peptide according to one embodiment of the present invention may contain a cyclic moiety composed of a 28- to 55-, 28- to 49-, 31- to 46-, 34- to 43-, 34- to 40-, 34- to 37-, or 34-membered ring. The cyclic peptide according to one embodiment of the present invention may contain a cyclic moiety composed of a 34- to 40-membered ring, and the cyclic peptide according to another embodiment may contain a cyclic moiety composed of a 34-membered ring.

In the case where the cyclic peptide according to one embodiment of the present invention contains a cyclic moiety composed of a 34-membered ring, the above cyclic moiety composed of a 34-membered ring may be a cyclic peptide composed of 10 residues of α-amino acids and 1 residue of an amino acid having a β-amino acid skeleton, with a total of 11 residues of amino acids.

The cyclic peptide according to one embodiment of the present invention may have the following structure. 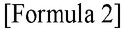 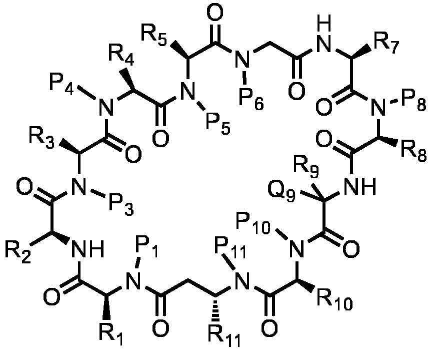 [Here, P₁, P₃, P₅, P₆, P₁₀, and P₁₁ are each a C₁ to C₆ alkyl; P₄ is a C₁ to C₆ alkyl or P₄ forms a 4-to 7-membered saturated heterocyclic ring together with the nitrogen atom to which P₄ is bonded, R₄, and the carbon atom to which R₄ is bonded; P₈ is a C₁ to C₆ alkyl or P₈ forms a 4- to 7-membered saturated heterocyclic ring together with the nitrogen atom to which P₈ is bonded, R₈, and the carbon atom to which R₈ is bonded, the 4- to 7-membered saturated heterocyclic ring being optionally substituted by a C₁ to C₆ alkoxy; R₁, R₂, R₃, R₅, R₇, and R₁₀ are each a hydrogen atom, a C₁ to C₆ alkyl, a C₃ to C₆ cycloalkyl, or an aralkyl optionally having a substituent; R₄ is a hydrogen atom or a C₁ to C₆ alkyl, except when R₄ and P₄ form a 4- to 7-membered saturated heterocyclic ring; R₈ is a hydrogen atom or a C₁ to C₆ alkyl, except when R₈ and P₈ form a 4- to 7-membered saturated heterocyclic ring; R₉ forms a 3- to 7-membered saturated carbocyclic ring together with Q₉ and the carbon atom to which R₉ and Q₉ are bonded; and R₁₁ is a hydrogen atom, a C₁ to C₆ alkyl, a di-C₁ to C₆ alkylaminocarbonyl, or a 4- to 8-membered cyclic aminocarbonyl.]

In one embodiment, P₁, P₃, P₅, P₆, P₁₀, and P₁₁ may be each methyl or ethyl. Also, in one embodiment, P₄ may be methyl, or form a 4-membered saturated heterocyclic ring together with the nitrogen atom to which P₄ is bonded, R₄, and the carbon atom to which R₄ is bonded. Also, in one embodiment, P₈ may form a 5-membered saturated heterocyclic ring together with the nitrogen atom to which P₈ is bonded, R₈, and the carbon atom to which R₈ is bonded, the 5-membered saturated heterocyclic ring being optionally substituted by a C₁ to C₆ alkoxy.

In one embodiment, R₁ and R₂ may be each a C₁ to C₆ alkyl; R₃ may be a hydrogen atom or a C₁ to C₆ alkyl; R₄ may be a hydrogen atom, except when R₄ and P₄ form a 4- to 7-membered saturated heterocyclic ring; R₅ may be a C₃ to C₆ cycloalkyl or benzyl optionally having a substituent; R₇ may be phenylethyl optionally having a substituent; R₉ may form a 5-membered saturated carbocyclic ring together with Q₉ and the carbon atom to which R₉ and Q₉ are bonded; R₁₀ may be a C₁ to C₆ alkyl or a C₃ to C₆ cycloalkyl; and R₁₁ may be methyl, a di-C₁ to C₆ alkylaminocarbonyl, or a 6-membered cyclic aminocarbonyl.

The ClogP of the cyclic peptide according to one embodiment of the present invention can be determined in accordance with the principle described in "CLOGP Reference Manual Daylight Version 4.9 (release date: August 1, 2011, https://www.daylight.com/dayhtml/doc/clogp/)". Examples of the method for calculating the ClogP include calculating using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

The ClogP of the cyclic peptide according to one embodiment of the present invention may be 5 or more, 6 or more, 7 or more, 8 or more, or 9 or more, and may be 24 or less, 23 or less, 22 or less, 21 or less, 20 or less, 19 or less, or 18 or less. Examples of the range of the ClogP of the cyclic peptide according to one embodiment of the present invention include 5 or more and 23 or less, 6 or more and 21 or less, 7 or more and 20 or less, 8 or more and 19 or less, 9 or more and 18 or less, 10 or more and 17 or less, 11 or more and 16.5 or less, and 11.2 or more and 16.1 or less. Also, the ClogP of the cyclic peptide according to one embodiment of the present invention may be 4 or more and 25 or less, and the ClogP of the cyclic peptide according to another embodiment may be 9 or more and 18 or less. In addition, the ClogP of the cyclic peptide according to one embodiment of the present invention may be larger than the ClogP of cyclosporin A (ClogP: 14.36). The ClogP of the cyclic peptide of the present invention is preferably 5 or more and 23 or less, more preferably 9 or more and 18 or less, and most preferably 11.2 or more and 16.1 or less.

It is preferred that the cyclic peptide according to one embodiment of the present invention has a ClogP/number of amino acid residues of 1.0 or more. The ClogP/number of amino acid residues is a value calculated by dividing the ClogP of a cyclic peptide by the number of amino acid residues contained in the cyclic peptide. For example, when the ClogP of a cyclic peptide is 14.0 and the number of amino acid residues contained in the cyclic peptide is 7, the ClogP/number of amino acid residues of the cyclic peptide is calculated to be 2.0.

The ClogP/number of amino acid residues of the cyclic peptide according to one embodiment of the present invention is preferably 0.3 or more, more preferably 0.5 or more, and most preferably 0.8 or more. The upper limit of the ClogP/number of amino acid residues of the cyclic peptide according to one embodiment of the present invention is preferably 2.3 or less, more preferably 1.9 or less, and most preferably 1.6 or less. Examples of the range of the ClogP/number of amino acid residues of the cyclic peptide according to one embodiment of the present invention include 0.3 or more and 2.3 or less, 0.4 or more and 2.3 or less, 0.5 or more and 1.9 or less, 0.7 or more and 1.8 or less, and 0.8 or more and 1.6 or less. The ClogP/number of amino acid residues of the cyclic peptide according to one embodiment may be 0.8 or more and 1.6 or less.

The solubility of the cyclic peptide according to one embodiment of the present invention in 50 mM phosphate buffer (pH 6.5) can be measured by a general method. For example, to freeze-dried powder of the compound, a 50 mM phosphate buffer solution (PPB: Phosphate buffer, pH 6.5) is added, and after shaking (1800 rpm, for 22 to 24 hours), the mixed solution is filtered through a filter, and the compound concentration of the filtrate is measured by LC/MS/MS. From the measured compound concentration, the solubility (µg/mL) can be calculated. It should be noted that the "solubility" means a solubility under the conditions of 25°C, 1 atm. The solubility of the cyclic peptide according to one embodiment of the present invention in 50 mM phosphate buffer (pH 6.5) may be 1200 mg/mL or less, 800 mg/mL or less, 600 mg/mL or less, 300 mg/mL or less, 200 mg/mL or less, 100 mg/mL or less, 50 mg/mL or less, 25 mg/mL or less, 10 mg/mL or less, 5.0 mg/mL or less, or 2.6 mg/mL or less, and may be 0.8 mg/mL or more, 0.9 mg/mL or more, 1.0 mg/mL or more, or 1.1 mg/mL or more. The solubility of the cyclic peptide according to one embodiment in 50 mM phosphate buffer (pH 6.5) may be 0.8 mg/mL or more, and may be 1.1 mg/mL or more in another embodiment.

Specific examples of the cyclic peptide having a solubility of 10 mg/mL or less in 50 mM phosphate buffer (pH 6.5) according to one embodiment of the present invention may include cyclosporin A.

The cyclic peptide crystals according to one embodiment of the present invention may have one or more diffraction peaks in powder X-ray diffraction using CuKα radiation. The cyclic peptide crystals according to another embodiment of the present invention may have two or more diffraction peaks in powder X-ray diffraction using CuKα radiation. The cyclic peptide crystals according to another embodiment of the present invention may have three or more diffraction peaks in powder X-ray diffraction using CuKα radiation.

Note that the powder X-ray diffraction using CuKα radiation can be measured under the conditions as follows, for example. Specifically, it can be measured using a powder X-ray diffractometer such as D8 Discover, 2D VÅNTEC-500 solid state detector (manufactured by Bruker), using CuKα as the radiation source, under the conditions where the tube voltage/tube current is 40 kV/40 mA or 50 kV/1000 µA, etc., and under the conditions where the measurement range is 5 to 31° and the exposure time is 40 to 600 seconds, etc.

The cyclic peptide crystals according to one embodiment of the present invention may be crystals having one or more diffraction peaks in powder X-ray diffraction using CuKα radiation, or having polarization in observation using a polarization microscope.

The cyclic peptide according to one embodiment of the present invention may be a cyclic peptide excluding cyclosporin A, the cyclic peptide according to another embodiment may be a cyclic peptide excluding (3s,9s,12s,17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide, and the cyclic peptide according to another embodiment may be a cyclic peptide excluding cyclosporin A and (3s,9s,12s,17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide. The cyclic peptide according to another embodiment of the present invention may be (3s,9s, 12s, 17s,20s,23s,27s,30s,36s)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1s)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.0^{12,15}]nonatriacontane-33,1'-cyclopentane]-27-carboxamide.

The term "amide-based solvent" as used herein means a solvent that contains an amide bond in the molecule. Examples of the "amide-based solvent" or "amide-based solvent with a molecular weight of 18 or more and 170 or less" as used herein may include formamide, N-methylformamide, N,N-dimethylformamide, N,N-dimethylacetamide, 2-pyrrolidone, and N-methylpyrrolidone. The "amide-based solvent" or "amide-based solvent with a molecular weight of 18 or more and 170 or less" in one embodiment of the present invention may be one or more selected from the group consisting of formamide, N-methylformamide, N,N-dimethylformamide, N,N-dimethylacetamide, 2-pyrrolidone, and N-methylpyrrolidone, and is preferably formamide, N-methylformamide, N,N-dimethylformamide, 2-pyrrolidone, or N-methylpyrrolidone, more preferably formamide or N,N-dimethylformamide, and most preferably formamide.

The term "sulfoxide-based solvent" as used herein means a solvent that falls under the category of sulfoxide. Examples of the "sulfoxide-based solvent" or "sulfoxide-based solvent with a molecular weight of 18 or more and 170 or less" as used herein may include dimethyl sulfoxide, phenyl methyl sulfoxide, and diethyl sulfoxide. The "sulfoxide-based solvent" or "sulfoxide-based solvent with a molecular weight of 18 or more and 170 or less" in one embodiment of the present invention may be one or more selected from the group consisting of dimethyl sulfoxide and diethyl sulfoxide, and is preferably dimethyl sulfoxide, phenyl methyl sulfoxide, or diethyl sulfoxide, more preferably dimethyl sulfoxide or diethyl sulfoxide, and most preferably dimethyl sulfoxide.

The term "hydrocarbon-based solvent" as used herein means a solvent that is solely composed of a carbon atom and a hydrogen atom.

The term "aromatic hydrocarbon-based solvent" as used herein means a hydrocarbon-based solvent that has one or more aromatic rings in the molecule. The "aromatic hydrocarbon-based solvent" as used herein is preferably a solvent that has one or more benzene rings in the molecule, and more preferably a solvent that has one benzene ring in the molecule. Examples of the "aromatic hydrocarbon-based solvent" or "aromatic hydrocarbon-based solvent with a molecular weight of 18 or more and 170 or less" as used herein may include benzene, toluene, xylene, ethylbenzene, tetralin, and cumene. The "aromatic hydrocarbon-based solvent" or "aromatic hydrocarbon-based solvent with a molecular weight of 170 or less" in one embodiment of the present invention may be one or more selected from the group consisting of benzene, toluene, xylene, ethylbenzene, tetralin, and cumene, and is preferably one or more selected from the group consisting of toluene, xylene, ethylbenzene, tetralin, and cumene, more preferably toluene, xylene, tetralin, or cumene, and most preferably toluene, tetralin, or cumene.

The term "halogen-based solvent" as used herein means a solvent that has one or more halogen atoms in the molecule. The "halogen-based solvent" as used herein is preferably a solvent that has one or more chlorine atoms and/or bromine atoms in the molecule, and more preferably a solvent that has one or more chlorine atoms in the molecule. Examples of the "halogen-based solvent" or "halogen-based solvent with a molecular weight of 18 or more and 170 or less" as used herein may include dichloromethane, chloroform, 1,2-dichloroethane, chlorobenzene, bromobenzene, and carbon tetrachloride. The "halogen-based solvent" or "halogen-based solvent with a molecular weight of 170 or less" in one embodiment of the present invention may be one or more selected from the group consisting of dichloromethane, chloroform, 1,2-dichloroethane, chlorobenzene, bromobenzene, and carbon tetrachloride, and is preferably one or more selected from the group consisting of dichloromethane, chloroform, 1,2-dichloroethane, and chlorobenzene, more preferably dichloromethane, chloroform, 1,2-dichloroethane, or chlorobenzene, and most preferably dichloromethane or chlorobenzene.

The term "alcohol-based solvent" as used herein means a solvent that has one or more hydroxy groups bonded to a carbon atom in the molecule. Examples of the "alcohol-based solvent" or "alcohol-based solvent with a molecular weight of 18 or more and 170 or less" as used herein may include methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, and benzyl alcohol. The "alcohol-based solvent" or "alcohol-based solvent with a molecular weight of 18 or more and 170 or less" in one embodiment of the present invention may be one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, and benzyl alcohol, and is preferably one or more selected from the group consisting of methanol, ethanol, 1-propanol, isopropanol, and n-butanol, more preferably methanol, ethanol, 1-propanol, isopropanol, or n-butanol, and most preferably ethanol.

The term "ether-based solvent" as used herein means a solvent that has one or more ether bonds in the molecule. Examples of the "ether-based solvent" or "ether-based solvent with a molecular weight of 18 or more and 170 or less" as used herein may include diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether. The "ether-based solvent" or "ether-based solvent with a molecular weight of 18 or more and 170 or less" in one embodiment of the present invention may be one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether, and is preferably one or more selected from the group consisting of tetrahydrofuran, 1,4-dioxane, diisopropyl ether, anisole, and t-butyl methyl ether, more preferably tetrahydrofuran, 1,4-dioxane, diisopropyl ether, anisole, or t-butyl methyl ether, and most preferably 1,4-dioxane, anisole, or t-butyl methyl ether.

The term "ester-based solvent" as used herein means a solvent that has one or more ester bonds in the molecule. Examples of the "ester-based solvent" or "ester-based solvent with a molecular weight of 18 or more and 170 or less" as used herein may include ethyl formate, methyl acetate, ethyl acetate, methyl propionate, n-butyl acetate, propyl acetate, isopropyl acetate, isobutyl acetate, pentyl acetate, and γ-valerolactone. The "ester-based solvent" or "ester-based solvent with a molecular weight of 18 or more and 170 or less" in one embodiment of the present invention may be one or more selected from the group consisting of ethyl formate, methyl acetate, ethyl acetate, methyl propionate, n-butyl acetate, propyl acetate, isopropyl acetate, isobutyl acetate, pentyl acetate, and γ-valerolactone, and is preferably one or more selected from the group consisting of ethyl acetate, isopropyl acetate, and n-butyl acetate, more preferably ethyl acetate, isopropyl acetate, or n-butyl acetate, and most preferably ethyl acetate or n-butyl acetate.

The term "nitrile-based solvent" as used herein means a solvent that has one or more cyano groups bonded to a carbon atom in the molecule. Examples of the "nitrile-based solvent" or "nitrile-based solvent with a molecular weight of 18 or more and 170 or less" as used herein may include acetonitrile, benzonitrile, and propionitrile. The "nitrile-based solvent" or "nitrile-based solvent with a molecular weight of 18 or more and 170 or less" in one embodiment of the present invention may be one or more selected from the group consisting of acetonitrile and propionitrile, and is preferably one or more selected from the group consisting of acetonitrile, benzonitrile, and propionitrile, more preferably acetonitrile, benzonitrile, or propionitrile, and most preferably acetonitrile.

The term "ketone-based solvent" as used herein means a solvent represented by R₁-C(=O)-R₂, where R₁ and R₂ are each independently an alkyl group, an aryl group, or a heteroaryl group, or R₁ and R₂ together form an alkylene group. Examples of the "ketone-based solvent" or "ketone-based solvent with a molecular weight of 18 or more and 170 or less" as used herein may include acetone, methyl ethyl ketone, methyl isobutyl ketone, methyl butyl ketone, cyclohexanone, diethyl ketone, cyclopentanone, and 3-acetylpyridine. The "ketone-based solvent" or "ketone-based solvent with a molecular weight of 18 or more and 170 or less" in one embodiment of the present invention may be one or more selected from the group consisting of acetone, methyl ethyl ketone, methyl isobutyl ketone, methyl butyl ketone, cyclohexanone, diethyl ketone, cyclopentanone, and 3-acetylpyridine, and is preferably one or more selected from the group consisting of acetone, methyl ethyl ketone, cyclohexanone, methyl isobutyl ketone, and 3-acetylpyridine, more preferably acetone, methyl ethyl ketone, cyclohexanone, or methyl isobutyl ketone, and most preferably acetone or methyl ethyl ketone.

The term "aliphatic hydrocarbon-based solvent" as used herein means a hydrocarbon-based solvent that has no aromatic ring in the molecule. Examples of the "aliphatic hydrocarbon-based solvent" or "aliphatic hydrocarbon-based solvent with a molecular weight of 18 or more and 170 or less" as used herein may include n-pentane, n-hexane, n-heptane, n-octane, cyclopentane, cyclohexane, methylcycloheptane, and methylcyclohexane. The "aliphatic hydrocarbon-based solvent" or "aliphatic hydrocarbon-based solvent with a molecular weight of 18 or more and 170 or less" in one embodiment of the present invention may be one or more selected from the group consisting of n-pentane, n-hexane, n-heptane, n-octane, cyclopentane, cyclohexane, methylcycloheptane, and methylcyclohexane, and is preferably one or more selected from the group consisting of n-heptane, cyclohexane, and methylcyclohexane, more preferably n-heptane, cyclohexane, or methylcyclohexane, and most preferably n-heptane or cyclohexane.

The "water-soluble organic solvent" as used herein means an organic solvent that is miscible with water in an arbitrary proportion. Examples of the "water-soluble organic solvent" as used herein may include an alcohol-based solvent, an amide-based solvent, a nitrile-based solvent, and a sulfoxide-based solvent. The "water-soluble organic solvent" in one embodiment of the present invention may be an alcohol-based solvent, an amide-based solvent, a nitrile-based solvent, or a sulfoxide-based solvent, and is preferably an alcohol-based solvent or a sulfoxide-based solvent, more preferably methanol, ethanol, 1-propanol, 2-propanol or dimethyl sulfoxide, and most preferably ethanol or dimethyl sulfoxide.

The "PEG-based solvent" as used herein means a solvent represented by R₁(OCHR₃CH₂)nOR₂, or a mixture thereof, where n is a natural number of 1 or more, R¹ and R² are each independently hydrogen, a C₁ to C₄ alkyl, or -C(=O)R⁴, R³ is hydrogen or a C₁ to C₄ alkyl, and R⁴ is a C₁ to C₁₈ alkyl optionally substituted with a hydroxy group or a C₁ to C₁₈ alkenyl optionally substituted with a hydroxy group. The "PEG-based solvent" in one embodiment of the present invention may be (i) a solvent represented by R¹(OCHR³CH₂)nOR², n being a natural number of 1 or more and 10 or less, or (ii) a mixture of solvents represented by R¹(OCHR³CH₂)nOR², an average of n being 3 to 100, where R¹ and R² are each independently hydrogen, a C₁ to C₄ alkyl, or -C(=O)R⁴, R³ is hydrogen or a C₁ to C₄ alkyl, and R⁴ is a C₁ to C₁₈ alkyl optionally substituted with a hydroxy group or a C₁ to C₁₈ alkenyl optionally substituted with a hydroxy group. The "PEG-based solvent" in one preferred embodiment of the present invention may be (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or a C₁ to C₄ alkyl, R² is hydrogen, a C₁ to C₄ alkyl, or - C(=O)R⁴, R³ is hydrogen or a C₁ to C₄ alkyl, and R⁴ is a C₁₀ to C₁₈ alkyl. The "PEG-based solvent" in one more preferred embodiment of the present invention may be (i) a solvent represented by R¹(OCHR³CH₂)nOR², n being a natural number of 1, 2, 3, or 4, or (ii) a mixture of solvents represented by R¹(OCHR³CH₂)nOR², an average of n being 3 to 100, where R¹ is hydrogen or methyl, R² is hydrogen, methyl, or -C(=O)R⁴, R³ is hydrogen or methyl, and R⁴ is a C₁₁ to C₁₇ alkyl. The "PEG-based solvent" in one still more preferred embodiment of the present invention may be diglyme, triglyme, tetraglyme, ethylene glycol, diethylene glycol, triethylene glycol, tetraethylene glycol, polyethylene glycol, polypropylene glycol, or a polyethylene glycol monofatty acid ester. The "PEG-based solvent" in one even more preferred embodiment of the present invention may be polyethylene glycol, and in this case, the number average molecular weight of the polyethylene glycol may be 150 to 5000, and may be preferably 360 to 440, 540 to 660, 900 to 1100, or 1800 to 2200. Also, the polyethylene glycol may be PEG400, PEG600, PEG1000, or PEG2000. The "PEG-based solvent" in another even more preferred embodiment of the present invention may be a polyethylene glycol monofatty acid ester, and in this case, the polyethylene glycol monofatty acid ester may be polyethylene glycol monostearate or polyethylene glycol monolaurate. The "PEG-based solvent" in the present invention is preferably diglyme, triglyme, tetraglyme, ethylene glycol, diethylene glycol, triethylene glycol, tetraethylene glycol, polyethylene glycol, polypropylene glycol, or a polyethylene glycol monofatty acid ester, more preferably polypropylene glycol or a polyethylene glycol monofatty acid ester, and most preferably a polyethylene glycol monofatty acid ester.

One aspect of the present disclosure is a method for producing cyclic peptide crystals, the method comprising a step of bringing a cyclic peptide into contact with a solvent.

The solvent in the production method according to one aspect of the present disclosure may be any solvent selected from the group consisting of the following (1), (2), and (3):
(1) (i) a solvent with a molecular weight of 18 or more and 170 or less, or (ii) a mixed solvent containing two or more solvents with a molecular weight of 18 or more and 170 or less;
(2) water containing 0.01 to 30 wt/v% of a surfactant and 5 to 50 v/v% of a water-soluble organic solvent, based on the total amount of the solvent; and
(3) (i) a PEG-based solvent, or (ii) a mixed solvent containing one or more selected from the group consisting of an alcohol-based solvent, an aliphatic hydrocarbon-based solvent, and water, and a PEG-based solvent.

The solvent in the production method according to one aspect of the present disclosure may be (i) a solvent with a molecular weight of 18 or more and 170 or less, or (ii) a mixed solvent containing two or more solvents with a molecular weight of 18 or more and 170 or less.

In the case where the solvent in the production method according to one aspect of the present disclosure is (i) a solvent with a molecular weight of 18 or more and 170 or less, or (ii) a mixed solvent containing two or more solvents with a molecular weight of 18 or more and 170 or less, the solvent may be a solvent selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a ketone-based solvent, an aliphatic hydrocarbon-based solvent, ethylene glycol, and water, or it may be a solvent selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent. It is preferably a solvent selected from the group consisting of an amide-based solvent, a sulfoxide-based solvent, an aromatic hydrocarbon-based solvent, a halogen-based solvent, and an ester-based solvent, more preferably a solvent selected from the group consisting of an amide-based solvent, a sulfoxide-based solvent, an aromatic hydrocarbon-based solvent, and a halogen-based solvent, and most preferably a solvent selected from the group consisting of an amide-based solvent, a sulfoxide-based solvent, and an aromatic hydrocarbon-based solvent.

The solvent in the production method according to one aspect of the present disclosure may be, in one embodiment, solvent (A) with a molecular weight of 18 or more and 170 or less, or a mixed solvent of solvent (A) with a molecular weight of 18 or more and 170 or less and solvent (B) with a molecular weight of 18 or more and 170 or less, and may be, in another embodiment, solvent (A) or a mixed solvent containing solvent (A) and solvent (B). In these cases, the above solvent (A) may be one or more selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent, and the above solvent (B) may be one or more selected from the group consisting of an aliphatic hydrocarbon-based solvent, ethylene glycol, and water. Also, in these cases, the above solvent (A) may be selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent, and the above solvent (B) may be selected from the group consisting of an aliphatic hydrocarbon-based solvent, ethylene glycol, and water.

For example, the above solvent (A) may be one or more selected from the group consisting of an amide-based solvent, a sulfoxide-based solvent, an aromatic hydrocarbon-based solvent, a halogen-based solvent, and an ester-based solvent, may be one or more selected from the group consisting of an amide-based solvent, a sulfoxide-based solvent, an aromatic hydrocarbon-based solvent, and a halogen-based solvent, may be one or more selected from the group consisting of an amide-based solvent, a sulfoxide-based solvent, and an aromatic hydrocarbon-based solvent, may be an amide-based solvent, may be a sulfoxide-based solvent, may be an aromatic hydrocarbon-based solvent, may be a halogen-based solvent, may be an alcohol-based solvent, may be an ether-based solvent, may be an ester-based solvent, may be a nitrile-based solvent, or may be a ketone-based solvent. The above solvent (A) is preferably one or more selected from the group consisting of an amide-based solvent, a sulfoxide-based solvent, an aromatic hydrocarbon-based solvent, a halogen-based solvent, and an ester-based solvent, more preferably an amide-based solvent, a sulfoxide-based solvent, an aromatic hydrocarbon-based solvent, or a halogen-based solvent, and most preferably an amide-based solvent, a sulfoxide-based solvent, or an aromatic hydrocarbon-based solvent.

For example, the above solvent (B) may be one or more solvents selected from the group consisting of an aliphatic hydrocarbon-based solvent, ethylene glycol, and water, may be an aliphatic hydrocarbon-based solvent, ethylene glycol, or water, may be an aliphatic hydrocarbon-based solvent, or may be water. The above solvent (B) is preferably one or more solvents selected from the group consisting of an aliphatic hydrocarbon-based solvent, ethylene glycol, and water, more preferably an aliphatic hydrocarbon-based solvent, ethylene glycol, or water, and most preferably n-heptane, cyclohexane, or water.

In the solvent in the production method according to one aspect of the present disclosure, the volume ratio (v/v) between the above solvent (A) and the above solvent (B) may be 1:0 to 1:40, may be 1:0 to 1:30, 1:0 to 1:20, 1:0 to 1:10, 1:0 to 1:7, or 1:0 to 1:5, may be 1:0 to 1:4, may be 10:1 to 1:40, may be 5:1 to 1:30, 3:1 to 1:20, 2:1 to 1:10, 1:1 to 1:7, or 1:2 to 1:5, or may be 1:2 to 1:4. The volume ratio (v/v) between the above solvent (A) and the above solvent (B) in the solvent is preferably 1:0 to 1:4, more preferably 1:1 to 1:4, and most preferably 1:2 to 1:4.

In the solvent in the production method according to one aspect of the present disclosure, the above solvent (A) may be any solvent with a molecular weight of 18 or more and 170 or less, and may be a solvent with a molecular weight of 18 or more and 160 or less, a molecular weight of 18 or more and 150 or less, a molecular weight of 18 or more and 140 or less, or a molecular weight of 32 or more and 135 or less. The above solvent (A) preferably has a molecular weight of 18 or more and 150 or less, more preferably a molecular weight of 18 or more and 140 or less, and most preferably a molecular weight of 32 or more and 135 or less.

In the solvent in the production method according to one aspect of the present disclosure, the above solvent (B) may be any solvent with a molecular weight of 18 or more and 170 or less, and may be a solvent with a molecular weight of 18 or more and 160 or less, a molecular weight of 18 or more and 135 or less, a molecular weight of 18 or more and 120 or less, or a molecular weight of 18 or more and 105 or less. The above solvent (B) is preferably a solvent having a molecular weight of 18 or more and 135 or less, more preferably a molecular weight of 18 or more and 120 or less, and most preferably a molecular weight of 18 or more and 105 or less.

In the solvent in the production method according to one aspect of the present disclosure, the above solvent (A) and the above solvent (B) may have a melting point of 25°C or lower.

The solvent in the production method according to one aspect of the present disclosure may be water containing 0.01 to 30 wt/v% of a surfactant and 5 to 50 v/v% of a water-soluble organic solvent, based on the total amount of the solvent.

In the case where the solvent in the production method according to one aspect of the present disclosure is water containing 0.01 to 30 wt/v% of a surfactant and 5 to 50 v/v% of a water-soluble organic solvent, based on the total amount of the solvent, the above surfactant may be, in one embodiment, one or more selected from the group consisting of a cationic surfactant, an anionic surfactant, an amphoteric surfactant, and a nonionic surfactant. It may be, in another embodiment, one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, a lecithin, an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester, and may be, in still another embodiment, one or more selected from the group consisting of an alkyl sulfate, an alkyl aryl polyoxyethylene ether, a polyoxyethylene glycerin fatty acid ester, and a polyoxyethylene sorbitan fatty acid ester. It is preferably an alkyl sulfate, an alkyl aryl polyoxyethylene ether, a polyoxyethylene glycerin fatty acid ester, or a polyoxyethylene sorbitan fatty acid ester, more preferably sodium lauryl sulfate, 4-(1,1,3,3-tetramethylbutyl)phenyl-polyethylene glycol (Triton X-100 (R)), Polyoxyl 35 Hydrogenated Castor Oil (Cremophor EL (R)), or polyoxyethylene sorbitan monolaurate (Tween (R)), and most preferably Polyoxyl 35 Hydrogenated Castor Oil (Cremophor EL (R)).

In the case where the solvent in the production method according to one aspect of the present disclosure is water containing 0.01 to 30 wt/v% of a surfactant and 5 to 50 v/v% of a water-soluble organic solvent, based on the total amount of the solvent, the above surfactant may be an ionic surfactant or a nonionic surfactant in one embodiment, may be an ionic surfactant in another embodiment, and may be a nonionic surfactant in still another embodiment.

The ionic surfactant in one embodiment of the present invention may be one or more selected from the group consisting of a cationic surfactant, an anionic surfactant, and an amphoteric surfactant, and it may be, in another embodiment, one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, and a lecithin. It is preferably an alkyl sulfate, more preferably a lauryl sulfate, and most preferably sodium lauryl sulfate.

The nonionic surfactant in one embodiment of the present invention may be one or more selected from the group consisting of an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester. It may be preferably one or more selected from the group consisting of an alkyl aryl polyoxyethylene ether, a polyoxyethylene glycerin fatty acid ester, and a polyoxyethylene sorbitan fatty acid ester, more preferably one or more selected from the group consisting of 4-(1,1,3,3-tetramethylbutyl)phenyl-polyethylene glycol (Triton X-100 (R)), Polyoxyl 35 Hydrogenated Castor Oil (Cremophor EL (R)), and polyoxyethylene sorbitan monolaurate (Tween (R)), and most preferably Polyoxyl 35 Hydrogenated Castor Oil (Cremophor EL (R)).

In the case where the solvent in the production method according to one aspect of the present disclosure is water containing 0.01 to 30 wt/v% of a surfactant and 5 to 50 v/v% of a water-soluble organic solvent, based on the total amount of the solvent, in one embodiment, the content of the above surfactant may be 0.02 to 20 wt/v%, 0.05 to 15 wt/v%, 0.1 to 10 wt/v%, 0.12 to 8 wt/v%, 0.15 to 5 wt/v%, or 0.18 to 3 wt/v% based on the total amount of the solvent, and the content of the above water-soluble organic solvent may be 5 to 40 v/v%, 5 to 30 v/v%, 5 to 25 v/v%, 8 to 20 v/v%, or 10 to 15 v/v% based on the total amount of the solvent. Preferably, the content of the above surfactant is 0.01 to 30 wt/v% based on the total amount of the solvent, and the content of the above water-soluble organic solvent is 5 to 40 v/v% based on the total amount of the solvent. More preferably, the content of the above surfactant is 0.05 to 15 wt/v% based on the total amount of the solvent, and the content of the above water-soluble organic solvent is 5 to 30 v/v% based on the total amount of the solvent. Most preferably, the content of the above surfactant is 0.1 to 10 wt/v% based on the total amount of the solvent, and the content of the above water-soluble organic solvent is 5 to 25 v/v% based on the total amount of the solvent.

The solvent in the production method according to one aspect of the present disclosure may be (i) a PEG-based solvent, or (ii) a mixed solvent containing one or more selected from the group consisting of an alcohol-based solvent, an aliphatic hydrocarbon-based solvent, and water, and a PEG-based solvent.

The solvent in the production method according to one aspect of the present disclosure may be a PEG-based solvent.

The solvent in the production method according to one aspect of the present disclosure may be a mixed solvent of one or more selected from the group consisting of an alcohol-based solvent, an aliphatic hydrocarbon-based solvent, and water, and a PEG-based solvent.

The "one or more selected from the group consisting of an alcohol-based solvent, an aliphatic hydrocarbon-based solvent, and water" in one embodiment of the present invention may be, for example, one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, benzyl alcohol, n-pentane, n-hexane, n-heptane, n-octane, cyclopentane, cyclohexane, methylcycloheptane, and water. They are preferably one or more selected from the group consisting of methanol, ethanol, 1-propanol, 2-propanol, n-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, benzyl alcohol, n-pentane, n-hexane, n-heptane, n-octane, cyclopentane, cyclohexane, methylcycloheptane, and water, more preferably one or more selected from the group consisting of 2-propanol, n-heptane, and water, and most preferably 2-propanol, n-heptane, or water.

The production method according to one aspect of the present disclosure can be carried out by, for example, the following method. The cyclic peptide can be dissolved in the solvent and shaken for a predetermined time under predetermined temperature conditions to bring it into contact with the solvent, thereby producing cyclic peptide crystals.

In the step of bringing a cyclic peptide into contact with a solvent, included in the production method according to one aspect of the present disclosure, the concentration of the cyclic peptide may be 1 mg to 2000 mg/mL, and may be 5 mg to 1500 mg/mL, 10 mg to 1000 mg/mL, 10 mg to 500 mg/mL, 10 mg to 100 mg/mL, 10 mg to 50 mg/mL, 100 mg to 400 mg/mL, 100 mg to 200 mg/mL, or 500 mg to 1000 mg/mL.

The cyclic peptide used in the step of bringing a cyclic peptide into contact with a solvent, included in the production method according to one aspect of the present disclosure, may be a freeze-dried product, and may be, for example, a freeze-dried product from a dimethyl sulfoxide solution.

The cyclic peptide used in the step of bringing a cyclic peptide into contact with a solvent, included in the production method according to one aspect of the present disclosure, may be 0.5 mg to 200 kg in one embodiment, may be 1 mg to 1 g in another embodiment, and may be 0 g to 200 kg, 100 g to 100 kg, 0.5 mg to 10 g, 1 mg to 1 g, 1 mg to 100 mg, 1 mg to 10 mg, or 1 mg to 5 mg in still another embodiment.

In one embodiment of the production method according to one aspect of the present disclosure, the step of bringing a cyclic peptide into contact with a solvent does not comprise adding seed crystals. In another embodiment, the step of bringing a cyclic peptide into contact with a solvent comprises adding seed crystals. The seed crystals may be cyclic peptide crystals produced by the production method according to one aspect of the present disclosure, or may be cyclic peptide crystals produced by other methods.

In one embodiment of the production method according to one aspect of the present disclosure, the method further comprises a filtration step after the step of bringing a cyclic peptide into contact with a solvent. The filtration step is a step of filtering the solvent containing the cyclic peptide crystals to collect the crystals by solid-liquid separation. The filtration step can be carried out by, for example, filtering the solvent containing the cyclic peptide crystals through filter paper or the like, and collecting the cyclic peptide crystals by filtration.

In the production method according to one aspect of the present disclosure, the step of bringing a cyclic peptide into contact with a solvent may be performed at a temperature of -10°C to 120°C for 30 minutes to 12 weeks in one embodiment, and may be performed at a constant temperature of 20°C to 90°C for 12 hours to 7 days in one preferred embodiment. Also, in the production method according to one aspect of the present disclosure, the step of bringing a cyclic peptide into contact with a solvent may be, in one embodiment, performed at a constant temperature in 0°C to 110°C, 10°C to 100°C, 15°C to 90°C, or 20°C to 80°C, or performed repeating heating and cooling between a lower limit temperature of 10°C, 20°C, 30°C, 40°C, 45°C, 50°C, or 55°C and an upper limit temperature of 100°C, 90°C, 85°C, 80°C, 75°C, 70°C, or 60°C, 10 times or more, 20 times or more, or 30 times or more, and 1000 times or less, 500 times or less, or 100 times or less, and may be performed for 1 hour to 6 weeks, 2 hours to 4 weeks, 4 hours to 2 weeks, or 6 hours to 7 days. The step may be, in one preferred embodiment, performed repeating heating and cooling between a lower limit temperature of 50°C and an upper limit temperature of 90°C, 30 times or more and 100 times or less, and may be performed for 12 hours to 7 days.

That the cyclic peptide crystals have been produced by the production method according to one aspect of the present disclosure can be confirmed by, for example, observing the presence of diffraction peaks in powder X-ray diffraction using CuKα radiation and the polarization and appearance using a polarization microscope.

Another aspect of the present disclosure is a method for screening cyclic peptide crystals, the method comprising the following steps (a) and (b):
(a) a step of producing cyclic peptide crystals by the method for producing cyclic peptide crystals according to one aspect of the present disclosure; and
(b) a step of analyzing the crystals by powder X-ray crystal diffraction. In this case, the crystals to be screened may include crystals produced using a solvent containing formamide in one embodiment, may include crystals produced using a solvent containing formamide and a solvent containing dimethyl sulfoxide in one preferred embodiment, and may include crystals produced using a solvent containing formamide, a solvent containing dimethyl sulfoxide, a solvent containing toluene, and a solvent containing dichloromethane in one more preferred embodiment. Also, in this case, the crystals to be screened may include crystals produced using a solvent containing Polyoxyl 35 Hydrogenated Castor Oil (Cremophor EL (R)) in one embodiment, may include crystals produced using a solvent containing a polyethylene glycol monofatty acid ester in another embodiment, may include crystals produced using a solvent containing polyethylene glycol in still another embodiment, may include crystals produced using a solvent containing Polyoxyl 35 Hydrogenated Castor Oil (Cremophor EL (R)), a solvent containing a polyethylene glycol monofatty acid ester, and a solvent containing polyethylene glycol in one preferred embodiment, and may include crystals produced using a solvent containing Polyoxyl 35 Hydrogenated Castor Oil (Cremophor EL (R)), crystals produced using a solvent containing a polyethylene glycol monofatty acid ester, and crystals produced using a solvent containing formamide in another preferred embodiment.

Another aspect of the present disclosure is a method for screening a method for crystallizing a cyclic peptide, the method comprising the following steps (a) and (b):
(a) a step of producing cyclic peptide crystals by the method for producing cyclic peptide crystals according to one aspect of the present disclosure; and
(b) a step of analyzing the crystals by powder X-ray crystal diffraction. In this case, the crystallization method to be screened may include a crystallization method using a solvent containing formamide in one embodiment, may include a crystallization method using a solvent containing formamide and a solvent containing dimethyl sulfoxide in one preferred embodiment, and may include a crystallization method using a solvent containing formamide, a solvent containing dimethyl sulfoxide, a solvent containing toluene, and a solvent containing dichloromethane in one more preferred embodiment. Also, in this case, the crystallization method to be screened may include a crystallization method using a solvent containing Polyoxyl 35 Hydrogenated Castor Oil (Cremophor EL (R)) in one embodiment, may include a crystallization method using a solvent containing a polyethylene glycol monofatty acid ester in another embodiment, may include a crystallization method using a solvent containing polyethylene glycol in still another embodiment, may include a crystallization method using a solvent containing Polyoxyl 35 Hydrogenated Castor Oil (Cremophor EL (R)), a solvent containing a polyethylene glycol monofatty acid ester, and a solvent containing polyethylene glycol in one preferred embodiment, and may include a crystallization method using a solvent containing Polyoxyl 35 Hydrogenated Castor Oil (Cremophor EL (R)), a solvent containing a polyethylene glycol monofatty acid ester, and a solvent containing formamide in another preferred embodiment.

In the step of bringing a cyclic peptide into contact with a solvent, included in the above method for screening a method for crystallizing a cyclic peptide, the cyclic peptide used per condition may be 0.5 mg to 10 mg.

In the above method for screening a method for crystallizing a cyclic peptide, the crystals or crystallization method to be screened may be of 2 or more types, 5 or more types, 10 or more types, 15 or more types, or 20 or more types.

In one embodiment, the step of bringing a cyclic peptide into contact with a solvent, included in the above method for screening a method for crystallizing a cyclic peptide, may not comprise adding seed crystals.

Another aspect of the present disclosure is a method for increasing the possibility that cyclic peptide crystals are produced, the method comprising a step of producing cyclic peptide crystals by the method for producing cyclic peptide crystals according to one aspect of the present disclosure.

Another aspect of the present disclosure is use of a solvent in production of cyclic peptide crystals. In one embodiment, the solvent may be a solvent selected from the group consisting of an amide-based solvent, a sulfoxide-based solvent, an aromatic hydrocarbon-based solvent, a halogen-based solvent, a polyoxyethylene glycerin fatty acid ester, and a polyethylene glycol monofatty acid ester. In another embodiment, the solvent may be an amide-based solvent, and in one preferred embodiment, the solvent may be formamide. In another embodiment, the solvent may be a sulfoxide-based solvent, and in one preferred embodiment, the solvent may be dimethyl sulfoxide. In another embodiment, the solvent may be an aromatic hydrocarbon-based solvent, and in one preferred embodiment, the solvent may be toluene, tetralin, or cumene. In another preferred embodiment, the solvent may be Polyoxyl 35 Hydrogenated Castor Oil (Cremophor EL (R)), in still another preferred embodiment, the solvent may be a polyethylene glycol monofatty acid ester, and in still another preferred embodiment, the solvent may be polyethylene glycol.

Another aspect of the present disclosure is a screening kit for producing cyclic peptide crystals, wherein cyclic peptide crystals are produced by the use of a solvent in production of cyclic peptide crystals according to one aspect of the present disclosure.

Another aspect of the present disclosure is a method for purifying a cyclic peptide, the method comprising a step of producing cyclic peptide crystals by the method for producing cyclic peptide crystals according to one aspect of the present disclosure, and a step of collecting the crystals by solid-liquid separation. The step of collecting the crystals by solid-liquid separation may be, for example, a filtration step.

Another aspect of the present disclosure is a method for producing a cyclic peptide, the method comprising a step of producing cyclic peptide crystals containing formamide. **In** one embodiment, the production method may be the method for producing cyclic peptide crystals according to one aspect of the present disclosure. That is, the crystals produced by the method for producing cyclic peptide crystals according to one aspect of the present disclosure may be crystals of a cyclic peptide formamide solvate.

Another aspect of the present disclosure is a method for producing a cyclic peptide, the method comprising a step of producing cyclic peptide crystals containing dimethyl sulfoxide. In one embodiment, the production method may be the method for producing cyclic peptide crystals according to one aspect of the present disclosure. That is, the crystals produced by the method for producing cyclic peptide crystals according to one aspect of the present disclosure may be crystals of a cyclic peptide dimethyl sulfoxide solvate.

Another aspect of the present disclosure is a method for producing a cyclic peptide, the method comprising a step of producing cyclic peptide crystals containing an aromatic hydrocarbon-based solvent. In one embodiment, the production method may be the method for producing cyclic peptide crystals according to one aspect of the present disclosure. That is, the crystals produced by the method for producing cyclic peptide crystals according to one aspect of the present disclosure may be crystals of a cyclic peptide aromatic hydrocarbon-based solvate.

Another aspect of the present disclosure is a method for producing a cyclic peptide, the method comprising a step of producing cyclic peptide crystals containing toluene, tetralin, or cumene. In one embodiment, the production method may be the method for producing cyclic peptide crystals according to one aspect of the present disclosure. That is, the crystals produced by the method for producing cyclic peptide crystals according to one aspect of the present disclosure may be crystals of a cyclic peptide toluene solvate, tetralin solvate, or cumene solvate.

Another aspect of the present disclosure is a method for producing a cyclic peptide, the method comprising a step of producing cyclic peptide crystals containing Polyoxyl 35 Hydrogenated Castor Oil (Cremophor EL (R)). In one embodiment, the production method may be the method for producing cyclic peptide crystals according to one aspect of the present disclosure. That is, the crystals produced by the method for producing cyclic peptide crystals according to one aspect of the present disclosure may be a cyclic peptide hydrate, or may be crystals of a cyclic peptide Polyoxyl 35 Hydrogenated Castor Oil (Cremophor EL (R)) solvate.

Another aspect of the present disclosure is a method for producing a cyclic peptide, the method comprising a step of producing cyclic peptide crystals containing a polyethylene glycol monofatty acid ester. In one embodiment, the production method may be the method for producing cyclic peptide crystals according to one aspect of the present disclosure. That is, the crystals produced by the method for producing cyclic peptide crystals according to one aspect of the present disclosure may be a cyclic peptide hydrate, or may be crystals of a cyclic peptide polyethylene glycol monofatty acid ester solvate.

Another aspect of the present disclosure is a method for producing a cyclic peptide, the method comprising a step of producing cyclic peptide crystals containing polyethylene glycol. In one embodiment, the production method may be the method for producing cyclic peptide crystals according to one aspect of the present disclosure. That is, the crystals produced by the method for producing cyclic peptide crystals according to one aspect of the present disclosure may be a cyclic peptide hydrate, or may be crystals of a cyclic peptide polyethylene glycol solvate.

### [Examples]

The contents of the present invention will be further described by the following Examples, but the present invention is not limited to their contents. Except those as specifically described, starting substances, starting raw materials, solvents, and reagents were obtained from commercial suppliers, or synthesized using known methods.

### [Example 1] Synthesis of cyclic peptide

Cyclic peptides CP01 to CP08 (they are also referred to simply as compounds CP01 to CP08.) shown in Table 1 were synthesized by the same method as described in International Publication No. WO 2013/100132, International Publication No. WO 2018/225864, or International Publication No. WO 2021/90855, and the final products were obtained as dry products. Specifically, compound 2118 in International Publication No. WO 2021/90855 corresponds to compound CP01, compound 1787 corresponds to compound CP02, compound 926 corresponds to compound CP03, compound 1147 corresponds to compound CP04, compound 1217 corresponds to compound CP05, compound 1201 corresponds to compound CP06, compound 301 corresponds to compound CP07, and compound 640 corresponds to compound CP08. Table 1 shows the structural formulas of compounds CP01 to CP08.

**[Table 1]**

| Compound No. | Structural formula |
|---|---|
| CP01 | 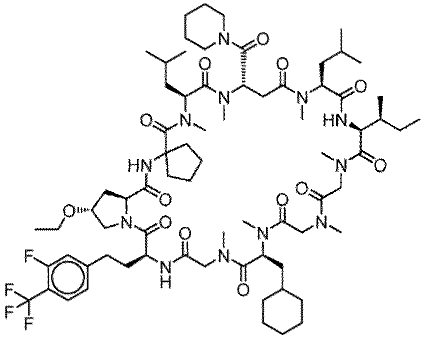 |
| CP02 | 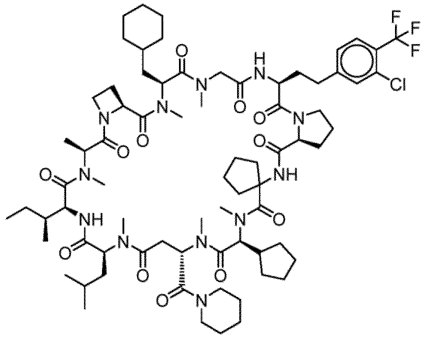 |
| CP03 | 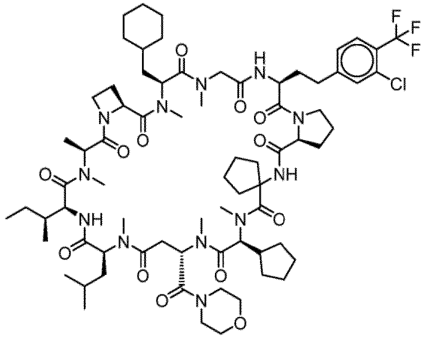 |
| CP04 | 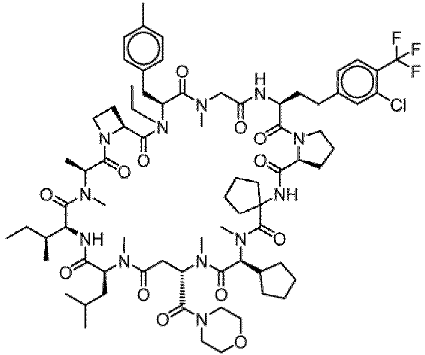 |
| CP05 | 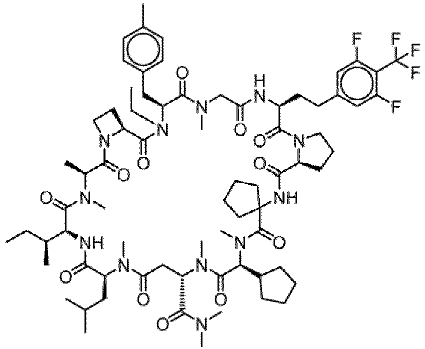 |
| CP06 | 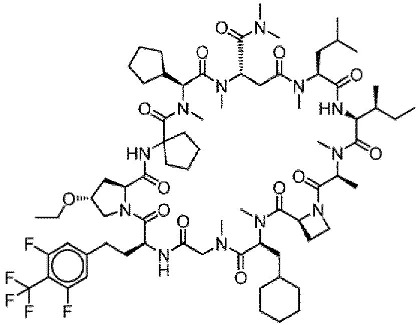 |
| CP07 | 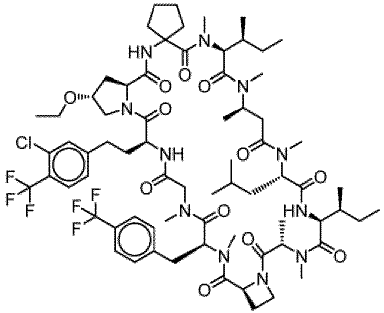 |
| CP08 | 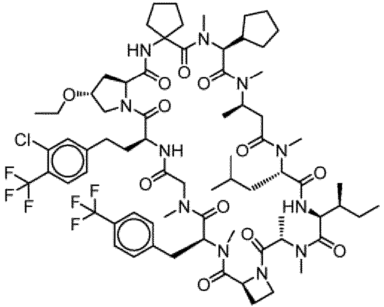 |

Note that the molecular weights of compounds CP01 to CP08 are as follows.
Compound CP01: 1443.8
Compound CP02: 1454.2
Compound CP03: 1456.2
Compound CP04: 1478.2
Compound CP05: 1437.7
Compound CP06: 1459.7
Compound CP07: 1451.1
Compound CP08: 1463.1

Also, the IUPAC names of compounds CP01 to CP08 are as follows.
Compound CP01: (3S,9S,18S,21S,25S,28S,34S,36R)-9-(cyclohexylmethyl)-36-ethoxy-3-[2-[3-fluoro-4-(trifluoromethyl)phenyl]ethyl]-21,28-diisobutyl-7,10,13,16,22,26,29-heptamethyl-18-[(1S)-1-methylpropyl]-25-(piperidine-1-carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone
Compound CP02: (3S,9S,12S,17S,20S,23S,27S,30S,36S)-3-[2-[3-chloro-4-(trifluoromethyl)phenyl]ethyl]-9-(cyclohexylmethyl)-30-cyclopentyl-23-isobutyl-7,10,17,18,24,28,31-heptamethyl-20-[(1S)-1-methylpropyl]-27-(piperidine-1-carbonyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP03: (3S,9S,12S,17S,20S,23S,27S,30S,36S)-3-[2-[3-chloro-4-(trifluoromethyl)phenyl]ethyl]-9-(cyclohexylmethyl)-30-cyclopentyl-23-isobutyl-7,10,17,18,24,28,31-heptamethyl-20-[(1S)-1-methylpropyl]-27-(morpholine-4-carbonyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP04: (3S,9S,12S,17S,20S,23S,27S,30S,36S)-3-[2-[3-chloro-4-(trifluoromethyl)phenyl]ethyl]-30-cyclopentyl-10-ethyl-23-isobutyl-7,17,18,24,28,31-hexamethyl-20-[(1S)-1-methylpropyl]-27-(morpholine-4-carbonyl)-9-(p-tolylmethyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP05: (3S,9S,12S,17S,20S,23S,27S,30S,36S)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1S)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.012,15]nonatriacontane-33,1'-cyclopentane]-27-carboxamide
Compound CP06: (3S,9S,12S,17S,20S,23S,27S,30S,36S,38R)-9-(cyclohexylmethyl)-30-cyclopentyl-3-[2-[3,5-difluoro-4-(trifluoromethyl)phenyl]ethyl]-38-ethoxy-23-isobutyl-N,N,7,10,17,18,24,28,31-nonamethyl-20-[(1S)-1-methylpropyl]-2,5,8,11,16,19,22,25,29,32,35-undecaoxo-spiro[1,4,7,10,15,18,21,24,28,31,34-undecazatricyclo[34.3.0.012,15]nonatriacontane-33,1'-cyclopentane]-27-carboxamide
Compound CP07: (3S,9S,12S,17S,20S,23S,27R,30S,36S,38R)-3-[2-[3-chloro-4-(trifluoromethyl)phenyl]ethyl]-38-ethoxy-23-isobutyl-7,10,17,18,24,27,28,31-octamethyl-20,30-[(1S)-1-methylpropyl]-9-[[4-(trifluoromethyl)phenyl]methyl]spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP08: (3S,9S,12S,17S,20S,23S,27R,30S,36S,38R)-3-[2-[3-chloro-4-(trifluoromethyl)phenyl]ethyl]-30-cyclopentyl-38-ethoxy-23-isobutyl-7,10,17,18,24,27,28,31-octamethyl-20-[(1S)-1-methylpropyl]-9-[[4-(trifluoromethyl)phenyl]methyl]spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone

### [Example 2] Production of cyclic peptide crystals using solvent with molecular weight of 18 or more and 170 or less, or mixed solvent of two or more thereof

Cyclic peptide crystals were produced by a method including a step of bringing into contact with (i) a solvent with a molecular weight of 18 or more and 170 or less, or (ii) a mixed solvent containing two or more solvents with a molecular weight of 18 or more and 170 or less. Note that seed crystals were not used in the step of bringing into contact with the solvent in the following production. Also, in the following Examples, % (percent) represents % by volume (v/v%).

(Example 2-1-1) Compound CP01 (136.7 mg) was dissolved in dimethyl sulfoxide (0.684 mL), and this dissolved solution (0.015 mL) was freeze-dried at -20°C for 2 days. To the resulting freeze-dried product, a water/isopropanol mixed solution (water ratio 75%, 0.015 mL) was added, and the mixture was shaken at room temperature for 5 days to obtain crystals of compound CP01.

(Example 2-1-2) Compound CP01 (72.5 mg) was dissolved in dimethyl sulfoxide (0.363 mL), and this dissolved solution (0.015 mL) was freeze-dried at -20°C for 3 days. To the resulting freeze-dried product, a dichloromethane/n-heptane mixed solution (n-heptane ratio 80%, 0.015 mL) was added, and the mixture was shaken at room temperature for 8 days to obtain crystals of compound CP01.

(Example 2-2-1) Compound CP02 (149.2 mg) was dissolved in dimethyl sulfoxide (0.746 mL), and this dissolved solution (0.015 mL) was freeze-dried at -20°C for 3 days. To the resulting freeze-dried product, formamide (0.015 mL) was added, and the mixture was shaken at room temperature for 3 days to obtain crystals of compound CP02.

(Example 2-2-2) Compound CP02 (72.4 mg) was dissolved in dimethyl sulfoxide (0.362 mL), and this dissolved solution (0.015 mL) was freeze-dried at -20°C for 3 days. To the resulting freeze-dried product, a formamide/n-heptane mixed solution (n-heptane ratio 80%, 0.015 mL) was added, and the mixture was shaken at room temperature for 8 days to obtain crystals of compound CP02.

(Example 2-2-3) Compound CP02 (72.4 mg) was dissolved in dimethyl sulfoxide (0.362 mL), and this dissolved solution (0.015 mL) was freeze-dried at -20°C for 3 days. To the resulting freeze-dried product, a dimethyl sulfoxide/t-butyl methyl ether mixed solution (t-butyl methyl ether ratio 80%, 0.015 mL) was added, and the mixture was shaken at room temperature for 8 days to obtain crystals of compound CP02.

(Example 2-3-1) Compound CP03 (147.4 mg) was dissolved in dimethyl sulfoxide (0.737 mL), and this dissolved solution (0.015 mL) was freeze-dried at -20°C for 3 days. To the resulting freeze-dried product, formamide (0.015 mL) was added, and the mixture was shaken at room temperature for 3 days to obtain crystals of compound CP03.

(Example 2-4-1) Compound CP04 (120.2 mg) was dissolved in dimethyl sulfoxide (0.601 mL), and this dissolved solution (0.015 mL) was freeze-dried at -20°C for 2 days. To the resulting freeze-dried product, t-butyl methyl ether (0.015 mL) was added, and the mixture was shaken at room temperature for 3 days to obtain crystals of compound CP04.

(Example 2-4-2) Compound CP04 (120.2 mg) was dissolved in dimethyl sulfoxide (0.601 mL), and this dissolved solution (0.015 mL) was freeze-dried at -20°C for 2 days. To the resulting freeze-dried product, toluene (0.015 mL) was added, and the mixture was shaken at room temperature for 3 days to obtain solids of compound CP04. The resulting solids were confirmed to be needle crystals having polarization by polarization microscopy.

(Example 2-4-3) Compound CP04 (120.2 mg) was dissolved in dimethyl sulfoxide (0.601 mL), and this dissolved solution (0.015 mL) was freeze-dried at -20°C for 2 days. To the resulting freeze-dried product, n-butyl acetate (0.015 mL) was added, and after shaking the mixture at room temperature for 3 days, n-heptane (0.015 mL) was further added. By shaking at room temperature for 4 days, solids of compound CP04 were obtained. The resulting solids were confirmed to be needle crystals having polarization by polarization microscopy.

(Example 2-4-4) Compound CP04 (120.2 mg) was dissolved in dimethyl sulfoxide (0.601 mL), and this dissolved solution (0.015 mL) was freeze-dried at -20°C for 2 days. To the resulting freeze-dried product, cumene (0.015 mL) was added, and the mixture was shaken at room temperature for 3 days to obtain solids of compound CP04. The resulting solids were confirmed to be needle crystals having polarization by polarization microscopy.

(Example 2-4-5) Compound CP04 (50.3 mg) was dissolved in dimethyl sulfoxide (0.252 mL), and this dissolved solution (0.015 mL) was freeze-dried at -20°C for 2 days. To the resulting freeze-dried product, an ethyl acetate/cyclohexane mixed solution (cyclohexane ratio 80%, 0.015 mL) was added, and the mixture was shaken at room temperature for 7 days to obtain solids of compound CP04. The resulting solids were confirmed to be needle crystals having polarization by polarization microscopy.

(Example 2-4-6) Compound CP04 (50.3 mg) was dissolved in dimethyl sulfoxide (0.252 mL), and this dissolved solution (0.015 mL) was freeze-dried at -20°C for 2 days. To the resulting freeze-dried product, a n-butyl acetate/cyclohexane mixed solution (cyclohexane ratio 80%, 0.015 mL) was added, and the mixture was shaken at room temperature for 7 days to obtain solids of compound CP04. The resulting solids were confirmed to be microcrystals having polarization by polarization microscopy.

(Example 2-5-1) Compound CP05 (122.3 mg) was dissolved in dimethyl sulfoxide (0.612 mL), and this dissolved solution (0.015 mL) was freeze-dried at -20°C for 2 days. To the resulting freeze-dried product, toluene (0.015 mL) was added, and the mixture was shaken at room temperature for 3 days to obtain crystals of compound CP05.

(Example 2-5-2) Compound CP05 (122.3 mg) was dissolved in dimethyl sulfoxide (0.612 mL), and this dissolved solution (0.015 mL) was freeze-dried at -20°C for 2 days. To the resulting freeze-dried product, dimethyl sulfoxide (0.015 mL) was added, and the mixture was shaken at room temperature for 3 days to obtain crystals of compound CP05.

(Example 2-5-3) Compound CP05 (122.3 mg) was dissolved in dimethyl sulfoxide (0.612 mL), and this dissolved solution (0.015 mL) was freeze-dried at -20°C for 2 days. To the resulting freeze-dried product, an ethanol/water mixed solution (water ratio 75%, 0.015 mL) was added, and the mixture was shaken at room temperature for 3 days to obtain crystals of compound CP05.

(Example 2-5-4) Compound CP05 (122.3 mg) was dissolved in dimethyl sulfoxide (0.612 mL), and this dissolved solution (0.015 mL) was freeze-dried at -20°C for 2 days. To the resulting freeze-dried product, an acetonitrile/water mixed solution (water ratio 75%, 0.015 mL) was added, and the mixture was shaken at room temperature for 3 days to obtain crystals of compound CP05.

(Example 2-5-5) Compound CP05 (122.3 mg) was dissolved in dimethyl sulfoxide (0.612 mL), and this dissolved solution (0.015 mL) was freeze-dried at -20°C for 2 days. To the resulting freeze-dried product, cumene (0.015 mL) was added, and the mixture was shaken at room temperature for 3 days to obtain crystals of compound CP05.

(Example 2-5-6) Compound CP05 (122.3 mg) was dissolved in dimethyl sulfoxide (0.612 mL), and this dissolved solution (0.015 mL) was freeze-dried at -20°C for 2 days. To the resulting freeze-dried product, tetralin (0.015 mL) was added, and the mixture was shaken at room temperature for 3 days to obtain crystals of compound CP05.

(Example 2-5-7) Compound CP05 (122.3 mg) was dissolved in dimethyl sulfoxide (0.612 mL), and this dissolved solution (0.015 mL) was freeze-dried at -20°C for 2 days. To the resulting freeze-dried product, formamide (0.015 mL) was added, and the mixture was shaken at room temperature for 3 days to obtain crystals of compound CP05.

(Example 2-5-8) Compound CP05 (122.3 mg) was dissolved in dimethyl sulfoxide (0.612 mL), and this dissolved solution (0.015 mL) was freeze-dried at -20°C for 2 days. To the resulting freeze-dried product, diisopropyl ether (0.015 mL) was added, and the mixture was shaken at room temperature for 7 days to obtain crystals of compound CP05.

(Example 2-5-9) Compound CP05 (148.8 mg) was dissolved in dimethyl sulfoxide (0.744 mL), and this dissolved solution (0.015 mL) was freeze-dried at -20°C for 3 days. To the resulting freeze-dried product, a 3-acetylpyridine/ethylene glycol mixed solution (3-acetylpyridine ratio 50% by volume, 0.015 mL) was added, and the mixture was shaken at room temperature for 11 days. Furthermore, about 10 zirconia beads with a diameter of 1 mm were added, and the mixture was shaken for 2 days. By leaving to stand for 5 days and further shaking for 3 days, crystals of compound CP05 were obtained.

(Example 2-6-1) Compound CP06 (122.1 mg) was dissolved in dimethyl sulfoxide (0.611 mL), and this dissolved solution (0.015 mL) was freeze-dried at -20°C for 2 days. To the resulting freeze-dried product, formamide (0.015 mL) was added, and the mixture was shaken at room temperature for 3 days to obtain crystals of compound CP06.

(Example 2-7-1) Compound CP07 (75.3 mg) was dissolved in 1,4-dioxane (0.377 mL), and this dissolved solution (0.015 mL) was freeze-dried at -20°C for 2 days. To the resulting freeze-dried product, acetonitrile (0.015 mL) was added, and the mixture was shaken at room temperature for 6 days to obtain crystals of compound CP07.

(Example 2-7-2) Compound CP07 (75.3 mg) was dissolved in 1,4-dioxane (0.377 mL), and this dissolved solution (0.015 mL) was freeze-dried at -20°C for 2 days. To the resulting freeze-dried product, ethyl acetate (0.015 mL) was added, and the mixture was shaken at room temperature for 6 days to obtain solids of compound CP07. The resulting solids were confirmed to be needle crystals having polarization by polarization microscopy.

(Example 2-7-3) Compound CP07 (75.3 mg) was dissolved in 1,4-dioxane (0.377 mL), and this dissolved solution (0.015 mL) was freeze-dried at -20°C for 2 days. To the resulting freeze-dried product, acetone (0.015 mL) was added, and the mixture was shaken at room temperature for 6 days to obtain crystals of compound CP07.

(Example 2-7-4) Compound CP07 (75.3 mg) was dissolved in 1,4-dioxane (0.377 mL), and this dissolved solution (0.015 mL) was freeze-dried at -20°C for 2 days. To the resulting freeze-dried product, propyl acetate (0.015 mL) was added, and the mixture was shaken at room temperature for 6 days to obtain crystals of compound CP07.

(Example 2-7-5) Compound CP07 (75.3 mg) was dissolved in 1,4-dioxane (0.377 mL), and this dissolved solution (0.015 mL) was freeze-dried at -20°C for 2 days. To the resulting freeze-dried product, n-butyl acetate (0.015 mL) was added, and the mixture was shaken at room temperature for 6 days to obtain solids of compound CP07. The resultant solids were confirmed to be needle crystals having polarization by polarization microscopy.

(Example 2-7-6) Compound CP07 (75.3 mg) was dissolved in 1,4-dioxane (0.377 mL), and this dissolved solution (0.015 mL) was freeze-dried at -20°C for 2 days. To the resulting freeze-dried product, methyl ethyl ketone (0.015 mL) was added, and the mixture was shaken at room temperature for 6 days to obtain solids of compound CP07. The resulting solids were confirmed to be needle crystals having polarization by polarization microscopy.

(Example 2-7-7) Compound CP07 (75.3 mg) was dissolved in 1,4-dioxane (0.377 mL), and this dissolved solution (0.015 mL) was freeze-dried at -20°C for 2 days. To the resulting freeze-dried product, methyl isobutyl ketone (0.015 mL) was added, and the mixture was shaken at room temperature for 6 days to obtain solids of compound CP07. The resulting solids were confirmed to be needle crystals having polarization by polarization microscopy.

(Example 2-7-8) Compound CP07 (75.3 mg) was dissolved in 1,4-dioxane (0.377 mL), and this dissolved solution (0.015 mL) was freeze-dried at -20°C for 2 days. To the resulting freeze-dried product, t-butyl methyl ether (0.015 mL) was added, and the mixture was shaken at room temperature for 6 days to obtain crystals of compound CP07.

(Example 2-7-9) Compound CP07 (75.3 mg) was dissolved in 1,4-dioxane (0.377 mL), and this dissolved solution (0.015 mL) was freeze-dried at -20°C for 2 days. To the resulting freeze-dried product, dimethyl sulfoxide (0.015 mL) was added, and the mixture was shaken at room temperature for 6 days to obtain crystals of compound CP07.

(Example 2-7-10) Compound CP07 (75.3 mg) was dissolved in 1,4-dioxane (0.377 mL), and this dissolved solution (0.015 mL) was freeze-dried at -20°C for 2 days. To the resulting freeze-dried product, a water/acetonitrile mixed solution (water ratio 75%, 0.015 mL) was added, and the mixture was shaken at room temperature for 6 days to obtain crystals of compound CP07.

(Example 2-7-11) Compound CP07 (75.3 mg) was dissolved in 1,4-dioxane (0.377 mL), and this dissolved solution (0.015 mL) was freeze-dried at -20°C for 2 days. To the resulting freeze-dried product, tetrahydrofuran (0.015 mL) was added, and after shaking the mixture at room temperature for 6 days, n-heptane (0.015 mL) was further added. By shaking at room temperature for 14 days, crystals of compound CP07 were obtained. The resulting solids were confirmed to be needle crystals having polarization by polarization microscopy.

(Example 2-7-12) Compound CP07 (75.3 mg) was dissolved in 1,4-dioxane (0.377 mL), and this dissolved solution (0.015 mL) was freeze-dried at -20°C for 2 days. To the resulting freeze-dried product, 1,4-dioxane (0.015 mL) was added, and after shaking the mixture at room temperature for 6 days, n-heptane (0.015 mL) was further added. By shaking at room temperature for 14 days, crystals of compound CP07 were obtained.

(Example 2-7-13) Compound CP07 (75.3 mg) was dissolved in 1,4-dioxane (0.377 mL), and this dissolved solution (0.015 mL) was freeze-dried at -20°C for 2 days. To the resulting freeze-dried product, toluene (0.015 mL) was added, and after shaking the mixture at room temperature for 6 days, n-heptane (0.015 mL) was further added. By shaking at room temperature for 14 days, solids of compound CP07 were obtained. The resulting solids were confirmed to be microcrystals having polarization by polarization microscopy.

(Example 2-7-14) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, an ethanol/n-heptane mixed solution (n-heptane ratio 80%, 0.015 mL) was added, and the mixture was shaken at room temperature for 8 days to obtain crystals of compound CP07.

(Example 2-7-15) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, a 2-propanol/n-heptane mixed solution (n-heptane ratio 80%, 0.015 mL) was added, and the mixture was shaken at room temperature for 8 days to obtain crystals of compound CP07.

(Example 2-7-16) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, an acetonitrile/t-butyl methyl ether mixed solution (t-butyl methyl ether ratio 80%, 0.015 mL) was added, and the mixture was shaken at room temperature for 8 days to obtain crystals of compound CP07.

(Example 2-7-17) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, a n-butanol/n-heptane mixed solution (n-heptane ratio 80%, 0.015 mL) was added, and the mixture was shaken at room temperature for 8 days to obtain crystals of compound CP07.

(Example 2-7-18) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, an anisole/n-heptane mixed solution (n-heptane ratio 80%, 0.015 mL) was added, and the mixture was shaken at room temperature for 8 days to obtain crystals of compound CP07.

(Example 2-7-19) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, a dimethyl sulfoxide/t-butyl methyl ether mixed solution (t-butyl methyl ether ratio 80%, 0.015 mL) was added, and the mixture was shaken at room temperature for 8 days to obtain crystals of compound CP07.

(Example 2-7-20) Compound CP07 (72.6 mg) was dissolved in a dimethyl sulfoxide/1,4-dioxane mixed solution (1,4-dioxane ratio 50%, 1.452 mL) at 70°C, and this dissolved solution (0.06 mL) was freeze-dried at -20°C for 3 days. To the resulting freeze-dried product, an acetonitrile/water mixed solution (water ratio 80%, 0.015 mL) was added, and the mixture was shaken at room temperature for 8 days to obtain crystals of compound CP07.

(Example 2-7-21) Compound CP07 (50.3 mg) was dissolved in 1,4-dioxane (0.252 mL), and this dissolved solution (0.015 mL) was freeze-dried at -20°C for 2 days. To the resulting freeze-dried product, an isopropanol/cyclohexane mixed solution (cyclohexane ratio 80%, 0.015 mL) was added, and the mixture was shaken at room temperature for 7 days to obtain crystals of compound CP07.

(Example 2-7-22) Compound CP07 (50.3 mg) was dissolved in 1,4-dioxane (0.252 mL), and this dissolved solution (0.015 mL) was freeze-dried at -20°C for 2 days. To the resulting freeze-dried product, a 1,4-dioxane/cyclohexane mixed solution (cyclohexane ratio 80%, 0.015 mL) was added, and the mixture was shaken at room temperature for 7 days to obtain solids of compound CP07. The resulting solids were confirmed to be needle crystals having polarization by polarization microscopy.

(Example 2-7-23) Compound CP07 (50.3 mg) was dissolved in 1,4-dioxane (0.252 mL), and this dissolved solution (0.015 mL) was freeze-dried at -20°C for 2 days. To the resulting freeze-dried product, an ethyl acetate/cyclohexane mixed solution (cyclohexane ratio 80%, 0.015 mL) was added, and the mixture was shaken at room temperature for 7 days to obtain crystals of compound CP07.

(Example 2-7-24) Compound CP07 (50.3 mg) was dissolved in 1,4-dioxane (0.252 mL), and this dissolved solution (0.015 mL) was freeze-dried at -20°C for 2 days. To the resulting freeze-dried product, an acetone/cyclohexane mixed solution (cyclohexane ratio 80%, 0.015 mL) was added, and the mixture was shaken at room temperature for 7 days to obtain crystals of compound CP07.

(Example 2-7-25) Compound CP07 (50.3 mg) was dissolved in 1,4-dioxane (0.252 mL), and this dissolved solution (0.015 mL) was freeze-dried at -20°C for 2 days. To the resulting freeze-dried product, a tetrahydrofuran/cyclohexane mixed solution (cyclohexane ratio 80%, 0.015 mL) was added, and the mixture was shaken at room temperature for 7 days to obtain crystals of compound CP07.

(Example 2-7-26) Compound CP07 (50.3 mg) was dissolved in 1,4-dioxane (0.252 mL), and this dissolved solution (0.015 mL) was freeze-dried at -20°C for 2 days. To the resulting freeze-dried product, a dichloromethane/cyclohexane mixed solution (cyclohexane ratio 80%, 0.015 mL) was added, and the mixture was shaken at room temperature for 7 days to obtain crystals of compound CP07.

(Example 2-7-27) Compound CP07 (50.3 mg) was dissolved in 1,4-dioxane (0.252 mL), and this dissolved solution (0.015 mL) was freeze-dried at -20°C for 2 days. To the resulting freeze-dried product, an anisole/cyclohexane mixed solution (cyclohexane ratio 80%, 0.015 mL) was added, and the mixture was shaken at room temperature for 7 days to obtain solids of compound CP07. The resulting solids were confirmed to be needle crystals having polarization by polarization microscopy.

(Example 2-7-28) Compound CP07 (50.3 mg) was dissolved in 1,4-dioxane (0.252 mL), and this dissolved solution (0.015 mL) was freeze-dried at -20°C for 2 days. To the resulting freeze-dried product, a n-butanol/cyclohexane mixed solution (cyclohexane ratio 80%, 0.015 mL) was added, and the mixture was shaken at room temperature for 7 days to obtain solids of compound CP07. The resulting solids were confirmed to be needle crystals having polarization by polarization microscopy.

(Example 2-7-29) Compound CP07 (50.3 mg) was dissolved in 1,4-dioxane (0.252 mL), and this dissolved solution (0.015 mL) was freeze-dried at -20°C for 2 days. To the resulting freeze-dried product, a toluene/cyclohexane mixed solution (cyclohexane ratio 80%, 0.015 mL) was added, and the mixture was shaken at room temperature for 7 days to obtain solids of compound CP07. The resulting solids were confirmed to be needle crystals having polarization by polarization microscopy.

(Example 2-7-30) Compound CP07 (50.3 mg) was dissolved in 1,4-dioxane (0.252 mL), and this dissolved solution (0.015 mL) was freeze-dried at -20°C for 2 days. To the resulting freeze-dried product, a propyl acetate/cyclohexane mixed solution (cyclohexane ratio 80%, 0.015 mL) was added, and the mixture was shaken at room temperature for 7 days to obtain solids of compound CP07. The resulting solids were confirmed to be microcrystals having polarization by polarization microscopy.

(Example 2-7-31) Compound CP07 (50.3 mg) was dissolved in 1,4-dioxane (0.252 mL), and this dissolved solution (0.015 mL) was freeze-dried at -20°C for 2 days. To the resulting freeze-dried product, a n-butyl acetate/cyclohexane mixed solution (cyclohexane ratio 80%, 0.015 mL) was added, and the mixture was shaken at room temperature for 7 days to obtain crystals of compound CP07.

(Example 2-7-32) Compound CP07 (50.3 mg) was dissolved in 1,4-dioxane (0.252 mL), and this dissolved solution (0.015 mL) was freeze-dried at -20°C for 2 days. To the resulting freeze-dried product, a methyl ethyl ketone/cyclohexane mixed solution (cyclohexane ratio 80%, 0.015 mL) was added, and the mixture was shaken at room temperature for 7 days to obtain crystals of compound CP07.

(Example 2-7-33) Compound CP07 (50.3 mg) was dissolved in 1,4-dioxane (0.252 mL), and this dissolved solution (0.015 mL) was freeze-dried at -20°C for 2 days. To the resulting freeze-dried product, a methyl isobutyl ketone/cyclohexane mixed solution (cyclohexane ratio 80%, 0.015 mL) was added, and the mixture was shaken at room temperature for 7 days to obtain solids of compound CP07. The resulting solids were confirmed to be needle crystals having polarization by polarization microscopy.

(Example 2-7-34) Compound CP07 (50.3 mg) was dissolved in 1,4-dioxane (0.252 mL), and this dissolved solution (0.015 mL) was freeze-dried at -20°C for 2 days. To the resulting freeze-dried product, a chlorobenzene/cyclohexane mixed solution (cyclohexane ratio 80%, 0.015 mL) was added, and the mixture was shaken at room temperature for 7 days to obtain crystals of compound CP07.

(Example 2-8-1) Compound CP08 (75.3 mg) was dissolved in 1,4-dioxane (0.376 mL), and this dissolved solution (0.015 mL) was freeze-dried at -20°C for 2 days. To the resulting freeze-dried product, acetonitrile (0.015 mL) was added, and the mixture was shaken at room temperature for 6 days to obtain crystals of compound CP08.

(Example 2-8-2) Compound CP08 (75.3 mg) was dissolved in 1,4-dioxane (0.376 mL), and this dissolved solution (0.015 mL) was freeze-dried at -20°C for 2 days. To the resulting freeze-dried product, ethyl acetate (0.015 mL) was added, and the mixture was shaken at room temperature for 6 days to obtain solids of compound CP08. The resulting solids were confirmed to be needle crystals having polarization by polarization microscopy.

(Example 2-8-3) Compound CP08 (75.3 mg) was dissolved in 1,4-dioxane (0.376 mL), and this dissolved solution (0.015 mL) was freeze-dried at -20°C for 2 days. To the resulting freeze-dried product, acetone (0.015 mL) was added, and the mixture was shaken at room temperature for 6 days to obtain crystals of compound CP08.

(Example 2-8-4) Compound CP08 (75.3 mg) was dissolved in 1,4-dioxane (0.376 mL), and this dissolved solution (0.015 mL) was freeze-dried at -20°C for 2 days. To the resulting freeze-dried product, propyl acetate (0.015 mL) was added, and the mixture was shaken at room temperature for 6 days to obtain solids of compound CP08. The resulting solids were confirmed to be needle crystals having polarization by polarization microscopy.

(Example 2-8-5) Compound CP08 (75.3 mg) was dissolved in 1,4-dioxane (0.376 mL), and this dissolved solution (0.015 mL) was freeze-dried at -20°C for 2 days. To the resulting freeze-dried product, n-butyl acetate (0.015 mL) was added, and the mixture was shaken at room temperature for 6 days to obtain solids of compound CP08. The resulting solids were confirmed to be microcrystals having polarization by polarization microscopy.

(Example 2-8-6) Compound CP08 (75.3 mg) was dissolved in 1,4-dioxane (0.376 mL), and this dissolved solution (0.015 mL) was freeze-dried at -20°C for 2 days. To the resulting freeze-dried product, methyl ethyl ketone (0.015 mL) was added, and the mixture was shaken at room temperature for 6 days to obtain crystals of compound CP08.

(Example 2-8-7) Compound CP08 (75.3 mg) was dissolved in 1,4-dioxane (0.376 mL), and this dissolved solution (0.015 mL) was freeze-dried at -20°C for 2 days. To the resulting freeze-dried product, methyl isobutyl ketone (0.015 mL) was added, and the mixture was shaken at room temperature for 6 days to obtain solids of compound CP08. The resulting solids were confirmed to be needle crystals having polarization by polarization microscopy.

(Example 2-8-8) Compound CP08 (75.3 mg) was dissolved in 1,4-dioxane (0.376 mL), and this dissolved solution (0.015 mL) was freeze-dried at -20°C for 2 days. To the resulting freeze-dried product, t-butyl methyl ether (0.015 mL) was added, and the mixture was shaken at room temperature for 6 days to obtain solids of compound CP08. The resulting solids were confirmed to be needle crystals having polarization by polarization microscopy.

(Example 2-8-9) Compound CP08 (75.3 mg) was dissolved in 1,4-dioxane (0.376 mL), and this dissolved solution (0.015 mL) was freeze-dried at -20°C for 2 days. To the resulting freeze-dried product, toluene (0.015 mL) was added, and the mixture was shaken at room temperature for 6 days to obtain solids of compound CP08. The resulting solids were confirmed to be plate crystals having polarization by polarization microscopy.

(Example 2-8-10) Compound CP08 (75.3 mg) was dissolved in 1,4-dioxane (0.376 mL), and this dissolved solution (0.015 mL) was freeze-dried at -20°C for 2 days. To the resulting freeze-dried product, dimethyl sulfoxide (0.015 mL) was added, and the mixture was shaken at room temperature for 6 days to obtain crystals of compound CP08.

(Example 2-8-11) Compound CP08 (75.3 mg) was dissolved in 1,4-dioxane (0.376 mL), and this dissolved solution (0.015 mL) was freeze-dried at -20°C for 2 days. To the resulting freeze-dried product, 1,4-dioxane (0.015 mL) was added, and after shaking the mixture at room temperature for 6 days, n-heptane (0.015 mL) was further added. By shaking at room temperature for 14 days, solids of compound CP08 were obtained. The resulting solids were confirmed to be needle crystals having polarization by polarization microscopy.

(Example 2-8-12) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an ethanol/n-heptane mixed solution (n-heptane ratio 80%, 0.015 mL) was added, and the mixture was shaken at room temperature for 8 days to obtain solids of compound CP08. The resulting solids were confirmed to be plate crystals having polarization by polarization microscopy.

(Example 2-8-13) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an isopropanol/n-heptane mixed solution (n-heptane ratio 80%, 0.015 mL) was added, and the mixture was shaken at room temperature for 8 days to obtain crystals of compound CP08.

(Example 2-8-14) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an ethyl acetate/n-heptane mixed solution (n-heptane ratio 80%, 0.015 mL) was added, and the mixture was shaken at room temperature for 8 days to obtain crystals of compound CP08.

(Example 2-8-15) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an acetone/n-heptane mixed solution (n-heptane ratio 80%, 0.015 mL) was added, and the mixture was shaken at room temperature for 8 days to obtain crystals of compound CP08.

(Example 2-8-16) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a tetrahydrofuran/n-heptane mixed solution (n-heptane ratio 80%, 0.015 mL) was added, and the mixture was shaken at room temperature for 8 days to obtain crystals of compound CP08.

(Example 2-8-17) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a dichloromethane/n-heptane mixed solution (n-heptane ratio 80%, 0.015 mL) was added, and the mixture was shaken at room temperature for 8 days to obtain crystals of compound CP08.

(Example 2-8-18) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a formamide/water mixed solution (water ratio 80%, 0.015 mL) was added, and the mixture was shaken at room temperature for 8 days to obtain solids of compound CP08. The resulting solids were confirmed to be microcrystals having polarization by polarization microscopy.

(Example 2-8-19) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a n-butanol/n-heptane mixed solution (n-heptane ratio 80%, 0.015 mL) was added, and the mixture was shaken at room temperature for 8 days to obtain crystals of compound CP08.

(Example 2-8-20) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a propyl acetate/n-heptane mixed solution (n-heptane ratio 80%, 0.015 mL) was added, and the mixture was shaken at room temperature for 8 days to obtain crystals of compound CP08.

(Example 2-8-21) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a n-butyl acetate/n-heptane mixed solution (n-heptane ratio 80%, 0.015 mL) was added, and the mixture was shaken at room temperature for 8 days to obtain crystals of compound CP08.

(Example 2-8-22) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a methyl ethyl ketone/n-heptane mixed solution (n-heptane ratio 80%, 0.015 mL) was added, and the mixture was shaken at room temperature for 8 days to obtain crystals of compound CP08.

(Example 2-8-23) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a methyl isobutyl ketone/n-heptane mixed solution (n-heptane ratio 80%, 0.015 mL) was added, and the mixture was shaken at room temperature for 8 days to obtain crystals of compound CP08.

(Example 2-8-24) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a formamide/n-heptane mixed solution (n-heptane ratio 80%, 0.015 mL) was added, and the mixture was shaken at room temperature for 8 days to obtain crystals of compound CP08.

(Example 2-8-25) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an anisole/n-heptane mixed solution (n-heptane ratio 80%, 0.015 mL) was added, and the mixture was shaken at room temperature for 8 days to obtain crystals of compound CP08.

(Example 2-8-26) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a 1,4-dioxane/n-heptane mixed solution (n-heptane ratio 80%, 0.015 mL) was added, and the mixture was shaken at room temperature for 8 days to obtain crystals of compound CP08.

(Example 2-8-27) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a dimethyl sulfoxide/t-butyl methyl ether mixed solution (t-butyl methyl ether ratio 80%, 0.015 mL) was added, and the mixture was shaken at room temperature for 8 days to obtain solids of compound CP08. The resulting solids were confirmed to be microcrystals having polarization by polarization microscopy.

(Example 2-8-28) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an ethanol/water mixed solution (water ratio 80%, 0.015 mL) was added, and the mixture was shaken at room temperature for 8 days to obtain crystals of compound CP08.

(Example 2-8-29) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an acetonitrile/water mixed solution (water ratio 80%, 0.015 mL) was added, and the mixture was shaken at room temperature for 8 days to obtain solids of compound CP08. The resulting solids were confirmed to be microcrystals having polarization by polarization microscopy.

(Example 2-8-30) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, a chlorobenzene/n-heptane mixed solution (n-heptane ratio 80%, 0.015 mL) was added, and the mixture was shaken at room temperature for 8 days to obtain crystals of compound CP08.

(Example 2-8-31) Compound CP08 (72.5 mg) was dissolved in a dimethyl sulfoxide/1,4-dioxane mixed solution (1,4-dioxane ratio 50%, 0.726 mL), and this dissolved solution (0.03 mL) was freeze-dried at -20°C for 3 days. To the resulting freeze-dried product, an acetone/water mixed solution (water ratio 80%, 0.015 mL) was added, and the mixture was shaken at room temperature for 8 days to obtain crystals of compound CP08.

(Example 2-8-32) Compound CP08 (50.6 mg) was dissolved in 1,4-dioxane (0.253 mL), and this dissolved solution (0.015 mL) was freeze-dried at -20°C for 2 days. To the resulting freeze-dried product, an isopropanol/cyclohexane mixed solution (cyclohexane ratio 80%, 0.015 mL) was added, and the mixture was shaken at room temperature for 7 days to obtain crystals of compound CP08.

(Example 2-8-33) Compound CP08 (50.6 mg) was dissolved in 1,4-dioxane (0.253 mL), and this dissolved solution (0.015 mL) was freeze-dried at -20°C for 2 days. To the resulting freeze-dried product, a 1,4-dioxane/cyclohexane mixed solution (cyclohexane ratio 80%, 0.015 mL) was added, and the mixture was shaken at room temperature for 7 days to obtain crystals of compound CP08.

(Example 2-8-34) Compound CP08 (50.6 mg) was dissolved in 1,4-dioxane (0.253 mL), and this dissolved solution (0.015 mL) was freeze-dried at -20°C for 2 days. To the resulting freeze-dried product, an ethyl acetate/cyclohexane mixed solution (cyclohexane ratio 80%, 0.015 mL) was added, and the mixture was shaken at room temperature for 7 days to obtain crystals of compound CP08.

(Example 2-8-35) Compound CP08 (50.6 mg) was dissolved in 1,4-dioxane (0.253 mL), and this dissolved solution (0.015 mL) was freeze-dried at -20°C for 2 days. To the resulting freeze-dried product, an acetone/cyclohexane mixed solution (cyclohexane ratio 80%, 0.015 mL) was added, and the mixture was shaken at room temperature for 7 days to obtain crystals of compound CP08.

(Example 2-8-36) Compound CP08 (50.6 mg) was dissolved in 1,4-dioxane (0.253 mL), and this dissolved solution (0.015 mL) was freeze-dried at -20°C for 2 days. To the resulting freeze-dried product, a tetrahydrofuran/cyclohexane mixed solution (cyclohexane ratio 80%, 0.015 mL) was added, and the mixture was shaken at room temperature for 7 days to obtain crystals of compound CP08.

(Example 2-8-37) Compound CP08 (50.6 mg) was dissolved in 1,4-dioxane (0.253 mL), and this dissolved solution (0.015 mL) was freeze-dried at -20°C for 2 days. To the resulting freeze-dried product, a dichloromethane/cyclohexane mixed solution (cyclohexane ratio 80%, 0.015 mL) was added, and the mixture was shaken at room temperature for 7 days to obtain crystals of compound CP08.

(Example 2-8-38) Compound CP08 (50.6 mg) was dissolved in 1,4-dioxane (0.253 mL), and this dissolved solution (0.015 mL) was freeze-dried at -20°C for 2 days. To the resulting freeze-dried product, an anisole/cyclohexane mixed solution (cyclohexane ratio 80%, 0.015 mL) was added, and the mixture was shaken at room temperature for 7 days to obtain crystals of compound CP08.

(Example 2-8-39) Compound CP08 (50.6 mg) was dissolved in 1,4-dioxane (0.253 mL), and this dissolved solution (0.015 mL) was freeze-dried at -20°C for 2 days. To the resulting freeze-dried product, a butanol/cyclohexane mixed solution (cyclohexane ratio 80%, 0.015 mL) was added, and the mixture was shaken at room temperature for 7 days to obtain crystals of compound CP08.

(Example 2-8-40) Compound CP08 (50.6 mg) was dissolved in 1,4-dioxane (0.253 mL), and this dissolved solution (0.015 mL) was freeze-dried at -20°C for 2 days. To the resulting freeze-dried product, a toluene/cyclohexane mixed solution (cyclohexane ratio 80%, 0.015 mL) was added, and the mixture was shaken at room temperature for 7 days to obtain crystals of compound CP08.

(Example 2-8-41) Compound CP08 (50.6 mg) was dissolved in 1,4-dioxane (0.253 mL), and this dissolved solution (0.015 mL) was freeze-dried at -20°C for 2 days. To the resulting freeze-dried product, a propyl acetate/cyclohexane mixed solution (cyclohexane ratio 80%, 0.015 mL) was added, and the mixture was shaken at room temperature for 7 days to obtain crystals of compound CP08.

(Example 2-8-42) Compound CP08 (50.6 mg) was dissolved in 1,4-dioxane (0.253 mL), and this dissolved solution (0.015 mL) was freeze-dried at -20°C for 2 days. To the resulting freeze-dried product, a n-butyl acetate/cyclohexane mixed solution (cyclohexane ratio 80%, 0.015 mL) was added, and the mixture was shaken at room temperature for 7 days to obtain crystals of compound CP08.

(Example 2-8-43) Compound CP08 (50.6 mg) was dissolved in 1,4-dioxane (0.253 mL), and this dissolved solution (0.015 mL) was freeze-dried at -20°C for 2 days. To the resulting freeze-dried product, a methyl ethyl ketone/cyclohexane mixed solution (cyclohexane ratio 80%, 0.015 mL) was added, and the mixture was shaken at room temperature for 7 days to obtain crystals of compound CP08.

(Example 2-8-44) Compound CP08 (50.6 mg) was dissolved in 1,4-dioxane (0.253 mL), and this dissolved solution (0.015 mL) was freeze-dried at -20°C for 2 days. To the resulting freeze-dried product, a methyl isobutyl ketone/cyclohexane mixed solution (cyclohexane ratio 80%, 0.015 mL) was added, and the mixture was shaken at room temperature for 7 days to obtain crystals of compound CP08.

(Example 2-8-45) Compound CP08 (50.6 mg) was dissolved in 1,4-dioxane (0.253 mL), and this dissolved solution (0.015 mL) was freeze-dried at -20°C for 2 days. To the resulting freeze-dried product, a chlorobenzene/cyclohexane mixed solution (cyclohexane ratio 80%, 0.015 mL) was added, and the mixture was shaken at room temperature for 7 days to obtain crystals of compound CP08.

From the above results, crystals of all eight cyclic peptides were obtained under either of the conditions using a solvent with a molecular weight of 18 or more and 170 or less, or a mixed solvent of two or more thereof. Specifically, under the conditions using a solvent containing formamide as the solvent, crystals of five out of the eight cyclic peptides were obtained (Examples). Under the conditions using a solvent containing dimethyl sulfoxide as the solvent, crystals of four out of the eight cyclic peptides were obtained (Examples). Under the conditions using a solvent containing toluene, cumene, or tetralin, which is an aromatic hydrocarbon-based solvent, as the solvent, crystals of four out of the eight cyclic peptides were obtained (Examples). Under the conditions using a solvent containing dichloromethane or chlorobenzene, which is a halogen-based solvent, as the solvent, crystals of three out of the eight cyclic peptides were obtained (Examples). Under the conditions using a solvent containing an alcohol-based solvent as the solvent, crystals of four out of the eight cyclic peptides were obtained (Examples). Under the conditions using a solvent containing an ether-based solvent as the solvent, crystals of four out of the eight cyclic peptides were obtained (Examples). Under the conditions using a solvent containing an ester-based solvent as the solvent, crystals of three out of the eight cyclic peptides were obtained (Examples). Under the conditions using a solvent containing a nitrile-based solvent as the solvent, crystals of three out of the eight cyclic peptides were obtained (Examples). Under the conditions using a solvent containing a ketone-based solvent as the solvent, crystals of two out of the eight cyclic peptides were obtained (Examples).

The solvent for crystallizing a variety of cyclic peptides is preferably one or more selected from the group consisting of formamide, dimethyl sulfoxide, an aromatic hydrocarbon-based solvent, and a halogen-based solvent, and by using at least one of these solvents, all of the eight cyclic peptides were successfully crystallized. The results showed a solvent containing formamide or dimethyl sulfoxide to be more preferred, and a solvent containing formamide to be most preferred.

### [Example 3] Production of cyclic peptide crystals using aqueous solution containing surfactant and water-soluble organic solvent

Cyclic peptide crystals were produced by a method including a step of bringing into contact with water containing 0.01 to 30 wt/v% of a surfactant, as for a liquid surfactant, and 5 to 50 v/v% of a water-soluble organic solvent, based on the total amount of the solvent. Note that seed crystals were not used in the step of bringing into contact with the solvent in the following production.

(Example 3-1-1) Compound CP01 (19.7 mg) was dissolved in ethanol (0.197 mL). To this dissolved solution (0.010 mL), a Cremophor EL/water mixed solution (Cremophor EL ratio 5 v/v% (5.25 wt/v%), 0.090 mL) was added, and the mixture was stirred for 10 hours, moving the temperature up and down 36 times in the range of 50°C to 90°C, thereby obtaining crystals of compound CP01.

(Example 3-4-1) Compound CP04 (19.5 mg) was dissolved in ethanol (0.195 mL). To this dissolved solution (0.010 mL), a Cremophor EL/water mixed solution (Cremophor EL ratio 5 v/v% (5.25 wt/v%), 0.090 mL) was added, and the mixture was stirred for 10 hours, moving the temperature up and down 36 times in the range of 50°C to 90°C, thereby obtaining crystals of compound CP04.

(Example 3-5-1) Compound CP05 (19.1 mg) was dissolved in ethanol (0.191 mL). To this dissolved solution (0.010 mL), a Cremophor EL/water mixed solution (Cremophor EL ratio 1 v/v% (1.05 wt/v%), 0.090 mL) was added, and the mixture was stirred for 10 hours, moving the temperature up and down 36 times in the range of 50°C to 90°C, thereby obtaining crystals of compound CP05.

(Example 3-5-2) Compound CP05 (19.1 mg) was dissolved in ethanol (0.191 mL). To this dissolved solution (0.010 mL), a Cremophor EL/water mixed solution (Cremophor EL ratio 5 v/v% (5.25 wt/v%), 0.090 mL) was added, and the mixture was stirred for 10 hours, moving the temperature up and down 36 times in the range of 50°C to 90°C, thereby obtaining crystals of compound CP05.

(Example 3-5-3) Compound CP05 (19.2 mg) was dissolved in dimethyl sulfoxide (0.192 mL). To this dissolved solution (0.010 mL), a Cremophor EL/water mixed solution (Cremophor EL ratio 1 v/v% (1.05 wt/v%), 0.090 mL) was added, and the mixture was stirred for 10 hours, moving the temperature up and down 36 times in the range of 50°C to 90°C, thereby obtaining crystals of compound CP05.

(Example 3-5-4) Compound CP05 (19.2 mg) was dissolved in dimethyl sulfoxide (0.192 mL). To this dissolved solution (0.010 mL), a Cremophor EL/water mixed solution (Cremophor EL ratio 5 v/v% (5.25 wt/v%), 0.090 mL) was added, and the mixture was stirred for 10 hours, moving the temperature up and down 36 times in the range of 50°C to 90°C, thereby obtaining crystals of compound CP05.

(Example 3-5-5) Compound CP05 (19.1 mg) was dissolved in ethanol (0.191 mL). To this dissolved solution (0.010 mL), a Tween 80/water mixed solution (Tween 80 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP05.

(Example 3-5-6) Compound CP05 (19.1 mg) was dissolved in ethanol (0.191 mL). To this dissolved solution (0.010 mL), a Tween 80/water mixed solution (Tween 80 ratio 3 v/v% (3.24 wt/v%), 0.090 mL) was added, and the mixture was stirred for 10 hours, moving the temperature up and down 36 times in the range of 50°C to 90°C, thereby obtaining crystals of compound CP05.

(Example 3-5-7) Compound CP05 (19.2 mg) was dissolved in dimethyl sulfoxide (0.192 mL). To this dissolved solution (0.010 mL), a Tween 80/water mixed solution (Tween 80 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP05.

(Example 3-5-8) Compound CP05 (19.2 mg) was dissolved in dimethyl sulfoxide (0.192 mL). To this dissolved solution (0.010 mL), a Tween 80/water mixed solution (Tween 80 ratio 3 v/v% (3.24 wt/v%), 0.090 mL) was added, and the mixture was stirred for 10 hours, moving the temperature up and down 36 times in the range of 50°C to 90°C, thereby obtaining crystals of compound CP05.

(Example 3-5-9) Compound CP05 (19.1 mg) was dissolved in ethanol (0.191 mL). To this dissolved solution (0.010 mL), a Triton X-100/water mixed solution (Triton X-100 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP05.

(Example 3-5-10) Compound CP05 (19.1 mg) was dissolved in ethanol (0.191 mL). To this dissolved solution (0.010 mL), a Triton X-100/water mixed solution (Triton X-100 ratio 3 v/v% (3.21 wt/v%), 0.090 mL) was added, and the mixture was stirred for 10 hours, moving the temperature up and down 36 times in the range of 50°C to 90°C, thereby obtaining crystals of compound CP05.

(Example 3-5-11) Compound CP05 (19.2 mg) was dissolved in dimethyl sulfoxide (0.192 mL). To this dissolved solution (0.010 mL), a Triton X-100/water mixed solution (Triton X-100 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP05.

(Example 3-5-12) Compound CP05 (19.2 mg) was dissolved in dimethyl sulfoxide (0.192 mL). To this dissolved solution (0.010 mL), a Triton X-100/water mixed solution (Triton X-100 ratio 3 v/v% (3.21 wt/v%), 0.090 mL) was added, and the mixture was stirred for 10 hours, moving the temperature up and down 36 times in the range of 50°C to 90°C, thereby obtaining crystals of compound CP05.

(Example 3-5-13) Compound CP05 (19.1 mg) was dissolved in ethanol (0.191 mL). To this dissolved solution (0.010 mL), a sodium lauryl sulfate/water mixed solution (sodium lauryl sulfate ratio 1 wt/v%, 0.090 mL) was added, and the mixture was stirred for 10 hours, moving the temperature up and down 36 times in the range of 50°C to 90°C, thereby obtaining crystals of compound CP05.

(Example 3-5-14) Compound CP05 (19.2 mg) was dissolved in dimethyl sulfoxide (0.192 mL). To this dissolved solution (0.010 mL), a sodium lauryl sulfate/water mixed solution (sodium lauryl sulfate ratio 0.2 wt/v%, 0.090 mL) was added, and the mixture was stirred for 10 hours, moving the temperature up and down 36 times in the range of 50°C to 90°C, thereby obtaining crystals of compound CP05.

(Example 3-5-15) Compound CP05 (19.2 mg) was dissolved in dimethyl sulfoxide (0.192 mL). To this dissolved solution (0.010 mL), a sodium lauryl sulfate/water mixed solution (sodium lauryl sulfate ratio 1 wt/v%, 0.090 mL) was added, and the mixture was stirred for 10 hours, moving the temperature up and down 36 times in the range of 50°C to 90°C, thereby obtaining crystals of compound CP05.

(Example 3-5-16) Compound CP05 (19.1 mg) was dissolved in ethanol (0.191 mL). To this dissolved solution (0.010 mL), a Cremophor EL/water mixed solution (Cremophor EL ratio 1 v/v% (1.05 wt/v%), 0.090 mL) was added, and the mixture was stirred at 25°C for 10 hours, thereby obtaining crystals of compound CP05.

(Example 3-5-17) Compound CP05 (19.1 mg) was dissolved in ethanol (0.191 mL). To this dissolved solution (0.010 mL), a Cremophor EL/water mixed solution (Cremophor EL ratio 5 v/v% (5.25 wt/v%), 0.090 mL) was added, and the mixture was stirred at 25°C for 10 hours, thereby obtaining crystals of compound CP05.

(Example 3-5-18) Compound CP05 (19.2 mg) was dissolved in dimethyl sulfoxide (0.192 mL). To this dissolved solution (0.010 mL), a Cremophor EL/water mixed solution (Cremophor EL ratio 1 v/v% (1.05 wt/v%), 0.090 mL) was added, and the mixture was stirred at 25°C for 10 hours, thereby obtaining crystals of compound CP05.

(Example 3-5-19) Compound CP05 (19.2 mg) was dissolved in dimethyl sulfoxide (0.192 mL). To this dissolved solution (0.010 mL), a Cremophor EL/water mixed solution (Cremophor EL ratio 5 v/v% (5.25 wt/v%), 0.090 mL) was added, and the mixture was stirred at 25°C for 10 hours, thereby obtaining crystals of compound CP05.

(Example 3-5-20) Compound CP05 (19.1 mg) was dissolved in ethanol (0.191 mL). To this dissolved solution (0.010 mL), a Tween 80/water mixed solution (Tween 80 ratio 0.5 v/v% (0.54 wt/v%), 0.090 mL) was added, and the mixture was stirred at 25°C for 10 hours, thereby obtaining crystals of compound CP05.

(Example 3-5-21) Compound CP05 (19.1 mg) was dissolved in ethanol (0.191 mL). To this dissolved solution (0.010 mL), a Tween 80/water mixed solution (Tween 80 ratio 3 v/v% (3.24 wt/v%), 0.090 mL) was added, and the mixture was stirred at 25°C for 10 hours, thereby obtaining crystals of compound CP05.

(Example 3-5-22) Compound CP05 (19.2 mg) was dissolved in dimethyl sulfoxide (0.192 mL). To this dissolved solution (0.010 mL), a Tween 80/water mixed solution (Tween 80 ratio 0.5 v/v% (0.54 wt/v%), 0.090 mL) was added, and the mixture was stirred at 25°C for 10 hours, thereby obtaining crystals of compound CP05.

(Example 3-5-23) Compound CP05 (19.2 mg) was dissolved in dimethyl sulfoxide (0.192 mL). To this dissolved solution (0.010 mL), a Tween 80/water mixed solution (Tween 80 ratio 3 v/v% (3.24 wt/v%), 0.090 mL) was added, and the mixture was stirred at 25°C for 10 hours, thereby obtaining crystals of compound CP05.

(Example 3-5-24) Compound CP05 (19.1 mg) was dissolved in ethanol (0.191 mL). To this dissolved solution (0.010 mL), a Triton X-100/water mixed solution (Triton X-100 ratio 0.5 v/v% (0.54 wt/v%), 0.090 mL) was added, and the mixture was stirred at 25°C for 10 hours, thereby obtaining crystals of compound CP05.

(Example 3-5-25) Compound CP05 (19.1 mg) was dissolved in ethanol (0.191 mL). To this dissolved solution (0.010 mL), a Triton X-100/water mixed solution (Triton X-100 ratio 3 v/v% (3.21 wt/v%), 0.090 mL) was added, and the mixture was stirred at 25°C for 10 hours, thereby obtaining crystals of compound CP05.

(Example 3-5-26) Compound CP05 (19.2 mg) was dissolved in dimethyl sulfoxide (0.192 mL). To this dissolved solution (0.010 mL), a Triton X-100/water mixed solution (Triton X-100 ratio 0.5 v/v% (0.54 wt/v%), 0.090 mL) was added, and the mixture was stirred at 25°C for 10 hours, thereby obtaining crystals of compound CP05.

(Example 3-5-27) Compound CP05 (19.2 mg) was dissolved in dimethyl sulfoxide (0.192 mL). To this dissolved solution (0.010 mL), a Triton X-100/water mixed solution (Triton X-100 ratio 3 v/v% (3.21 wt/v%), 0.090 mL) was added, and the mixture was stirred at 25°C for 10 hours, thereby obtaining crystals of compound CP05.

(Example 3-5-28) Compound CP05 (19.1 mg) was dissolved in ethanol (0.191 mL). To this dissolved solution (0.010 mL), a sodium lauryl sulfate/water mixed solution (sodium lauryl sulfate ratio 1 wt/v%, 0.090 mL) was added, and the mixture was stirred at 25°C for 10 hours, thereby obtaining crystals of compound CP05.

(Example 3-5-29) Compound CP05 (19.2 mg) was dissolved in dimethyl sulfoxide (0.192 mL). To this dissolved solution (0.010 mL), a sodium lauryl sulfate/water mixed solution (sodium lauryl sulfate ratio 0.2 wt/v%, 0.090 mL) was added, and the mixture was stirred at 25°C for 10 hours, thereby obtaining crystals of compound CP05.

(Example 3-5-30) Compound CP05 (19.2 mg) was dissolved in dimethyl sulfoxide (0.192 mL). To this dissolved solution (0.010 mL), a sodium lauryl sulfate/water mixed solution (sodium lauryl sulfate ratio 1 wt/v%, 0.090 mL) was added, and the mixture was stirred at 25°C for 10 hours, thereby obtaining crystals of compound CP05.

(Example 3-7-1) Compound CP07 (19.3 mg) was dissolved in dimethyl sulfoxide (0.193 mL). To this dissolved solution (0.010 mL), a Tween 80/water mixed solution (Tween 80 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP07.

(Example 3-8-1) Compound CP08 (19.5 mg) was dissolved in ethanol (0.195 mL). To this dissolved solution (0.010 mL), a Cremophor EL/water mixed solution (Cremophor EL ratio 5 v/v% (5.25 wt/v%), 0.090 mL) was added, and the mixture was stirred for 10 hours, moving the temperature up and down 36 times in the range of 50°C to 90°C, thereby obtaining crystals of compound CP08.

(Example 3-8-2) Compound CP08 (19.5 mg) was dissolved in ethanol (0.195 mL). To this dissolved solution (0.010 mL), a Triton X-100/water mixed solution (Triton X-100 ratio 0.5 v/v% (0.54 wt/v%), 0.090 mL) was added, and the mixture was stirred for 10 hours, moving the temperature up and down 36 times in the range of 50°C to 90°C, thereby obtaining crystals of compound CP08.

(Example 3-8-3) Compound CP08 (19.5 mg) was dissolved in ethanol (0.195 mL). To this dissolved solution (0.010 mL), a Cremophor EL/water mixed solution (Cremophor EL ratio 1 v/v% (1.05 wt/v%), 0.090 mL) was added, and the mixture was stirred at 25°C for 10 hours, thereby obtaining crystals of compound CP08.

(Example 3-8-4) Compound CP08 (19.5 mg) was dissolved in ethanol (0.195 mL). To this dissolved solution (0.010 mL), a Tween 80/water mixed solution (Tween 80 ratio 0.5 v/v% (0.54 wt/v%), 0.090 mL) was added, and the mixture was stirred at 25°C for 10 hours, thereby obtaining crystals of compound CP08.

(Example 3-8-5) Compound CP08 (19.5 mg) was dissolved in ethanol (0.195 mL). To this dissolved solution (0.010 mL), a Tween 80/water mixed solution (Tween 80 ratio 3 v/v% (3.24 wt/v%), 0.090 mL) was added, and the mixture was stirred at 25°C for 10 hours, thereby obtaining crystals of compound CP08.

(Example 3-8-6) Compound CP08 (19.5 mg) was dissolved in ethanol (0.195 mL). To this dissolved solution (0.010 mL), a Triton X-100/water mixed solution (Triton X-100 ratio 3 v/v% (3.21 wt/v%), 0.090 mL) was added, and the mixture was stirred at 25°C for 10 hours, thereby obtaining crystals of compound CP08.

From the above results, under the conditions using an aqueous solution containing a surfactant and a water-soluble organic solvent, crystals of five out of the eight cyclic peptides were obtained. In the case where Cremophor EL was used as the surfactant, crystals of four out of the eight cyclic peptides were obtained (Examples). In the case where Triton X-100 was used as the surfactant, crystals of three out of the eight cyclic peptides were obtained (Examples).

As the conditions for crystallizing a variety of cyclic peptides, the conditions using Cremophor EL, Tween 80, or Triton X-100 as the surfactant were preferred, and as for the temperature, moving it up and down was preferred. As for the water-soluble organic solvent, the conditions using ethanol were preferred conditions. The conditions using Cremophor EL as the surfactant were more preferred. Furthermore, the most preferred results were the conditions under which Cremophor EL was used as the surfactant, the temperature was moved up and down in the range of 50°C to 90°C, and ethanol was used as the water-soluble organic solvent.

### [Example 4] Production of cyclic peptide crystals using PEG-based solvent, or mixed solvent containing PEG-based solvent

Cyclic peptide crystals were produced by a method including a step of bringing into contact with (i) a PEG-based solvent, or (ii) a mixed solvent containing one or more selected from the group consisting of an alcohol-based solvent, an aliphatic hydrocarbon-based solvent, and water, and a PEG-based solvent. Note that seed crystals were not used in the step of bringing into contact with the solvent in the following production. Also, in the following Examples, % (percent) represents % by volume (v/v%).

(Example 4-1-1) To compound CP01 (4.8 mg), PEG400 (0.015 mL) was added, isopropanol (0.005 mL) was added, and water (0.005 mL) was further added, and the mixture was stirred at 80°C for dissolution. The next day, the temperature was lowered to room temperature and the mixture was further stirred for 3 days, after which crystals of compound CP01 were precipitated.

(Example 4-3-1) To compound CP03 (5.8 mg), polyethylene glycol monolaurate (n = about 10) (0.015 mL) was added, and the mixture was stirred at 80°C for dissolution. This solution was further stirred at 80°C for 1 day, after which crystals of compound CP03 were precipitated.

(Example 4-4-1) To compound CP04 (6.3 mg), tetraethylene glycol (0.030 mL) was added, and the mixture was stirred at 80°C for 1 hour. After cooling to room temperature and further stirring for 1 day, crystals of compound CP04 were obtained.

(Example 4-4-2) To compound CP04 (5.5 mg), PEG400 (0.030 mL) was added, and the mixture was stirred at 80°C for 40 minutes to obtain crystals of compound CP04.

(Example 4-4-3) To compound CP04 (5.6 mg), PEG400 (0.020 mL) was added, and the mixture was stirred at 80°C for 30 minutes to obtain crystals of compound CP04.

(Example 4-4-4) To compound CP04 (5.4 mg), polyethylene glycol monolaurate (n = about 10) (0.020 mL) was added, and the mixture was stirred at 80°C for 40 minutes to obtain crystals of compound CP04.

(Example 4-4-5) To compound CP04 (5.6 mg), PEG600 (0.020 mL), which had been heated to 60°C and melted, was added, and the mixture was stirred at 60°C for 10 minutes. The mixture was further stirred at 80°C for 16 hours. Further stirring at room temperature for 3 hours obtained crystals of compound CP04.

(Example 4-4-6) To compound CP04 (5.7 mg), PEG2000 (0.020 mL), which had been heated to 60°C and melted, was added, and the mixture was stirred at 60°C for 30 minutes. The temperature was raised to 80°C, PEG2000 (0.080 mL) was added, and the mixture was stirred for 15 hours. After cooling to room temperature, a mixture of crystals of compound CP04 and crystals of PEG2000 was obtained. Figure 26 (D) shows the X-ray diffraction peaks of PEG2000, which has characteristic peaks at 19.2° and 24.4°. Figure 26 (C) shows the X-ray diffraction peaks of the obtained mixture, and multiple diffraction peaks other than 19.2° and 24.4° are observed. These are the diffraction peaks of the crystals of CP04.

(Example 4-4-7) To compound CP04 (6.1 mg), polyethylene glycol monostearate (n = about 4) (palmitate, stearate mixture) (0.030 mL), which had been heated to 60°C and melted, was added, and the mixture was then stirred at 80°C for 16 hours. Further stirring at room temperature for 3 hours obtained a mixture of crystals of compound CP04 and crystals of polyethylene glycol monostearate (n = about 4) (palmitate, stearate mixture). Figure 27 (B) shows the X-ray diffraction peaks of polyethylene glycol monostearate (n = about 4) (palmitate, stearate mixture), which has characteristic peaks at 21.7° and 24.1°. Figure 27 (A) shows the X-ray diffraction peaks of the obtained mixture, and multiple diffraction peaks other than 21.7° and 24.1° are observed. These are the diffraction peaks of the crystals of CP04.

(Example 4-4-8) To compound CP04 (5.0 mg), polyethylene glycol monostearate (n = about 10) (palmitate, stearate mixture) (0.020 mL), which had been heated to 60°C and melted, was added, and the mixture was stirred at 80°C for 16 hours. Further stirring at room temperature for 3 hours obtained crystals of compound CP04.

(Example 4-4-9) To compound CP04 (6.1 mg), diglyme (0.015 mL) was added, n-heptane (0.015 mL) was further added, and the mixture was stirred at room temperature for 5 days to obtain crystals of compound CP04.

(Example 4-4-10) To compound CP04 (5.0 mg), triglyme (0.005 mL) was added, and the mixture was stirred at 80°C for dissolution. This solution was further stirred at 80°C for 18 hours, after which crystals of compound CP04 were precipitated.

(Example 4-5-1) To compound CP05 (about 5 mg), tetraethylene glycol (0.020 mL) was added, and the mixture was stirred for dissolution. This solution was heated to 80°C and stirred for 1 hour, after which crystals of compound CP05 were precipitated.

(Example 4-5-2) To compound CP05 (about 5 mg), PEG400 (0.020 mL) was added, and the mixture was stirred for dissolution. This solution was heated to 80°C and stirred for 1 hour, after which crystals of compound CP05 were precipitated.

(Example 4-5-3) To compound CP05 (about 5 mg), polypropylene glycol (average molecular weight about 425) (0.020 mL) was added, and the mixture was stirred for dissolution. This solution was heated to 80°C and stirred for 1 hour, after which crystals of compound CP05 were precipitated.

(Example 4-5-4) To compound CP05 (about 5 mg), polyethylene glycol monolaurate (n = about 10) (0.020 mL) was added, and the mixture was stirred for dissolution. This solution was heated to 80°C and stirred for 1 hour, after which crystals of compound CP05 were precipitated.

(Example 4-5-5) To compound CP05 (about 5 mg), triglyme (0.020 mL) was added, and the mixture was stirred for dissolution. This solution was heated to 80°C and stirred for 1 hour, after which crystals of compound CP05 were precipitated.

(Example 4-5-6) To compound CP05 (5.3 mg), a water/ethylene glycol mixed solution (ethylene glycol ratio 80% by volume, 0.015 mL) was added, and the mixture was stirred at 100°C for dissolution. This solution was cooled to room temperature and stirred for 3 days, after which crystals of compound CP05 were precipitated.

(Example 4-7-1) To compound CP07 (4.8 mg), triglyme (0.010 mL) was added, and the mixture was stirred at room temperature for dissolution. This solution was further stirred at room temperature for 35 minutes, after which crystals of compound CP07 were precipitated.

(Example 4-7-2) To compound CP07 (4.6 mg), polyethylene glycol monolaurate (n = about 10) (0.015 mL) was added, and the mixture was stirred at 80°C for dissolution. This solution was further stirred at 80°C for 18 hours, after which crystals of compound CP07 were precipitated.

(Example 4-7-3) To compound CP07 (4.8 mg), triglyme (0.010 mL) was added, and the mixture was stirred at room temperature for 15 hours, after which crystals of compound CP07 were precipitated.

(Example 4-7-4) To compound CP07 (10.0 mg), PEG400 (0.020 mL) was added, and the mixture was stirred at 80°C for dissolution. This solution was further stirred at 80°C for 18 hours, after which crystals of compound CP07 were precipitated.

(Example 4-8-1) To compound CP08 (5.0 mg), triglyme (0.010 mL) was added, and the mixture was stirred at room temperature for 20 minutes, after which crystals of compound CP08 were precipitated.

(Example 4-8-2) To compound CP08 (5.1 mg), PEG400 (0.015 mL) was added to dissolve it at 80°C. After stirring at 80°C for 18 hours, the solution was returned to room temperature and further stirred for 1 day, after which crystals of compound CP08 were precipitated.

(Example 4-8-3) To compound CP08 (5.3 mg), polyethylene glycol monolaurate (n = about 10) (0.015 mL) was added, and the mixture was stirred at 80°C for dissolution. This solution was further stirred at 80°C for 10 minutes, after which crystals of compound CP08 were precipitated.

(Example 4-8-4) To compound CP08 (5.1 mg), triglyme (0.010 mL) was added, and the mixture was stirred at room temperature for dissolution. This solution was further stirred at room temperature for 15 hours, after which crystals of compound CP08 were precipitated.

(Example 4-8-5) To compound CP08 (10.1 mg), PEG400 (0.020 mL) was added, and the mixture was stirred at 80°C for dissolution. This solution was further stirred at 80°C for 5 hours, after which crystals of compound CP08 were precipitated.

From the above results, under the conditions using a PEG-based solvent or a mixed solvent containing a PEG-based solvent, crystals of six out of the eight cyclic peptides were obtained. In the case where a polyethylene glycol monofatty acid ester was used as the PEG-based solvent, crystals of five out of the eight cyclic peptides were obtained (Examples). In the case where a solvent containing polyethylene glycol (PEG400, PEG600, PEG2000) was used as the PEG-based solvent, crystals of five out of the eight cyclic peptides were obtained (Examples).

As the conditions for crystallizing a variety of cyclic peptides, the conditions using a polyethylene glycol monofatty acid ester or polyethylene glycol as the PEG-based solvent was used were preferred conditions. More preferably, polyethylene glycol monolaurate or PEG400 was used as the PEG-based solvent, and the temperature was in the range of room temperature to 80°C. The most preferred results were the conditions under which polyethylene glycol monolaurate was used as the PEG-based solvent and the temperature was 80°C.

### [Example 5] X-ray diffraction measurement of cyclic peptide crystals

### (Example 5-1)

The crystals obtained in Example 2, Example 3, and Example 4 were each subjected to powder X-ray diffraction measurement by the following measurement method. The results are shown in Figure 1A to Figure 30D.

### Measurement apparatus: D8 Discover, 2D VÅNTEC-500 solid state detector (manufactured by Bruker)

Radiation source: CuKα
Tube voltage and tube current: 40 kV and 40 mA or 50 kV and 1000 µA
Measurement range: 5 to 31°
Exposure time: 40 to 600 seconds

### (Example 5-2)

The crystals obtained in Example 2-1-1 were subjected to single crystal X-ray structural analysis by the following measurement method.
Measurement apparatus: Rigaku R-AXIS RAPID-II with a VariMax Cu diffractometer
(manufactured by Rigaku Corporation)
Radiation source: CuKα
Tube voltage and tube current: 40 kV and 30 mA
Temperature: -180°C
Measurement: Measurements were made using strategy and exposure time that were believed to yield sufficient diffraction spots for structural analysis.
Structural analysis: Initial structural determination was done by the direct method (SHELXD97, Crystal Structure 4.2.2, Rigaku Corporation), and structural refinement was done by full-matrix least-squares method (SHELXL97, Crystal Structure 4.2.2, Rigaku Corporation). Refinement was performed assuming that all temperature factors are isotropic. The results are shown in Figure 31A.

## Claims

1. A method for producing cyclic peptide crystals, the method comprising a step of bringing a cyclic peptide into contact with a solvent, wherein the cyclic peptide is a cyclic peptide having the following characteristics (I), (II), and (III):
(I) a characteristic of containing a cyclic moiety composed of 8 to 16 residues of amino acids in total, with a total number of amino acids being 8 to 20 residues;
(II) a characteristic of containing at least 2 residues of N-substituted amino acids; and
(III) a characteristic of having a molecular weight (g/mol) of 1204 or more and 3000 or less.

2. The method according to claim 1, wherein the crystals have one or more diffraction peaks in powder X-ray diffraction using CuKα radiation, or have polarization in observation using a polarization microscope.

3. The method according to claim 1, wherein the crystals have one or more diffraction peaks in powder X-ray diffraction using CuKα radiation.

4. The method according to any one of claims 1 to 3, wherein the solvent is any solvent selected from the group consisting of the following (1), (2) and (3):
(1) (i) a solvent with a molecular weight of 18 or more and 170 or less, or (ii) a mixed solvent containing two or more solvents with a molecular weight of 18 or more and 170 or less;
(2) water containing 0.01 to 30 wt/v% of a surfactant and 5 to 50 v/v% of a water-soluble organic solvent, based on a total amount of the solvent; and
(3) (i) a PEG-based solvent, or (ii) a mixed solvent containing one or more selected from the group consisting of an alcohol-based solvent, an aliphatic hydrocarbon-based solvent, and water, and a PEG-based solvent.

5. The method according to any one of claims 1 to 3, wherein the solvent is solvent (A) with a molecular weight of 18 or more and 170 or less, or a mixed solvent of solvent (A) with a molecular weight of 18 or more and 170 or less and solvent (B) with a molecular weight of 18 or more and 170 or less, the solvent (A) is one or more selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, and a ketone-based solvent, and the solvent (B) is one or more selected from the group consisting of an aliphatic hydrocarbon-based solvent, ethylene glycol, and water.

6. The method according to claim 5, wherein the solvent (A) is formamide.

7. The method according to claim 5 or 6, wherein the solvent (B) is one or more solvents selected from the group consisting of an aliphatic hydrocarbon-based solvent, ethylene glycol, and water.

8. The method according to any one of claims 5 to 7, wherein a volume ratio (v/v) between the solvent (A) and the solvent (B) in the solvent is 1:0 to 1:40.

9. The method according to any one of claims 5 to 8, wherein the solvent (A) and the solvent (B) have a melting point of 25°C or lower.

10. The method according to any one of claims 1 to 3, wherein the solvent is water containing 0.01 to 30 wt/v% of a surfactant and 5 to 50 v/v% of a water-soluble organic solvent, based on a total amount of the solvent, and the surfactant is one or more selected from the group consisting of a cationic surfactant, an anionic surfactant, an amphoteric surfactant, and a nonionic surfactant.

11. The method according to any one of claims 1 to 3, wherein the solvent is water containing 0.01 to 30 wt/v% of a surfactant and 5 to 50 v/v% of a water-soluble organic solvent, based on a total amount of the solvent, and the surfactant is one or more selected from the group consisting of a primary amine salt, an alkyltrimethyl ammonium salt, an alkylpyridinium salt, an alkylpolyoxyethyleneamine, a fatty acid salt, a rosin acid salt, an alkyl sulfate, an alkylpolyoxyethylene sulfate, an alkylnaphthalene sulfate, a lignin sulfate, an alkyl phosphate, a N-alkyl β-aminopropionic acid, a N-alkyl sulfobetaine, a N-alkyl hydroxysulfobetaine, a lecithin, an alkyl polyoxyethylene ether, an alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, and a polyoxyethylene sorbitan fatty acid ester.

12. The method according to any one of claims 1 to 3, wherein the solvent is (i) a PEG-based solvent, or (ii) a mixed solvent containing one or more selected from the group consisting of an alcohol-based solvent, an aliphatic hydrocarbon-based solvent, and water, and a PEG-based solvent, and the PEG-based solvent is (i) a solvent represented by R¹(OCHR³CH₂)nOR², n being a natural number of 1 or more and 10 or less, or (ii) a mixture of solvents represented by R¹(OCHR³CH₂)nOR², an average of n being 3 to 100, where R¹ and R² are each independently hydrogen, a C₁ to C₄ alkyl, or -C(=O)R⁴, R³ is hydrogen or a C₁ to C₄ alkyl, and R⁴ is a C₁ to C₁₈ alkyl optionally substituted with a hydroxy group or a C₁ to C₁₈ alkenyl optionally substituted with a hydroxy group.

13. The method according to any one of claims 1 to 3, wherein the solvent is (i) a PEG-based solvent, or (ii) a mixed solvent containing one or more selected from the group consisting of an alcohol-based solvent, an aliphatic hydrocarbon-based solvent, and water, and a PEG-based solvent, and the PEG-based solvent is diglyme, triglyme, tetraglyme, ethylene glycol, diethylene glycol, triethylene glycol, tetraethylene glycol, polyethylene glycol, polypropylene glycol, or a polyethylene glycol monofatty acid ester.

14. A method for producing cyclic peptide crystals, the method comprising a step of bringing a cyclic peptide into contact with a solvent, wherein the cyclic peptide is a cyclic peptide having the following characteristics (I) and (II):
(I) a characteristic of containing a cyclic moiety composed of 8 to 16 residues of amino acids in total, with a total number of amino acids being 8 to 20 residues; and
(II) a characteristic of containing at least 2 residues of N-substituted amino acids,
the solvent is solvent (A) or a mixed solvent containing solvent (A) and solvent (B), the solvent (A) is one or more selected from the group consisting of an amide-based solvent, a sulfoxide-based solvent, an aromatic hydrocarbon-based solvent, a halogen-based solvent, and an ester-based solvent, and the solvent (B) is one or more selected from the group consisting of an aliphatic hydrocarbon-based solvent, ethylene glycol, and water.

15. The method according to any one of claims 1 to 14, wherein, in the step of bringing a cyclic peptide into contact with a solvent, a concentration of the cyclic peptide is 1 mg to 2000 mg/mL.

16. The method according to any one of claims 1 to 15, wherein the step of bringing a cyclic peptide into contact with a solvent is performed at a temperature of -10°C to 120°C for 30 minutes to 12 weeks.

17. A method for screening a method for crystallizing a cyclic peptide, the method comprising the following steps (a) and (b):
(a) a step of producing cyclic peptide crystals by the method according to any one of claims 1 to 16; and
(b) a step of analyzing the crystals by powder X-ray crystal diffraction.
